# FASCICULE DE BREVET EUROPEEN

(11) **EP 1 484 051 B1**
(45) Date de publication et mention de la délivrance du brevet: **21.08.2013**
(21) Numéro de dépôt: 04291239.4
(22) Date de dépôt: 14.05.2004
(51) Int. Cl.: A61K 8/19, A61K 8/58, A61K 8/49, A61Q 17/04, A61K 8/31, A61K 8/35, A61K 8/60, A61K 8/72, C07F 7/08, C07F 7/10, C07D 251/70

(54) **Utilisation d'au moins un filtre de la lumière bleue pour préserver le capital de caroténoïdes endogènes de la peau; nouveaux filtres de la lumière bleue; compositions cosmétiques**
Verwendung mindestens eines Filters des blauen Lichts zum Schutz der endogenen Carotinoide in der Haut, neue Filter des blauen Lichts, kosmetische Zusammensetzungen
Use of at least one blue-light screening agent to preserve the amount of endogenous carotenoids in the skin, new blue- light screening agents, cosmetic compositions.

(30) Priorité: 05.06.2003 FR 0306800
(43) Date de publication de la demande: 08.12.2004
(73) Titulaire: L'Oréal, 75008 Paris (FR)
(72) Inventeur: Candau, Didier, 91570 Bievres (FR); Duranton, Albert, 78600 Maison Laffite (FR); Pruche, Francis, 60300 Senlis (FR); Richard, Hervé, 93420 Villepinte (FR)
(74) Mandataire: Miszputen, Laurent

(56) Documents cités:
- EP-A- 0 521 651
- EP-A- 0 933 376
- WO-A-02/053119
- FR-A- 2 816 839
- US-A- 3 062 721
- US-A- 3 272 713
- US-A- 3 846 556
- US-A- 5 236 698
- US-A- 6 110 478
- US-B1- 6 284 224
- US-B1- 6 509 008
- DATABASE CHEMICAL ABSTRACTS [Online] XP002272012 extrait de STN Database accession no. 109: 134 846 & JP 63 096120 A (MATSUURA YAKUGYO CO., LTD) 27 avril 1988 (1988-04-27)
- C. GHISALBERTI: "Mélanines végétales: une protection physiologique de la peau." PARFUMS, COSMETIQUES ACTUALITES., no. 164, mars 2002 (2002-03), pages 102-104, XP002272011
- F. BONINA ET AL.: "in vitro antioxidant activity and in vivo photoprotective effect of a red orange extract" INTERNATIONAL JOURNAL OF COSMETIC SCIENCE, no. 20, 1998, pages 331-342, XP000993169

## Description

L'invention concerne un procédé cosmétique **pour préserver le teint naturel et la protection antioxydante naturelle de la peau apportés par les caroténoïdes endogènes présents dans la peau, qui consiste à appliquée sur la surface de la peau une composition comprenant** au moins un agent filtrant les radiations lumineuses de longueur d'onde allant de 370 à 500 nm **particulier que l'on définira ci-dessous,** dans une composition appliquée sur la surface de la peau,

L'invention concerne également de nouveaux composés amino aryl vinyl-s-triazine ainsi que leur utilisation en cosmétique comme agent filtrant la lumière bleue.

L'invention concerne les compositions cosmétiques ou dermatologiques contenant ces composés amino aryl vinyl-s-triazine.

La peau est soumise aux agressions du rayonnement solaire qui a pour conséquence d'augmenter les agressions oxydatives comme :
- la déplétion des capacités antioxydantes de la peau, peroxydation lipidique et altération de la fonction barrière (Thiele JJ Skin Pharmacol Appl Skin Physiol 2001;14 Suppl 1:87-91)
- l'oxydation des protéines. (« Photoaging is associated with protein oxidation in human skin in vivo. » Sander CS, Chang H, Salzmann S, Muller CS, Ekanayake-Mudiyanselage S, Elsner P, Thiele JJ J Invest Dermatol 2002 Apr;118(4):618-25 et de provoquer une altération du teint de la peau.

Il existe une protection physiologique naturelle de la peau contre ces agressions grâce aux caroténoïdes endogènes présents dans les couches vivantes de la peau. Ces caroténoïdes d'origine alimentaire sont également un facteur important dans la perception de la couleur de la peau en particulier par leur contribution à la teinte (composante rouge/jaune)(Alaluf S, Heinrich U, Stahl W, Tronnier H, Wiseman S.« Dietary carotenoids contribute to normal human skin color and UV photosensitivity. » J Nutr. 2002 Mar;132(3):399-403)

On conçoit l'importance cosmétique de protéger ce capital de caroténoïdes endogènes présents dans la peau pour préserver le teint naturel de la peau.

Il existe le besoin de trouver un moyen de préserver le teint naturel et la protection naturelle anti-oxydante de la peau apportés par les caroténoïdes endogènes présents dans la peau.

On connaît dans la demande EP933376 des composés 2,4,6-trianilo-s-triazines greffés par des chaînes siliconées utilisées comme filtres UVB ayant une bonne solubilité dans les solvants habituellement utilisés dans les produits solaires. On connaît également dans le brevet US6,509,008 des compositions solaires associant des filtres UV du type 1,3,5-triazine associés à un ester de N-acylaminoacide en particulier l'isopropyl lauroyl sarcosinate (ELDEW SL 205 d'Ajinomoto) dans le but d'augmenter le facteur de protection solaire. On connaît dans le brevet US 5,236,698 des dérivés s-triazine portant des substituants benzalmalonates pouvant avoir des propriétés absorbantes des rayons UVA et/ou UV-B, ayant une bonne solubilité dans les huiles, une bonne stabilité chimique et photochimique et ayant un effet photostabilisant vis-à-vis des filtres UVA du type dibenzoylméthane. Dans la demande WO02/053119 sont décrites des formulations solaires à base de triazines asymétriques substituées contenant en plus un ester de saccharose dans le but d'améliorer la protection solaire, d'obtenir un effet non-collant, un effet non-gras et résistant au sable.

On connaît dans la demande EP521651 des compositions solaires à base de filtres du type 1,3-dicétones substituées comprenant un système conservateur constitué par un acide organique choisi parmi l'acide sorbique, l'acide benzoïque, l'acide salicylique, l'acide propionique, l'acide dehydroacétique, l'acide p-hydroxybenzoiîque et leurs sels alcalins ou leurs esters ainsi que leurs mélanges.

On connaît dans l'article du Dr Carlo GHISLABERTI extrait de la revue Parfums Cosmétiques Actualités N°164 Avril/Mai 2002 pages 102-104 des biopolymères phytomélanines obtenues par un procédé enzymatique et chimique à partir de polyphénols végétaux. Ils sont utilisés comme piégeurs de radicaux libres et de métaux, comme absorbeurs d'UV, limitent les effets oxydatifs et stimulent la mélanogénèse.

L'article de F. Bonina et al « In vitro antioxidant activity and in vivo photoprotective effect of a red oraneg extract » extrait de la revue International Journal of Cosmetic Science 20, 331-342 (1998) décrit les propriétés antioxydantes et photoprotectrice contre les radiations UV d'un colorant rouge extrait d'orange.

On connaît dans la demande JP 63-096120 des compositions solaires à large spectre (UVA et UVB) constituées de dérivés de chalcone de dérivés de flavone, de dérivés de coumarine

On connaît dans la demande FR2816839 des compositions administrables par voie orale pour protéger la peau contre le rayonnement UV, pour protéger la peau contre les mécanismes de vieillissement cellulaire et ayant parallèlement une activité imminostimulante. Ces compositions sont essentiellement constituées d'un β-carotène, d'au moins un flavonoïde en particulier une catéchine et au moins une vitamine (vitamines E et/ou C et/ou B5)

Le document US 6,110,478 décrit des compositions appliquées par voie topique ou orale ayant une activité bronzante et photoprotectrice comprenant un caroténoïde ayant une activité pro-vitamine A choisi parmi le β-carotène, l' α-carotène ou leurs mélanges en association avec le licopène et éventuellement en plus un caroténoïde choisi parmi la zéaxanthine, la cryptoxanthine, la lutéine ou leurs mélanges.

Le document US 3,272,713 décrit des compositions solaires destinées à protéger la peau contre les effets des radiations actiniques de longueurs d'onde allant de 268,5 à 290 nm constituées de l'association d'une quinione telle que la juglone, la ménadione, le henné et un composé carbonylé choisi parmi la dihydroxyacétone, le 5-(hydroxymethyl)-2-furaldéhyde, l'aldéhyde pyvurique, le glycéralaldéhyde, l'alloxane et le monohydroxyacétone ou leurs mélanges.

Le document US 3,062,721 décrit une composition cosmétique à base de graines de lécithine, de graines de borax, de graines d'alfata, de graines de papaïne, de graines d'aloe, de graines de sarsaparilla, de menthol, d'alcool et de hexachloropène. Celle-ci est notamment utilisée comme lotion après solaire pour traiter les brûlures dues au soleil.

Le document US3,846,556 décrit des formulations cosmétiques sous forme d'émulsion eau-dans-huile comprenant un système émulsifiant constitué d'un mélange comprenant (a) un lanolate de calcium, magnésium, aluminium, zinc, lithium et (b) de lanoline et/ou de lanoline hydrogénée. Ces formulations peuvent être des produits de maquillage à base de pigments (oxyde de fer, oxide de titane) et de colorant (D& C Red 8).

On sait que dans le document US 6,284224, la tartrazine ou l'érythrosine B peuvent être utilisées dans une solution d'acétone pour traiter des tumeurs dans le tissu épidermique.

La Demanderesse après d'intenses travaux de recherche a découvert que les radiations lumineuses de longueur d'onde allant de 370 et 500 nm (correspondant à la lumière bleue) avaient tendance à diminuer sensiblement le capital endogène des caroténoïdes de la peau. Ce problème technique n'a jamais été posé jusqu'à présent. A la suite de cette découverte la Demanderesse a donc trouvé de manière surprenante et inattendue un moyen de conserver le capital endogène des caroténoïdes de la peau par l'application sur la surface de la peau d'un agent filtrant les radiations lumineuses de longueur d'onde allant de 370 à 500 nm **particulier que l'on définira plus loin**. Ce qui permet d'obtenir à la fois une bonne protection du teint naturel de la peau ainsi qu'une bonne protection naturelle anti-oxydante de la peau.

La Demanderesse a découvert également de nouveaux composés amino aryl vinyl-s-triazine de formule que l'on définira en détail plus loin dans la description ayant des propriétés d'absorption des radiations lumineuses de longueur d'onde allant de 370 et 500 nm

L'objet de l'invention est donc un procédé cosmétique **pour préserver le teint naturel et la protection antioxydante naturelle de la peau apportés par les caroténoïdes endogènes présents dans la peau, qui consiste à appliquée sur la surface de la peau une composition comprenant** au moins un agent filtrant les radiations lumineuses de longueur d'onde allant de 370 à 500 nm **particulier que l'on définira ci-dessous,**

Un autre objet de l'invention consiste en de nouveaux composés amino aryl vinyl-s-triazine de formule générale (III) que l'on définira en détail plus loin dans la description.

L'invention concerne également les compositions cosmétiques ou dermatologiques contenant dans un milieu physiologiquement acceptable ces composés amino aryl vinyl-s-triazine de formule générale (III).

On entend par «agent filtrant les radiations lumineuses de longueur d'onde 370 à 500 nm» par tout composé ou mélanges de composés organiques ou minéraux, synthétiques ou naturels, capables de filtrer les radiations lumineuses de 370 à 500 nm et ayant une bonne compatibilité pour la peau. Leur spectre d'absorption dans ledit domaine présentera de préférence un maximum d'absorption se trouvant entre 380 et 450 nm. Lesdits composés peuvent être une simple molécule ou un polymère. La filtration de la lumière peut résulter d'un phénomène d'absorption, de réflexion et/ou de diffusion.
Les agents filtrant les radiations lumineuses allant de 370 à 500 nm, utilisables selon l'invention**sont choisis parmi**:
(i) les carbohydrates ;
(ii) les pigments minéraux jaunes ou jaunes oranges ;
(iii) les composés azoïques ou quinoniques ;
(iv) les colorants dérivés nitrés benzéniques ;
(v) les composés dérivés d'aryl vinylène cétone ;
(vi) les composés amino aryl vinyl-s-triazine et leurs mélanges.

### I les carbohydrates

Parmi les carbohydrates utilisables selon l'invention, on peut citer le les produits colorés jaune issus du chauffage ou oxydation de mono ou polysaccharides (glucose, fructose, saccharose) tels que le caramel

### II les pigment minéraux jaunes ou oranges

Parmi les pigments minéraux, on peut citer en particulier les ceux du type oxyde de fer et plus particulièrement les nanopigments de taille de particule élémentaire inférieure à 100 nm tels que
- l'oxyde de fer jaune transparent (aiguilles de 10nm X 100nm) vendu sous le nom « Cappoxyt jaune 4214 X » par la Société Capelle ;
- l'oxyde de fer jaune enrobé stéarine (10nm) vendu sous le nom « oxyde de fer transparent » par la BASF ;
- l'oxyde de fer jaune micronisé vendu sous le nom « TY-220 » par la Société MITSUBISHI.

### III les colorants du type azoïque ou du type quinonique

Parmi les colorants du type quinonique naturels ou synthétiques utilisables selon l'invention, on peut citer par exemple ceux de type naphtoquinone jaune ou jaune-orange et leur produits d'oxydation tels que la juglone, la lawsone ; ceux de type anthraquinone et leur produits d'oxydation jaune ou jaune-orange et ayant une bonne compatibilité pour la peau.

Parmi les colorants du type azoïque utilisables selon l'invention, on peut citer par exemple la tartrazine de structure :

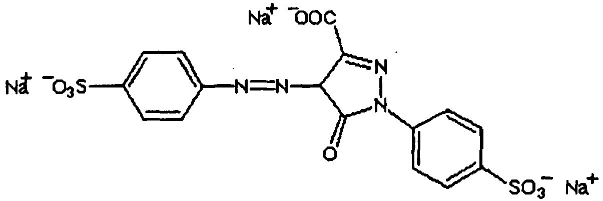

Une famille particulière de colorants du type azoïque et ceux du type quinonique utilisable selon l'invention est constitué par ceux décrits dans la demande de brevet EP0839815 formule (1) ou (2) ou (3) : 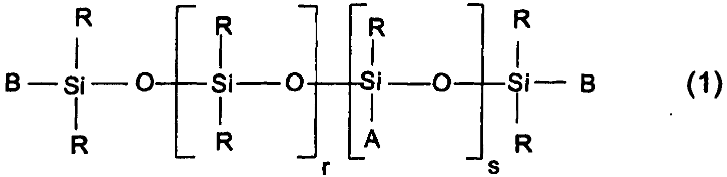 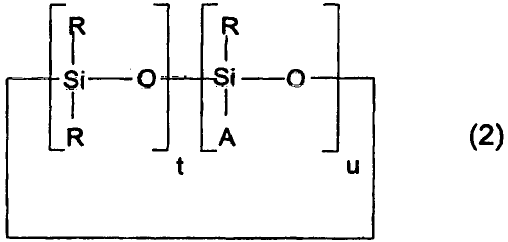 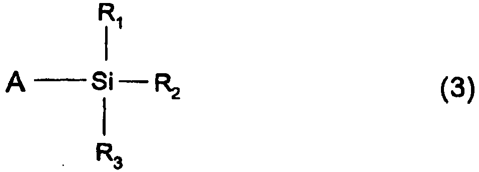 dans lesquelles, pour les formules (1) et (2) :
- R, identiques ou différents, sont choisis parmi les radicaux alkyles en C₁₋C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et
- t est un nombre entier compris entre 0 et 10 inclusivement,
   étant entendu que t + u est égal ou supérieur à 3,
- R₁, R₂, R₃, identiques ou différents, sont choisis parmi les radicaux alkyles et alcényles en C₁-C₈, linéaires ou ramifiés, saturés ou insaturés,
   les symboles A désignent identiques ou différents, un radical de formules (4a) (4b) (4c) ou (4d) suivantes : 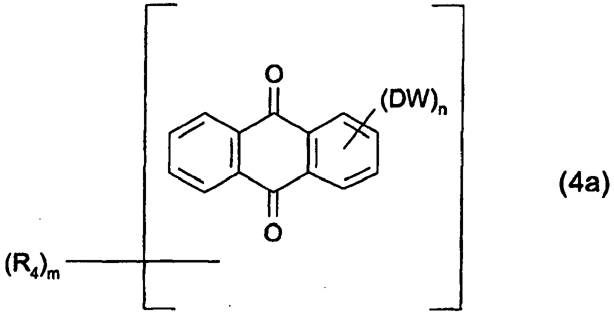 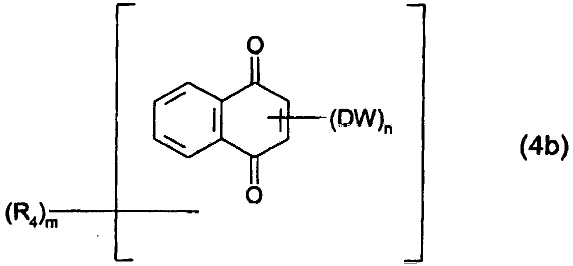 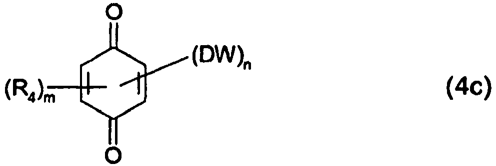 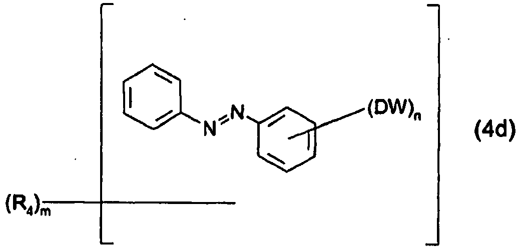
- R₄, identiques ou différents, représentent un radical alkyl, linéaire ou ramifié en C₁-C₆, un radical OH, alcoxy en C₁-C₄, hydroxyalkyl(C₁C₄), COOH, CONH₂, CN, SO₃H, halogène, NO₂, NR₅R₆ dans lequel R₅ et R₆, identiques ou différents, désignent un atome d'hydrogène, un radical alkyl en C₁-C₈, ou hydroxyalkyl(C₁-C₄), ou aminoalkyl(C₁-C₄), ou forment ensemble avec l'atome d'azote auquel ils sont attachés un hétérocycle à 5 ou 6 chaînons éventuellement interrompu par un atome d'oxygène ou de soufre,
- m est un nombre entier compris entre 0 et 2 inclusivement,
- n est un nombre entier égal à 1 ou 2,
- D est est un radical -SO₂NH-, -CONH-, -O-, ou un radical -NR₇- dans lequel R₇ est H ou CH₃,
- W est un radical divalent de formule (5) : 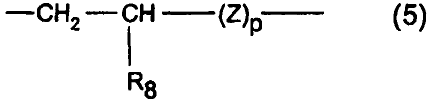 ou de formule (6) :

   -HC=CH-(Z)ₚ- (6)

   dans lesquelles
- R₈ désigne un atome d'hydrogène, un radical hydroxyle, un radical alkyl en C₁-C₈, linéaire ou ramifié, saturé ou insaturé,
- Z est un radical alkylène en C₁-C₆ linéaire ou ramifié éventuellement substitué par un radical OH ou un radical alcoxy en C₂-C₈ linéaire ou ramifié, saturé ou insaturé,
- p est un nombre entier égal à 0 ou 1.

Selon l'invention, les composés de formule (1), (2) ou (3) plus particulièrement préférés sont les suivants :
- 4-(4-diméthylaminophénylazo)-N-[3-[1,3,3,3-tétraméthyl-1-[(triméthylsilyl)-oxy]-disiloxanyl]-propyl]-benzènesulfonamide [composé (7)] : 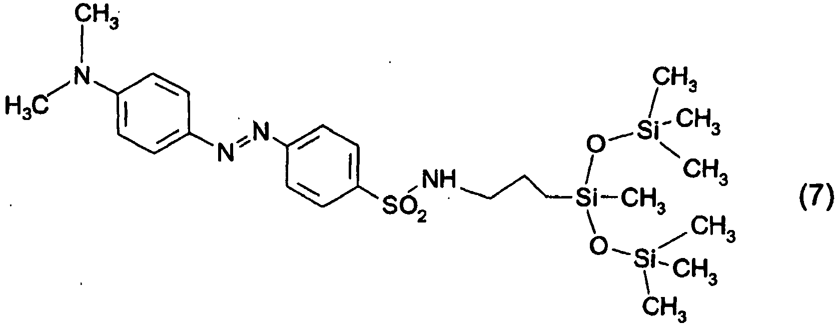
- 4-(4-diméthylaminophénylazo)-N-[3-[1,3,3,3-tétraméthyl-1-[(triméthylsilyl)-oxy]-disiloxanyl]-propyl]-benzamide [composé (8)] : 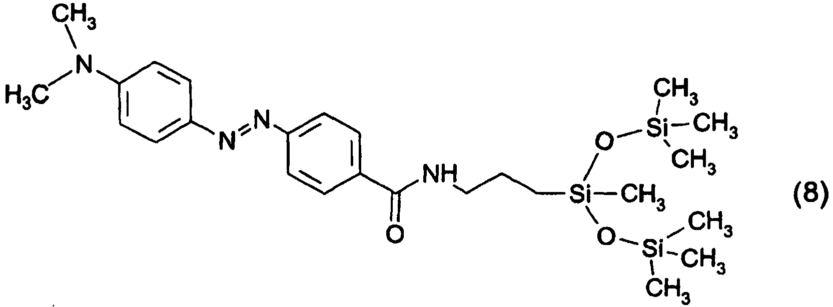
- 2-(4-méthoxy-2-nitrophénylazo)-5-(3-triméthylsilanyl-propoxy)-phénol [composé (9)] : 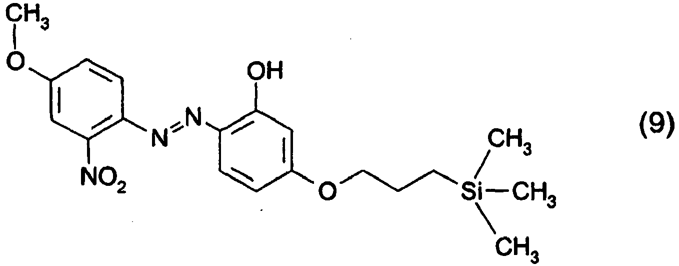
- 2,5-bis-[3-[1,3,3,3-tétraméthyl-1-[(triméthylsilyl)-oxy]-disiloxanyl]-propylamino]-benzoquinone [composé (10)] : 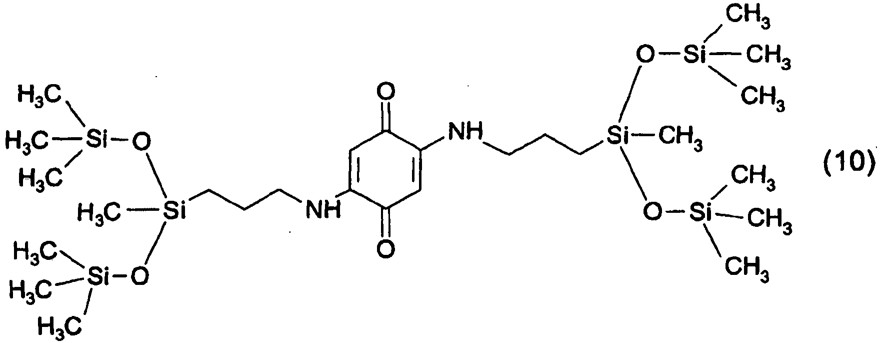
- 2-chloro-3-[3-[1,3,3,3-tétraméthyl-1-[(triméthylsilyl)-oxy]-disiloxanyl]-propyl-amino]-[1,4]-naphtoquinone [composé (11)] : 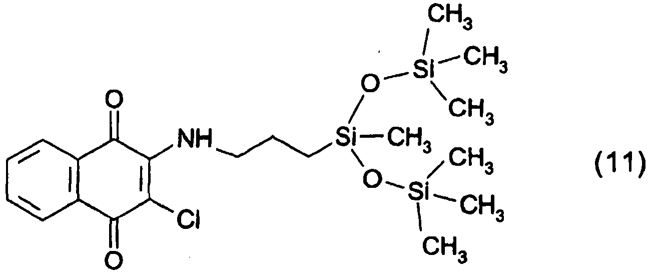
- 2-[3-[1,3,3,3-tétraméthyl-1-[(triméthylsilyl)-oxy]-disiloxanyl]-propylamino]-[1,4]-naphtoquinone [composé (12)] : 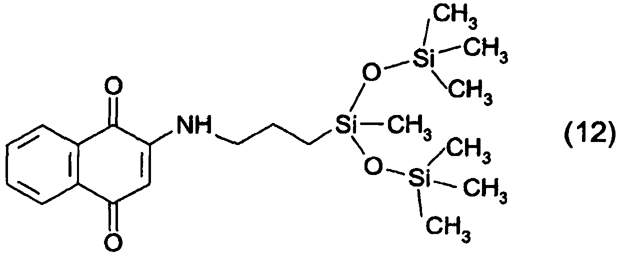
- 1-hydroxy-4-[3-[1,3,3,3-tétraméthyl-1-[(triméthylsilyl)-oxy]-disiloxanyl]-propyl-amino]-anthraquinone [composé (13)] : 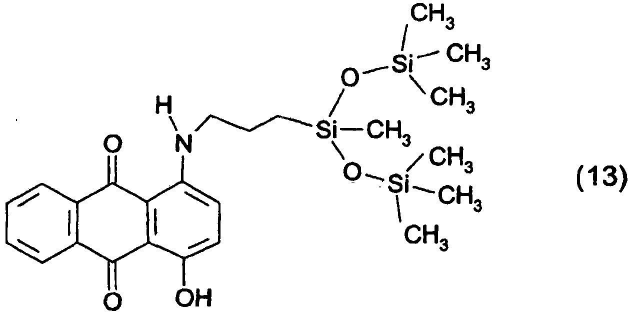
- 1,4-di-[3-[1,3,3,3-tétraméthyl-1-[(triméthylsilyl)-oxy]-disiloxanyl]-propyl-amino]-anthraquinone [composé (14)] :

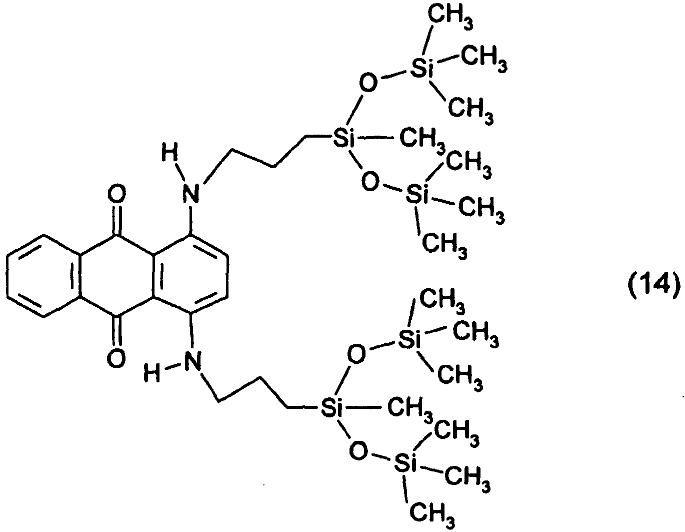

### IV les colorants nitrés benzéniques

Parmi les colorants directs nitrés benzéniques utilisables selon l'invention, on peut citer ceux décrits dans la demande de brevet WO 97 34904 de formule (15) ou (16) : 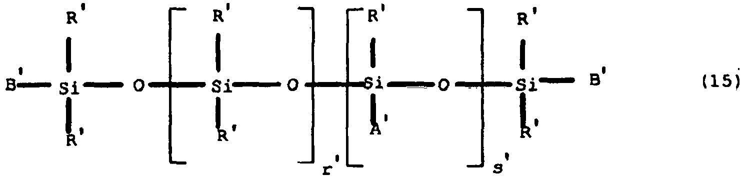 ou 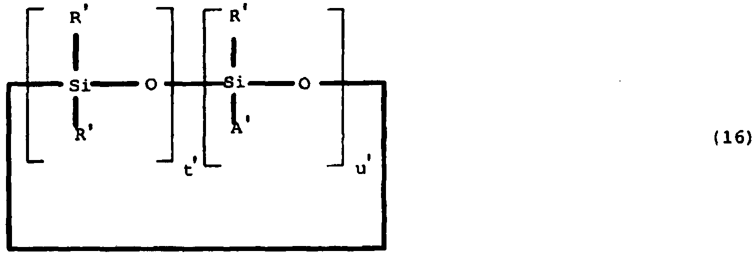 dans lesquelles :
- R', identiques ou différents, sont choisis parmi les radicaux alkyles linéaires ou ramifiés en C₁-C₁₀, phényle et trifluora-3,3,3 propyle, au moins 80% en nombre des radicaux R' étant méthyle,
- B', identiques ou différents, sont choisis parmi les radicaux R' ci-dessus et le radical A défini ci-après,
- r' est un nombre entier compris entre 0 et 50 inclusivement, et s' est un nombre entier compris entre 0 et 20 inclusivement, avec la condition que si s' est nul alors au moins l'un des deux symboles B désigne A,
- u' est un nombre entier compris entre 1 et 6 inclusivement, et t' est un nombre entier compris entre 0 et 10 inclusivement, étant entendu que t' + u' est égal ou supérieur à 3,
- et le symbole A' désigne un radical monovalent lié directement à un atome de silicium, et qui répond à la formule (17) suivante : 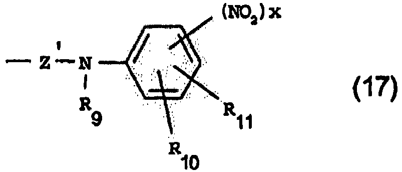
- Z' est le radical divalent : 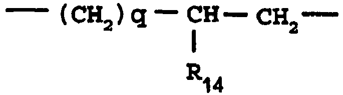 ou hydrogène,
- x est 1 ou 2,
- q représente un nombre entier compris entre 0 et 10, inclusivement,
- R₉ représente l'hydrogène ou un radical alkyle en C₁-C₄,
- R₁₀ représente l'hydrogène ou un radical alkyle en C₁-C₄, ou le radical divalent Z' défini ci-dessus,
- R₁₁ représente l'hydrogène ou un radical NR₁₂R₁₃ dans lequel R₁₂ et R₁₃ représentent l'hydrogène ou un radical alkyle en C1-C4, un radical mono- ou di-hydroxyalkyle en C₂-C₄ ou le radical divalent Z,
- R₁₄ représente l'hydrogène, un radical OH ou halogène, un radical alkyle en C₁-C₄, ou un radical alkoxy en C₁-C₄.

Selon l'invention, les composés de formule (15) plus particulièrement préférés sont les suivants :

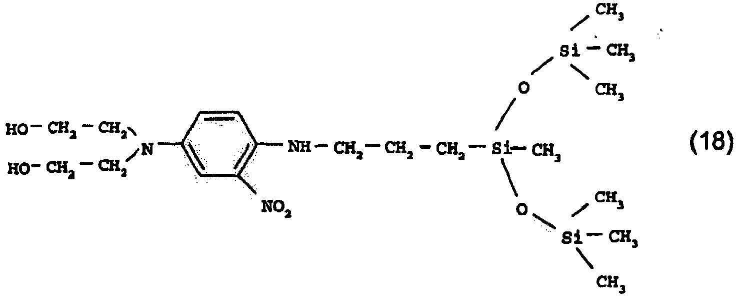

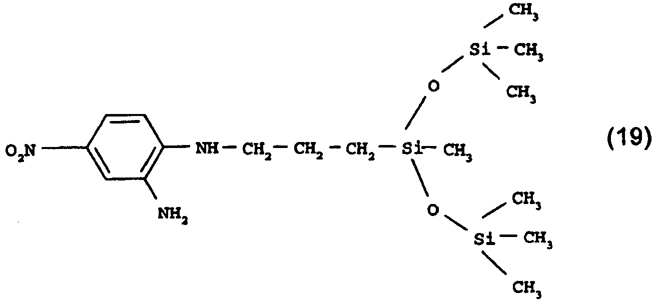

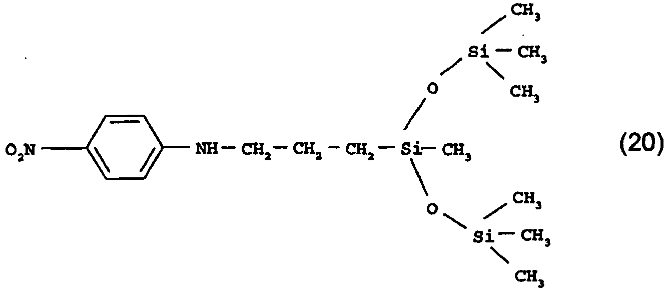

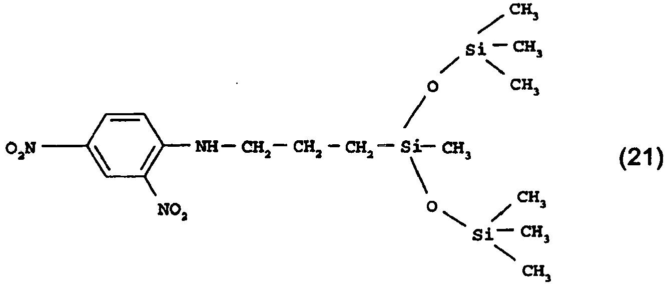

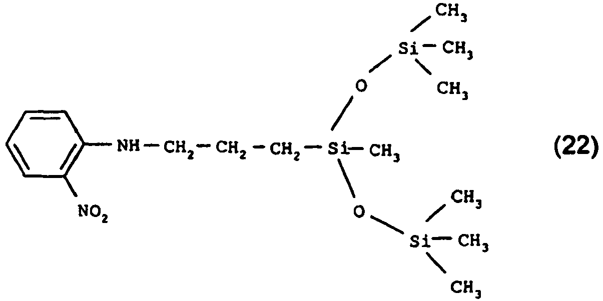

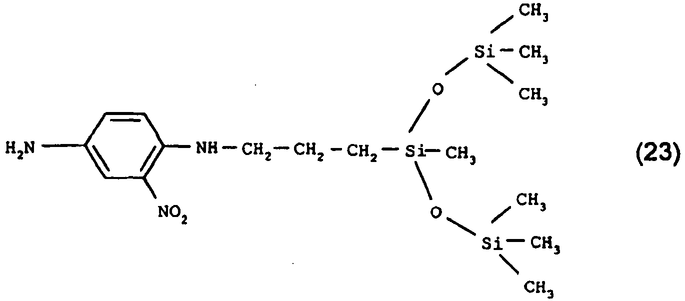

### V les composés du type aryl vinylène cétone

Parmi les composés organiques du type aryl vinylène cétone capables de filtrer les radiations allant de préférence de 380 à 500 nm selon l'invention, on peut citer ceux décrits dans la demande de brevet FR 2827510 correspondant à l'une des formules (I) et (II) suivantes : 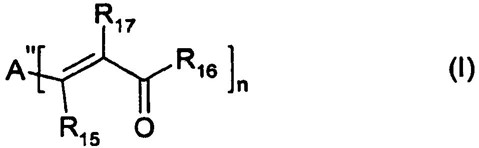 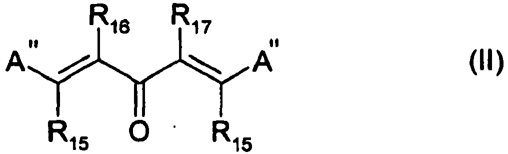 dans lesquelles :
n = 1 ou 2,
A", dans la formule (I) lorsque n=1 ou dans la formule (II), est un radical aryle choisi parmi les formules (a) à (d) suivantes, ou dans la formule (I) lorsque n=2, est un radical choisi parmi les formules (e) à (h) suivantes : 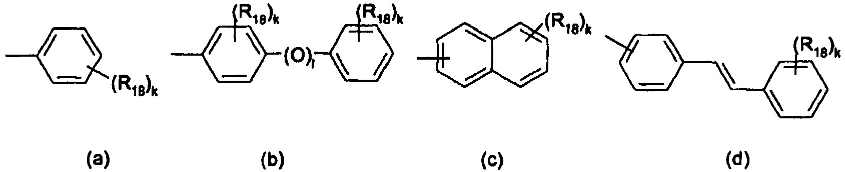 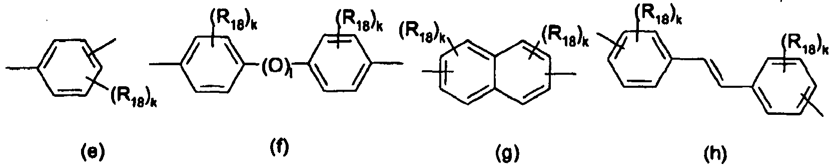
dans lesquelles :
- chacun des symboles R₁₈ représente indépendamment un groupe OH ; un atome d'halogène ; un groupe alkyle en C₁-C₆, linéaire ou ramifié et contenant éventuellement un atome de silicium ou un groupement siloxanique ; un groupe alcoxy en C₁-C₆, linéaire ou ramifié et contenant éventuellement un atome de silicium ou un groupement siloxanique ; un groupe alcoxycarbonyle en C₁-C₅, linéaire ou ramifié ou un groupe alkylsulfonamide en C₁-C₆, linéaire ou ramifié et contenant éventuellement un atome de silicium, un groupement siloxanique ou une fonction aminoacide ;
- k représente un nombre entier compris entre 0 et 3 indus ;
- l représente 0 ou 1;
- R₁₅ représente l'hydrogène ou un groupe OH ;
- R₁₆ représente l'hydrogène, un groupe alkyle en C₁-C₆, linéaire ou ramifié et contenant éventuellement un atome de silicium ou un groupement siloxanique, un groupe cyano, un groupe alkylsulfonyle en C₁-C₆, un groupe phénylsulfonyle,
- R₁₇ représente un groupe alkyle en C₁-C₆, linéaire ou ramifié et contenant éventuellement un atome de silicium, un groupement siloxanique ou un groupe phényle pouvant former un bicycle et éventuellement substitué par un ou deux radicaux R₁₈ tels que définis précédemment,
- R₁₆ et R₁₇ peuvent former ensemble un reste hydrocarboné en C₂-C₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium, un groupement siloxanique ou une fonction aminoacide ; à condition que lorsque n=1, R₁₆ et R₁₇ ne forment pas un noyau camphre.

A titre d'exemples de composés de formule (1) dans laquelle n=1 utilisables selon l'invention, on peut mentionner les familles suivantes :
- Styryl Cétone (Kao JP 04 134 042) telle que la 1-(2,4,5-triméthoxy-phényl)-4,4-diméthyl-pent-1-èn-3-one (λₘₐₓ 362 nm): 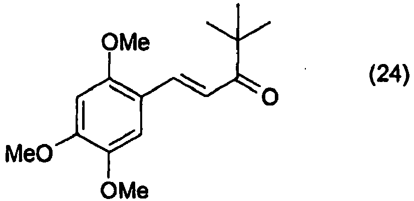
- Benzylidène Chromanone (Kao JP 04 134 043) telle que la 3-(3-éthoxy-4-butoxy-benzylidène)-2,3,4a,8a-tétrahydro-chromèn-4-one (λₘₐₓ 370 nm) : 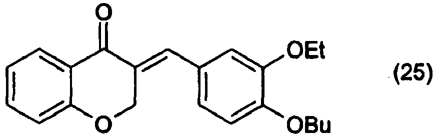
- Benzylidène Thiochromanone (Kao JP 04 134 043) telle que la 3-(4-méthoxy-benzylidène)-2,3,4a,8a-tétrahydro-chromèn-4-thione : 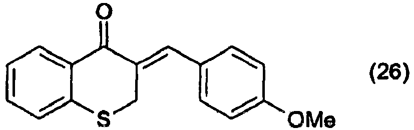
- Benzylidène Indanone (Kao JP 04 134 043) telle que la 2-(3-méthoxy-4-butoxy-benzylidène)-indan-1-one : 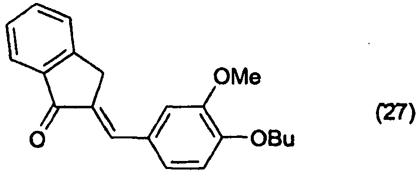
- Benzylidène Tétralone (Kao JP 04 134 043) telle que la 2-(3-méthoxy-4-butoxy-benzylidène)-3,4-dihydro-2H-naphthalen-1-one : 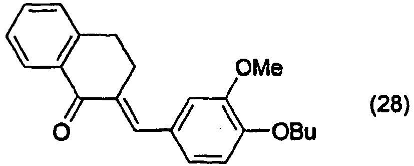
- Benzylidène Benzofuranone (Kao JP 04 134 041) telle que la 2-benzylidène-benzofuran-3-one (λₘₐₓ : 395 nm) : 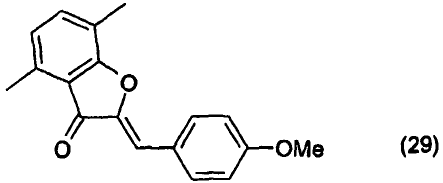
- Benzylidène Indanedione telle que la 2-(3,5-diméthoxy-4-hydroxy-benzylidène)-indan-1,3-dione : 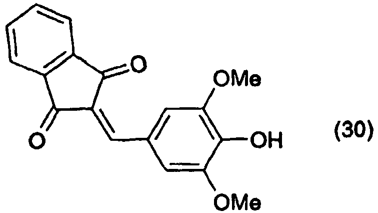
- Benzylidène Benzothiofuranone (Kao JP 04 134 043) telle que la 2-benzylidène-benzo[b]thiophen-3-one : (λₘₐₓ : 428 nm) 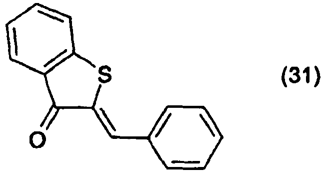
- Benzylidène Pyrazolone telle que la 4-(3-méthoxy-4-butoxy-benzylidène)-5-méthyl-2-phényl-2,4-dihydro-pyrazol-3-one : 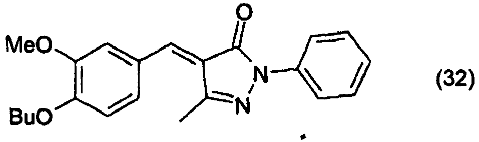
- Benzylidène Imidazolone telle que la 5-(3-méthoxy-4-butoxy-benzylidène)-2-phényl-3,5-dihydro-imidazol-4-one : 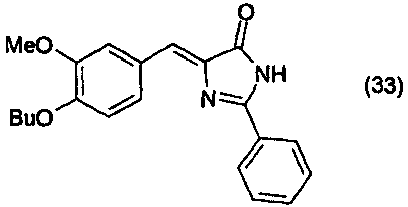
- Chalcone telle que la 1-(2-hydroxy-4-méthoxy-phényi)-3-(4'-méthoxy-phényl)-propènone : 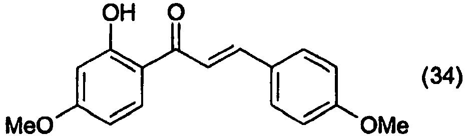
- Benzylidène One (forme tautomère filtrante des dibenzoylméthanes ; L'Oréal FR 2 506 156) telle que la 3-hydroxy-1-(2-hydroxy-4-méthoxy-phényl)-3-phényl-propènone :

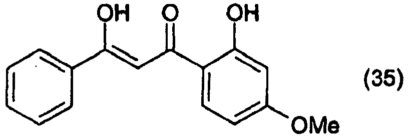

A titre d'exemples de composés de formule (I) dans laquelle n=2 utilisables selon l'invention, on peut mentionner les familles suivantes :
- Para-xylidène cétones (Kao JP 04 134 041) telle que le composé suivant (36) (λₘₐₓ 380 nm) : 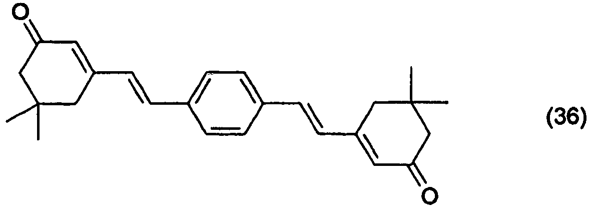
- Phénylène bis méthylidène-nor-camphre (Merck EP 0 693 471) tel que la 1,4-(2,5-diméthoxy-phénylène)-bis-{3-méthylidène-bicyclo[2.2.1]heptan-2-one}: 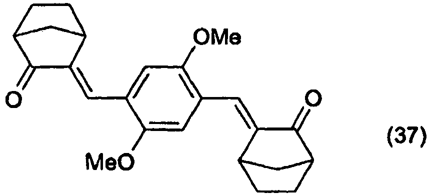
- Phénylène bis méthylidène camphre (L'Oréal FR 2 528 420) telle que la 1,4-(2,5-dihexyloxy-phénylène-bis-{3-méthylidène-1,7,7-triméthyl-bicyclo [2.2.1]heptan-2-one} :

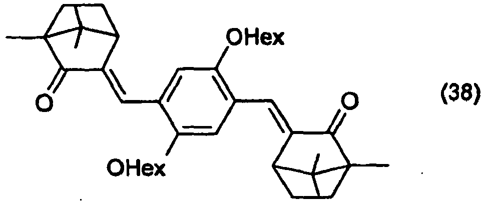

A titre de composés de formule (II), on peut mentionner les familles suivantes :
- bis benzylidène cycloalcanone (Shiseido JP 88 051 320) telle que la 2,5-bis-(4-hexyloxy-benzylidène)-cyclopentanone :

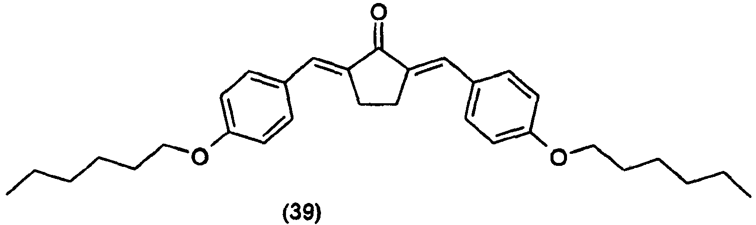

On peut également citer le 2,5-Bis-[4(3-trimethylsilanyl-propyloxy)-benzylidène]-cyclopentanone (λₘₐₓ = 395 nm) :

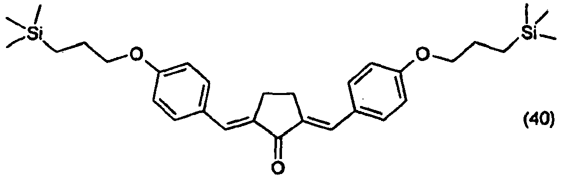

### VI les composés amino aryl vinyl-s-triazine

Parmi les composés amino aryl vinyl-s-triazine, on utilisera plus particulièrement ceux répondant à la formule (III) suivante : 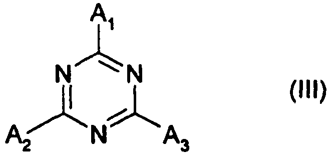 dans laquelle :
A₁, A₂ et A₃ , identiques ou différents sont choisis parmi les groupes de formules (IV) à (VIII) suivantes étant entendu qu'au moins un groupe de formules (IV) à (VII) est présent :

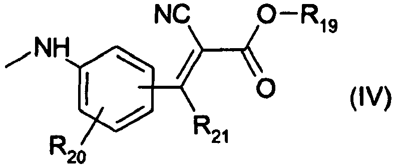

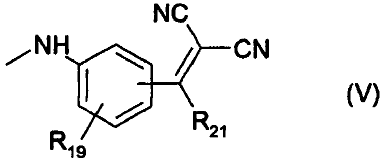

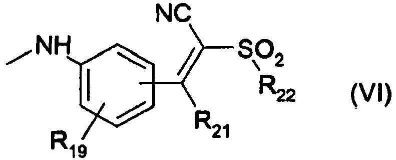

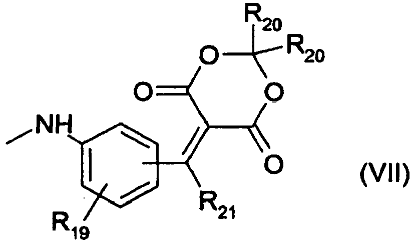

   -O-Z₁-W₁ (VIII)

   -NH-Z₂-(W₁)ₐ (IX)
dans lesquelles :
R₂₀ représente l'hydrogène, un radical alkyle en C₁-C₄, un radical alcoxy en C₁-C_{4,}
R₁₉ représente un radical alkyle en C₁-C₂₀, linéaire ou ramifié, un radical hydroxyalkyle en C₂-C₂₀, linéaire ou ramifié ou un radical alcoxy en C₁-C₂₀, linéaire ou ramifié,
R₂₁ représente l'hydrogène, le radical méthyle ou le radical phényle éventuellement substitué avec un radical alkyle en C₁-C₄ ou un radical alcoxy en C₁-C₄,
R₂₂ représente un radical alkyle en C₁-C₂₀, linéaire ou ramifié ou un radical phényle éventuellement substitué avec un radical alkyle en C₁-C₄ ou un radical alcoxy en C₁-C₄.
Z₁ représente un radical divalent assurant la liaison entre -O- et -W₁,
Z₂ représente un radical divalent lorsque a = 1 et trivalent lorsque a = 2 assurant la liaison entre -NH- et-(W₁)a,
Z₁ et Z₂ pouvant être alkylène en C₁-C₁₂, éventuellement substitué par un ou des groupements hydroxyles et pouvant contenir un ou des atomes d'oxygènes ou un ou des groupements amino et éventuellement contenant une double liaison,
W₁ représente :
   (i) soit un radical siliconé comprenant au moins une unité de formule (X) suivante 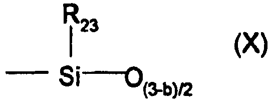 dans laquelle R₂₃ désigne un groupe hydrocarboné saturé ou insaturé, linéaire ou ramifié en C₁-C₃₀ ; un groupe hydrocarboné halogéné en C₁-C₈ ; le radical phényle ;le radical trifluoro-3,3,3 propyle ou un groupe triméthylsilyloxy, et b est égal à 1 ou 2,
   (ii) soit un radical de formule (XI) suivante :

      -SiR₂₄R₂₅R₂₆ (XI)
dans laquelle R₂₄, R₂₅, R₂₆, identiques ou différents, sont choisis parmi les radicaux alkyles et alcényles en C₁-C₈, linéaires ou ramifiés.

Les groupes A₁, A₂ et A₃ peuvent également être choisis parmi les groupements aryles substitués par des hydroxyles, des alkyles en C₁-C₂₀ linéaires ou ramifiés, des alcoxy en C1-C20, linéaires ou ramifiés ; les amino benzylidène camphre ; les amino benzotriazoles ; les groupements esters d'aminobenzoates, d'aminobenzalmalonates, d'aminosalicylates, d'amino cinnamates en C₁-C₂₀ ou d'anthranilates, linéaires ou ramifiés.

Selon une forme préférée de l'invention, W est un radical siliconé répondant à l'une des trois formules (XII) à (XIV) suivantes : 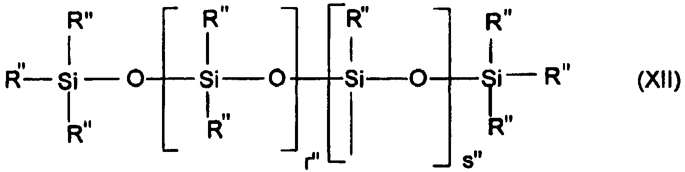 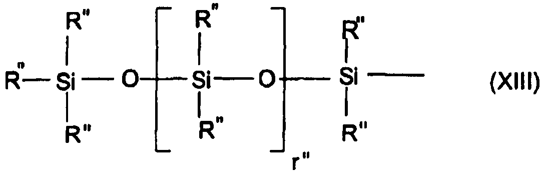 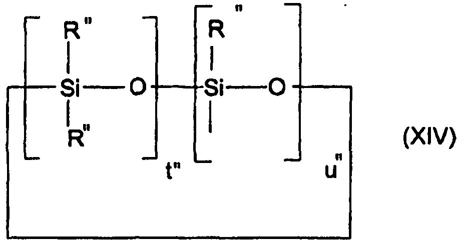 dans lesquelles :
- R", identiques ou différents sont choisis parmi les radicaux alkyles C₁-C₂₀, linéaires ou ramifiés, saturés ou insaturés, le radical phényle et le radical trifluoro-3,3,3 propyle, au moins 80% en nombre des radicaux R étant le radical méthyle,
- r" est un nombre entier choisi entre 0 et 50 inclusivement,
- s" est un nombre entier choisi entre 0 et 20 inclusivement,
- u" est un nombre entier compris entre 1 et 6 inclus,
- t" est un nombre entier entre 0 et 10 inclus,
- t" + u" est égal ou supérieur à 3.

Parmi les composés de formule (III) ci-dessus, on préfère mettre en oeuvre ceux pour lesquels le radical W répond à la formule (XII) ou à la formule (XIII), c'est-à-dire ceux pour lesquels le radical siliconé est un radical diorganosiloxanique linéaire.

Parmi les radicaux diorganosiloxaniques linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R" est alkyle et encore plus préférentiellement est méthyle,
- r" est compris entre 0 et 3 inclusivement ; s est compris entre 0 et 3 inclusivement.

Les composés de formule (III) plus particulièrement préférés sont choisis parmi :
- 2-(4'-ylamino α-cyano-cinnamate d'éthyle)-4-(4-méthoxyphényl)-6-{[1,3,3,3-tetramethyl-1-[(trimethylsilyl)oxy]disiloxanyl]propyl-3-ylamino}-s-triazine,
- 2-(4'-ylamino α-cyano-cinnamate d'éthyle)-4-(4'-ylamino benzoate de butyle)-6-{[1,3,3,3-tetramethyl-1-[(trimethylsilyl)oxy]disiloxanyl]propyl-3-ylamino}-s-triaine
- 2-(4'-ylamino-2-méthanesulfonyl-acrylonitrile)-4-(4'-ylamino benzoate de butyle)
- 6-{[1,3,3,3-tetramethyl-1-[(trimethylsilyl)oxy]disiloxanyl]propyl-3-ylamino}-s-triazine
- 2-(4'-ylamino α-cyano-cinnamate d'éthyle)-4-(4'-ylamino benzoate de butyle)-6-(triméthylsilanylméthyl-ylamino}-s-triazine
- 2,4-bis(4'-diylamino benzoate de butyle)-6-(4'-ylamino α-cyano-cinnamate d'éthyle-2 hexyle)-s-triazine
- 2,4,6-tris-(4'-ylamino α-cyano-cinnamate d'isobutyle)-s-triazine
- 2,4,6-tris-(4'-ylamino α-cyano-cinnamate d'éthyl-2 hexyle)-s-triazine
- 2,4-bis-(4'-ylamino α-cyano-cinnamate d'éthyl-2 hexyle)-6-{[1,3,3,3-tetramethyl-1-[(trimethylsilyl)oxy]disiloxany]propyl-3-ylamino}s-triazine :

Parmi les composés de formule (III), certains sont connus en soi dans la littérature chimique des composés s-triazine. Il s'agit :
- des composés de formule (III) où les 3 groupements A₁, A₂ et A₃ correspondent à la même formule (IV)
- des composés de formule (III) où A₂ et A₃ désignent un groupe de formule (IV) et A1 désigne un groupe de formule (VIII) ou (IX).

Hormis ces composés, les molécules de formule (III) sont nouvelles et constituent un objet de l'invention. Parmi les composés nouveaux de formule (III), on peut citer :
- le 2,4-bis(4'-diylamino benzoate de butyle)-6-(4'-ylamino α-cyano-cinnamate d'éthyle-2 hexyle)-s-triazine ;
- le 2-(4'-ylamino-2-méthanesulfonyl-acrylonitrile)-4-(4'-ylamino benzoate de butyle)-6-{[1,3,3,3-tetramethyl-1-[(trimethylsilyl)oxy]disiloxanyl]propyl-3-ylamino}-s-triazine.

Les composés de formule (III) peuvent être obtenus selon le schéma réactionnel ci-dessous : 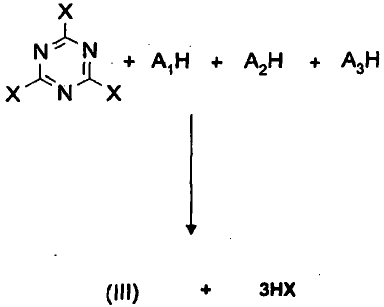 où A₁, A₂ et A₃ répondent aux définitions ci-dessus et X représente un halogène, en particulier le chlore ou le brome.

Les greffages des différents radicaux A₁, A₂ et A₃ ci-dessus sur la s-triazine peuvent se faire indépendamment les uns des autres et dans n'importe quel ordre.

De préférence, on greffe en premier lieu sur la s-triazine le radical -O-Z₁-W1 ou le radical -NH-Z₂-(W₁)ₐ correspondant aux chaînes aminosiliconées ou aminosilaniques puis les radicaux A₂ et A₃.

Dans le cas particulier des dérivés siloxaniques, ce groupement peut être introduit sur s-triazine par l'intermédiaire du dérivé amino siloxane tel que décrit dans le schéma précédent ou alors il est introduit par l'intermédiaire des dérivés amino insaturés de formules (XIV) et (XV) suivantes : 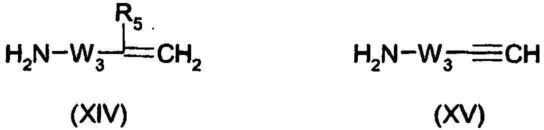 suivi par une réaction d'hydrosilylation de l'insaturation introduite par un dérivé de formule (XVI) :

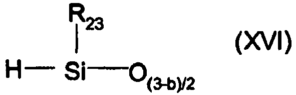

La préparation des aminosiloxanes est par exemple décrite dans GB 2 185 984. Comme aminosiloxanes convenant particulièrement bien à la préparation des composés selon l'invention, on peut citer l'aminopropyl heptaméthyl trisiloxane, l'aminoisobutyl heptaméthyl trisiloxane, ou encore les triméthylsilylamo-diméthicone telles que : le produit vendu sous la dénomination commerciale « X2-8260 » par la société DOW CORNING, d'indice d'amine 2,8 meq/gramme ; le produit vendu sous la dénomination commerciale « SLM 55051/3 » par la société WACKER, d'indice d'amine 0,47 meq/gramme ; les PDMS diméthylalkyle en C12, telles que le produit vendu sous la dénomination commerciale « SLM 23046/1 » par la société WACKER, d'indice d'amine 1,2 meq/gramme ; les polyméthylalkyl (gras) arylalkylsiloxane a, w triméthylées, telles que le produit vendu sous la dénomination commerciale « SLM 23056/2 » par la société WACKER, d'indice d'amine 1,3 meq/gramme ; les PDMS dont le radical NH2 est en position α et ω sur un site alkyle telles que les produits vendus sous les dénominations commerciales « TEGOMER A-SI 2120 », d'indice d'amine 1,95 meq/gramme et « TEGOMER A-SI 2320 », d'indice d'amine 0,86 meq/gramme, par la société GOLDSCHMIDT.

La préparation des aminosiloxanes cycliques est par exemple décrite dans l'article de A.Kopylov, Zh.Obshch. Khim., 54(2), 367-71 (1984).

La préparation des aminosilanes est par exemple décrite dans EP321174 ou l'article de J.P.Picard, Can.J.Chem, 78(11), 1363-1379 (2000).

Comme dérivés aminosilanes convenant particulièrement bien à la préparation des composés de la présente invention, on peut citer l'aminopropyl triméthylsilane, l'aminométhyltriméthylsilane et le 1,1-bis-(triméthylsilyl)méthylamine.

Les réactions ci-dessus peuvent être effectuées éventuellement en présence d'un solvant comme par exemple le toluène, le xylène ou encore un mélange acétone/eau.

Les réactions ci-dessus peuvent également éventuellement être réalisées en présence d'une base telle que la soude, les carbonates ou encore une amine.

Les composés A₁H à A₃H correspondant aux radicaux A₁ à A₃ peuvent être préparés selon des méthodes connues.

La préparation des dérivés aminés de cyano acrylate est par exemple décrite dans l'article J.Soc.Dyers Colour. (1977), 93, p126-133. Comme dérivés aminés de cyano acrylate convenant particulièrement bien à la préparation des composés de la présente invention, on peut citer l'a-cyano-4-aminocinnamate d'éthyl-2-hexyle.

Les agents filtrant les radiations lumineuses de longueur d'onde allant de 370 à 500 nm selon l'invention sont de préférence présents dans les compositions dans des concentrations allant de 0.1 à 15 % en poids et plus particulièrement de 0.1 à 10 % en poids par rapport au poids total de la composition.

Les compositions selon l'invention sont adaptées à une application topique externe. Elles peuvent se présenter sous toutes les formes galéniques appliquées sur la surface de la peau habituellement proposées en cosmétique et en dermatologie. Elles peuvent notamment comporter au moins une phase grasse et se présenter sous forme d'une solution huileuse, d'une émulsion huile-dans-eau ou eau-dans-huile ou multiple, d'une émulsion siliconée, d'une microémulsion ou nanoémulsion, d'un gel huileux ou d'un produit anhydre liquide, pâteux ou solide.

Les compositions selon l'invention peuvent être des produits cosmétiques destinés à la protection du teint naturel de la peau.

Les compositions selon l'invention peuvent être également des produits cosmétiques destinés à la protection naturelle antioxydante de la peau.

Les compositions selon l'invention peuvent être des produits dermatologiques destinés au traitement des désordres cutanés provoqués par le rayonnement solaire (photodermatoses) comme les urticaires solaires, la lucite, les tâches pigmentaires.

Les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β'-diphénylacrylate ; les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE1 9755649, EP1133980 et EP1133981 et leurs mélanges.

Comme exemples d'agents photoprotecteurs actifs dans l'UV-A et/ou l'UV-B, on peut citer désignés ci-dessus sous leur nom INCI:

### Dérivés de l'acide para-aminobenzoique:

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane:

Butyl Methoxydibenzoylmethane vendu notamment sous le nom commercial « PARSOL 1789 » par HOFFMANN LAROCHE,
Isopropyl Dibenzoylmethane,

### Dérivés cinnamiques :

Ethylhexyl Methoxycinnamate vendu notamment sous le nom commercial « PARSOL MCX » par HOFFMANN LAROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
   - Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12,
le 2-(4-diéthylamino-2-hydroxybenzoyl)-benzoate de n-hexyle

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
Polyacrylamidomethyl Benzylidene Camphor fabriqué sous le nom « MESORYL SW » par CHIMEX,

### Dérivés de benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de triazine :

Anisotriazine vendu sous le nom commercial «TINOSORB S » par CIBA SPECIALTY CHEMICALS
Ethylhexyl triazone vendu notamment sous le nom commercial «UVINUL T150 » par BASF,
Diethylhexyl Butamido Triazone vendu sous le nom commercial « UVASORB HEB » par SIGMA 3V
la 2,4,6- tris-(4'amino-benzalmalonate de diisobutyle)-s-triazine.

### Dérivés de benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés de benzaimalonate :

Polyorganosiloxanes à fonctions benzalmalonate comme le polysilicone-15 vendu sous la dénomination commerciale « PARSOL SLX » par HOFFMANN LAROCHE et leurs mélanges.

### Dérivés de 4,4-diarylbutadiène :

- 1,1-dicarboxy (2,2'-diméthyl-propyl)-4,4-diphénylbutadiène
et leurs mélanges.

Les agents photoprotecteurs organiques plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,.
Benzophenone-3,
Benzophenone-4,
Benzophenone-5,
le 2-(4-diéthylamino-2-hydroxybenzoyl)-benzoate de n-hexyle
4-Methylbenzylidene camphor,
Disodium Phenyl Dibenzimidazole Tetra-sulfonate ,
Anisotriazine,
Ethylhexyl triazone,
Diethylhexyl Butamido Triazone,
la 2,4,6- tris-(4'amino-benzalmalonate de diisobutyle)-s-triazine.
Methylene bis-Benzotriazolyl Tetramethylbutylphenol,
Drometrizole Trisiloxane,
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par :
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tiovell MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les oxydes de titane non traités peuvent par exemple être ceux vendus par la société TAYCA sous les dénominations commerciales « Microtitanium Dioxide MT 500 B » ou « Microtitanium Dioxide MT 600 B ».

Les oxydes de zinc non traités peuvent par exemple être ceux vendus par la société SUMITOMO sous la dénomination « Ultra Fine Zinc Oxide Powder », par la société PRESPERSE sous la dénomination « Finex 25 », par la société IKEDA sous la dénomination « MZO-25 » ou par la société SUNSMART sous la dénomination « Z-COTE ». Les oxydes de zinc traités peuvent par exemple être ceux vendus par la société SUNSMART sous la dénomination « Z-COTE HP 1 ».

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE O9 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan Gl 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Des exemples concrets, mais nullement limitatifs, illustrant l'invention vont maintenant être donnés.

### Exemple 1 : Synthèse de la de la 2,5-Bis-[4-(3-triméthylsilanyl-propyloxy)-benzylidène]-cyclopentanone

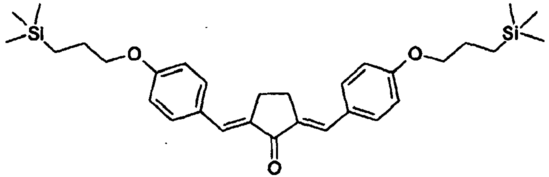

### Première étape: préparation du 4-(3-triméthylsilanyl-propyloxy)-benzaldéhyde :

Dans un mélange de 4-hydroxy benzaldéhyde (24,4 g, 0,2 mole) et de carbonate de potassium (30,4 g, 0,22 mole) dans 150 ml de DMF sec porté à 120°C sous azote, on ajoute goutte à goutte en 10 minutes du 3-chloropropyltriméthylsilane (33,14 g, 0,22 mole). On laisse 2 heures 30 minutes à 120-130°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide (0,2 mmHg), on obtient 40,5g (Rendement : 86 %) de 4-(3-triméthylsilanyl-propyloxy)-benzaldéhyde sous forme d'une huile incolore distillant à 110-114°C et utilisée telle quelle dans l'étape suivante.

### Deuxième étape: préparation du dérivé de l'exemple 1 :

Dans un mélange du produit précédent (26,2g, 0,105 mole) et de cyclopentanone (4,2 g, 0,05 mole) dans 200 ml de méthanol, sous azote, on ajoute à 35°C sous agitation goutte à goutte en 15 minutes 4,2 g de soude (0,105 mole) dissoute dans 22 ml d'eau. On porte au reflux pendant 6 heures. On refroidit, filtre Ime précipité jaune obtenu et le lave copieusement à l'eau. Après séchage, on obtient 25 g (Rendement : 96 %) du dérivé de l'exemple 1 sous forme d'un solide jaune.
- Point de fusion : 205-207°C.
- UV (CHCl₃) λₘₐₓ = 395 nm, εₘₐₓ = 49100,E1% = 940.
- Analyse élémentaire pour C₃₁H₄₄O₃Si₂
- Calculé : C71,49 H8,51 Si10,78
- Trouvé : C71,15 H8, 78 Si10,47.

### EXEMPLE 2 : Préparation du 2-(4'-ylamino α-cyano-cinnamate d'éthyle)-4-(4-méthoxyphényl)-6-{[1,3,3,3-tetramethyl-1-[(trimethylsilyl)oxy]disiloxanyl]propyl-3-ylamino}-s-trlazine :

### 1ère étape : préparation de l'ester éthylique de l'acide 3-{4-[4-chloro-6-(méthoxy-phényl)-[1,3,5] triazin-2-ylamino]-phényl}-2-cyano-acrylique :

A une solution de 2,4-dichloro-6-(4-méthoxy-phényl)-[1,3,5] triazine (6,4 g, 0,025 mole) dans 60 ml d'acétone, on ajoute à 50 °C, en alternance, goutte à goutte, une solution de l'ester éthylique de l'acide 3-(4-amino phényl)-2-cyano acrylique (5,4 g, 0,025 mole) en solution dans 50 ml d'acétone et 25 ml d' une solution aqueuse de bicarbonate de sodium (2,1 g, 0,025 mole) en 1 heure. L'agitation est maintenue 1 heure. Après essorage du précipité, lavage à l'eau et séchage, on obtient l'ester éthylique de l'acide 3-{4-[4-chloro-6-(méthoxy-phényl)-[1,3,5] triazin-2-ylamino]-phényl}-2-cyano-acrylique (7,5 g, rendement = 94 %) ayant les caractéristiques suivantes :
- solide jaune
- UV (DMSO/CH₂Cl₂) λₘₐₓ = 375 nm, εₘₐₓ = 36 850

### 2ème étape : préparation du composé de l'exemple 2 :

Le dérivé précédent (4,3 g, 0,01 mole) est mis en suspension dans 50 ml de toluène. On y ajoute l'amino-1 [1,3,3,3-tetramethyl-1-[(trimethylsilyl)oxy]disiloxanyl]-3-propane (5,6 g, 0,02 mole) et chauffe sous agitation à 90 °C pendant 2 heures sous barbotage d' azote. On évapore à sec et reprend l'huile obtenue dans de l'heptane. Le précipité obtenu est purifié par chromatographie sur colonne de silice (éluant : CH₂Cl₂). On obtient 3,2 g (Rendement : 47 %) du dérivé de l'exemple 2 sous forme d'un solide jaune vif :
- UV (Ethanol) λₘₐₓ = 390 nm, εₘₐₓ = 44 640
- Analyse élémentaire pour C₃₂H₄₆N₆O₅Si₃

| | | | | |
|---|---|---|---|---|
| calculé : | C56,61 | H6,83 | N12,38 | Si12,41 |
| trouvé : | C56,32 | H6,89 | N12,40 | Si12,60 |

### EXEMPLE 3 : Préparation du 2-(4'-ylamino α-cyano-cinnamate d'éthyle)-4-(4'-ylamino benzoate de butyle)-6-{[1,3,3,3-tetramethyl-1-[(trimethylsilyl)oxy]disiloxanyl]propyl-3-ylamino}-s-triazine :

### 1ère étape : préparation de l'ester éthylique de l'acide 3-{4-[4-chloro-6-(4-pentanoyl-phénylamino)-[1,3,5] triazin-2-ylamino]-phényl}-2-cyano-acrylique :

A une solution de 2-(4'-ylamino benzoate de butyle)-4,6-dichloro-s-triazine (19,7 g, 0,058 mole) dans 50 ml de DMF portée à 50°C, on ajoute à 50 °C, en alternance, goutte à goutte, une solution de l'ester éthylique de l'acide 3-(4-amino phényl)-2-cyano acrylique (12,5 g, 0,058 mole) en solution dans 120 ml d'acétone et 25 ml d' une solution aqueuse de bicarbonate de sodium (4,9 g, 0,058 mole) en 1 heure. L'agitation est maintenue 2 heures à 80 °C. L'acétone est éliminée et le mélange réactionnel dilué à l'eau. Le solide obtenu est filtré, lavé à l'eau et séché. On obtient l'ester éthylique de l'acide 3-{4-[4-chloro-6-(4-pentanoyl-phénylamino)-[1,3,5] triazin-2-ylamino]-phényl}-2-cyano-acrylique (12,5 g, rendement = 41 %) ayant les caractéristiques suivantes :
- solide jaune
- UV (DMSO/CH₂Cl₂) λₘₐₓ = 376 nm, εₘₐₓ = 26 870
   λₘₐₓ = 297 nm, εₘₐₓ = 28 180

### 2ème étape:

Le dérivé précédent (10,4 g, 0,02 mole) est mis en suspension dans 100 ml de toluène. On y ajoute l'amino-1 [1,3,3,3-tetramethyl-1-[(trimethylsilyl)oxy]disiloxanyl]-3-propane (11,2 g, 0,04 mole) et chauffe sous agitation à 90 °C pendant 2 heures sous barbotage d' azote. On évapore à sec et reprend l'huile obtenue dans de l'heptane. La pâte obtenue est purifiée par 2 chromatographies sur colonne de silice (éluant : Heptane/AcOEt 70 :30). On obtient 2 g (Rendement : 12 %) du produit de l'exemple 3 sous forme d'un solide jaune vif :
- UV (Ethanol) λₘₐₓ = 388 nm, εₘₐₓ = 36 000
λₘₐₓ = 301 nm, εₘₐₓ = 40 120
- Analyse élémentaire pour C₃₆H₅₃N₇O₆Si₃

| | | | | |
|---|---|---|---|---|
| calculé : | C56,59 | H6,99 | N12,83 | Si11,03 |
| trouvé: | C56,41 | H6,84 | N12,81 | Si10,82 |

### EXEMPLE 4 : Préparation du 2,4-bis-(4'-diylamino benzoate de butyle)-6-(4'-ylamino α-cyano-cinnamate d'éthyle-2 hexyle)-s-triazine:

### 1 ère étape : préparation de la 2,4-bis(4'-diylamino benzoate de butyle)-6-chloro-s-triazine :

A une solution de chlorure de cyanuryle (9,2 g, 0,05 mole) dans 100 ml de dioxane, on ajoute goutte à goutte l'aminobenzoate de butyle (9,94g, 0,0515 mole) en solution dans 15 ml de dioxane, puis 3,5 g de carbonate de potassium en solution dans 15 ml d'eau. Le milieu réactionnel est ensuite porté à 40°C et on introduit à nouveau 9,94 g d'aminobenzoate de butyle en solution dans 15 ml de dioxane puis 3,5 g de carbonate de potassium. L'agitation est ensuite maintenue 3 heures à 65°C. Le milieu réactionnel est filtré. Au filtrat, on ajoute 250 ml d'eau. Le précipité formé est essoré, séché et recristallisé dans 250 ml de toluène. On obtient 16.5g (Rendement : 66%) du dérivé attendu sous forme d'une poudre blanche.

### 2ème étape : préparation du composé de l'exemple 4 :

Le dérivé précédent (10 g, 0,033 mole) et l'ester éthyl-2 hexyle de l'acide 3-(4-amino-phényl)-2-cyano-acrylique (16,4 g, 0,033 mole) dans 100 ml de toluène sont portés au reflux pendant 2 heures. Après concentration, le résidu pâteux est repris dans un mélange isopropanol/eau 95 :5 à chaud. Le précipité jaune obtenu est séché sous vide. On obtient 12 g (Rendement : 48%) du produit de l'exemple 4 ayant les caractéristiques suivantes :
- solide jaune vif
- UV (Ethanol) λₘₐₓ = 389 nm, εₘₐₓ = 42210
λₘₐₓ = 312 nm, εₘₐₓ = 64300
- Analyse élémentaire pour C₄₃H₅₁N₇O₆,1 H₂O

| | | | | |
|---|---|---|---|---|
| calculé : | C : 66.24 | H : 6.80 | N : 12.58 | O : 14.37 |
| trouvé : | C : 66.01 | H : 6.99 | N : 12,50 | O : 14.48 |

### EXEMPLE 5 : Préparation du 2-(4'-ylamino-2-méthanesulfonyl-acrylonitrile)-4-(4'-ylamino benzoate de butyle)-6{[1,3,3,3-tetramethyl-1-[(trimethylsilyl)oxy]disiloxanyl]propyl-3-ylamino}-s-triazine

### 1ère étape : préparation du 3-(4-amino-phényl)-2-méthanesulfonyl-acrylonitrile :

Le mélange de 4-aminobenzaldéhyde oligomère (12 g, 0,1 mole), de méthanesulfonylacétonitrile (11,9 g, 0,1 mole) dispersé dans 150 ml d'éthanol en présence de quantité catalytique de diéthylamine (0,125 ml) et d'acide acétique (0,375 ml) est chauffé au reflux pendant 6 heures. Un insoluble est éliminé par filtration. Le filtrat est concentré au tiers sous vide. Le précipité obtenu est filtré et séché. On obtient 11.6g (Rendement : 52%) du dérivé attendu sous forme d'un solide orange.

### 2ème étape : préparation du 3-[4-(4,6-dichloro-[1,3,5] triazin-2-ylamino)-phényl]-2-méthanesulfonyl-acrylonitrile :

A une solution de chlorure de cyanuryle (9,2 g, 0,05 mole) dans 120 ml d'acétone on ajoute refroidit vers 5°C, on ajoute en séquence goutte à goutte une solution du dérivé précédent (11,1 g, 0,05 mole) dans 100 ml d'acétone et 100 ml d'une solution aqueuse de bicarbonate de sodium (4,2 g, 0,05 mole) en 30 minutes. On laisse revenir à température ambiante. Le précipité abondant formé est filtré et lavé à l'acétone. On obtient 10 g (Rendement: 54 %) du dérivé attendu sous forme d'une poudre jaune pâle.

### 3ème étape : préparation de l'ester butylique de l'acide 4-{4-chloro-6-[4-(2-cyano-2-methanesulfonyl-vinyl)-phénylamino]-[1,3,5]triazin-2-ylamino}-benzoïque :

Le dérivé précédent (6 g, 0,016 mole) est mis en suspension dans 50 ml de DMF. On chauffe à 60°C et ajoute de manière séquencée une solution de l'ester butylique de l'acide 4-amino benzoïque (3,1 g, 0,016 mole) dans 40 ml de DMF et 40 ml d'une solution aqueuse de bicarbonate de sodium (1,3 g, 0,016 mole). Le chauffage est poursuivi à 90°C pendant 2 heures. On refroidit. Le précipité jaune formé est filtré, lavé à l'eau, séché sous vide et utilisé tel quel dans l'étape suivante.

### 4ème étape :

Le mélange du dérivé précédent (3,8 g, 0,0072 mole) et d'amino-1[1,3,3,3-tetramethyl-1-[(trimethylsilyl)oxy]disiloxanyl]-3-propane (4 g, 0,014mole) dans 50 ml de toluène est chauffé à 90 °C pendant 2 heures sous agitation et sous barbotage d'azote. Le toluène est chassé sous vide et le résidu obtenu purifié par chromatographie sur silice (éluant : Heptane/AcOEt 70:30). On obtient 1,5 g (Rendement :26 %) du produit de l'exemple 5 ayant les caractéristiques suivantes :
- poudre jaune
- UV (Ethanol) λₘₐₓ = 389 nm, εₘₐₓ = 43590
λₘₐₓ = 300 nm, εₘₐₓ = 37 740
- Analyse élémentaire pour C₃₄H₅₁N₇O₆Si₃

| | | | | | |
|---|---|---|---|---|---|
| calculé : | C53,03 | H6.67 | N12.73 | S4.16 | Si10.94 |
| trouvé : | C52.52 | H6.71 | N12.21 | S3.83 | Si10.54 |

### EXEMPLE 6 : Préparation du 2-(4'-ylamino α-cyano-cinnamate d'éthyle)-4-(4'-ylamino benzoate de butyle)-6-(triméthy)silanylméthyl-ylamino}-s-triaxine

On chauffe vers 95 °C un mélange de l'ester éthylique de l'acide 3-{4-[4-chloro-6-(4-pentanoyl-phénylamino)-[1,3,5] triazin-2-ylamino]-phényl}-2-cyano-acrylique (1 g, 0,0019 mole) obtenu à la première étape du composé de l'exemple 2 et l'aminométhyltriméthylsilyle (0,39 g, 0,0038 mole) dans 30 ml de toluène pendant 1 heure. Le toluène est évaporé sous vide. La pâte obtenue est purifiée par 2 chromatographies sur colonne de silice (éluant : Heptane/AcOEt 50 :50). On obtient 0,25 g (Rendement : 20 %) du composé final sous forme d'une poudre jaune vif et dont le spectre RMN du proton est conforme à la structure attendue :
- UV (Ethanol) λₘₐₓ = 389 nm, εₘₐₓ = 30300
   λₘₐₓ = 303 nm, εₘₐₓ = 39820

### EXEMPLE 7 :Préparation du 2,4,6-tris-(4'-ylamino α-cyano-cinnamate d'isobutyle)-s-triazine :

### 1ère étape : préparation de l'ester isobutylique de l'acide 3-(4-amino-phényl)-2-cyano-acrylique :

Le 4-aminobenzaldéhyde oligomère (36,3 g, 0,3 mole) est mis en suspension dans 250 ml d'isobutanol. On y introduit le cyano acetate d'isobutyle (42,3 g, 0,3 mole) et le catalyseur constitué de diéthylamine (0,375 ml) et d'acide acétique (1,12 ml).On chauffe 3 heures au reflux. Un léger insoluble est filtré à chaud. Après refroidissement du filtrat, on filtre le précipité orange formé. Après séchage, on obtient 52,6 g (Rendement : 72 %) du dérivé attendu sous forme d'un solide orange.

### 2ème étape:

Le chlorure de cyanuryle (37 g, 0,02 mole) est mis en suspension dans le toluène à une température d'environ 10°C sous barbottage d'argon. On y ajoute le dérivé précédent (14,6 g, 0,06 mole) et chauffe progressivement le mélange réactionnel vers 45°C. On maintient la réaction vers 60°C pendant 1 heure puis au reflux du toluène pendant 2 heures. L'acide chlorhydrique formé est piégé dans un flacon laveur contenant une solution de soude. Le mélange réactionnel est refroidit et le précipité jaune formé est filtré et séché. On obtient 8,6 g (Rendement : 53%) du dérivé de l'exemple 7 ayant les caratéristiques suivantes :
- solide jaune vif
- UV (Ethanol) λₘₐₓ = 390 nm, εₘₐₓ = 88630

### Analyse élémentaire pour C₄₅H₄₅N₉O₆

| | | | | |
|---|---|---|---|---|
| calculé : | C : 66.90 | H : 5.61 | N : 15.60 | O : 11.88 |
| trouvé : | C : 66.35 | H : 5.70 | N : 15,43 | O : 11.99 |

### EXEMPLE 8 : Préparation du 2,4,6-tris-(4'-ylamino α-cyano-cinnamate d'éthyl-2 hexyle)-s-triazine

### 1ère étape : préparation de l'ester éthyl-2 hexyle de l'acide 3-(4-amino-phényl)-2-cyano-acrylique :

Le 4-aminobenzaldéhyde oligomère (36,3 g, 0,3 mole) est mis en suspension dans 450 ml d'isobutanol. On y introduit le cyano acetate d'éthyl-2 hexyle (59,1 g, 0,3 mole) et le catalyseur constitué de diéthylamine (0,375 ml) et d'acide acétique (1,12 ml).On chauffe 5 heures au reflux. Un deuxième ajout de catalyseur a été effectué et le mélange réactionnel est chauffé au reflux 4 heures de plus. Un léger insoluble est filtré à chaud. Après refroidissement du filtrat, on le concentre à un volume de 300 ml et on laisse cristalliser à froid. Après séchage, on obtient 59,6 g (Rendement : 66 %) du dérivé attendu sous forme d'un solide orange.

### 2ème étape :

Le chlorure de cyanuryle (37 g, 0,02 mole) est mis en suspension dans le toluène à une température d'environ 10°C sous barbottage d'argon. On y ajoute le dérivé précédent (18 g, 0,06 mole) et chauffe progressivement le mélangé réactionnel vers 45°C. On maintient la réaction vers 60°C pendant 1 heure puis au reflux du toluène pendant 2 heures. L'acide chlorhydrique formé est piégé dans un flacon laveur contenant une solution de soude. Le mélange réactionnel est refroidit et le précipité jaune formé est filtré et séché. On obtient 18,8 g (Rendement : 96 %) du dérivé de l'exemple 8 ayant les caratéristiques suivantes :
- solide jaune vif
- UV (Ethanol) λₘₐₓ = 398 nm, εₘₐₓ = 88630

### Analyse élémentaire pour C₅₇H₆₉N₉O₆, 1 H2O

| | | | | |
|---|---|---|---|---|
| calculé : | C : 68.86 | H : 7.20 | N : 12.68 | O : 11.26 |
| trouvé : | C : 68.01 | H : 7.02 | N : 12,64 | O : 10.63 |

### EXEMPLE 9 : Préparation du 2,4-bis-(4'-ylamino α-cyano-cinnamate d'éthyl-2 hexyle)-6-{[1,3,3,3-tetramethyl-1-[(trimethylsilyl)oxy]disiloxanyl]propyl-3-ylamino}-s-triazine :

### 1ère étape : préparation de la 2,4-dichloro-6-{[1,3,3,3-tetramethyl-1-[(trimothylsilyl)oxy]disiloxanyl]propyl-3-ylamino}-s-triazine:

A une solution de chlorure de cyanuryle (25 g, 0,136 mole) dans 250 ml d'acétone, on ajoute goutte à goutte à 0°C l'amino-1[1,3,3,3-tetramethyl-1-[(trimethylsilyl)oxy]disiloxanyl]-3-propane (41,7 g, 0,149 mole) et une solution de bicarbonate de sodium (11,4 g, 0,136 mole) dans 120 ml d'eau de telle sorte que le pH se situe entre 3 et 5. En fin d'introduction, le pH est 6,5. L'agitation est ensuite maintenue 1 heure 30 minutes à 10°C, puis laissé à température du labo. Le milieu réactionnel est filtré. Une huile décante. L'acétone est évaporée sous vide. La phase inférieure est récupérée et on laisse cristalliser. Après séchage, on obtient 55,2 g (Rendement : 95 %) du dérivé attendu sous forme d'une poudre blanche (Point de fusion : 59°C).

### 2ème étape:

Le dérivé précédent (2 g, 0,00468 mole) est dissout dans 50 ml de toluène. On y ajoute l'ester éthyl-2 hexyle de l'acide 3-(4-amino-phényl)-2-cyano-acrylique obtenu à la première étape du dérivé 7 (2.81 g, 0,00935 mole) et chauffe à 95 °C pendant 5 heures. Le solvant est évaporé et la gomme obtenue est purifiée par passage sur colonne de silice (éluant : Heptane/EtOAc 80 :20). On obtient 2,47 g (Rendement : 55%) du dérivé de l'exemple 9 ayant les caractéristiques suivantes
- spectre RMN du proton conforme à la structure attendue
- solide jaune vif
- UV (Ethanol) λₘₐₓ = 400 nm, εₘₐₓ = 76820

### Exemple 10: Emulsion eau dans huile contenant des oxydes de fer.

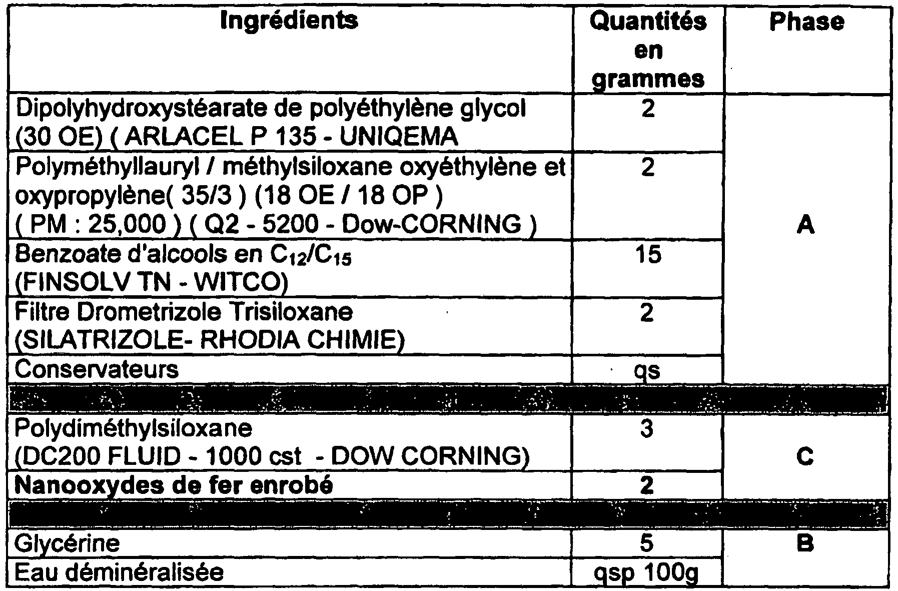

### Mode opératoire

On pèse les constituants de la phase A dans le bécher final. On pèse les constituants de la phase B dans un bécher annexe. On chauffe les phases A et B vers 80°C. On met la phase A sous agitation, on introduit lentement la phase B. On maintient l'agitation pendant 10mn. On ajoute la phase C. On homogénéise. On arrête l'agitation après obtention d'une émulsion lisse et brillante.

### Exemple 11: Emulsion huile dans eau contenant un dérivé de triazine de formule (III)

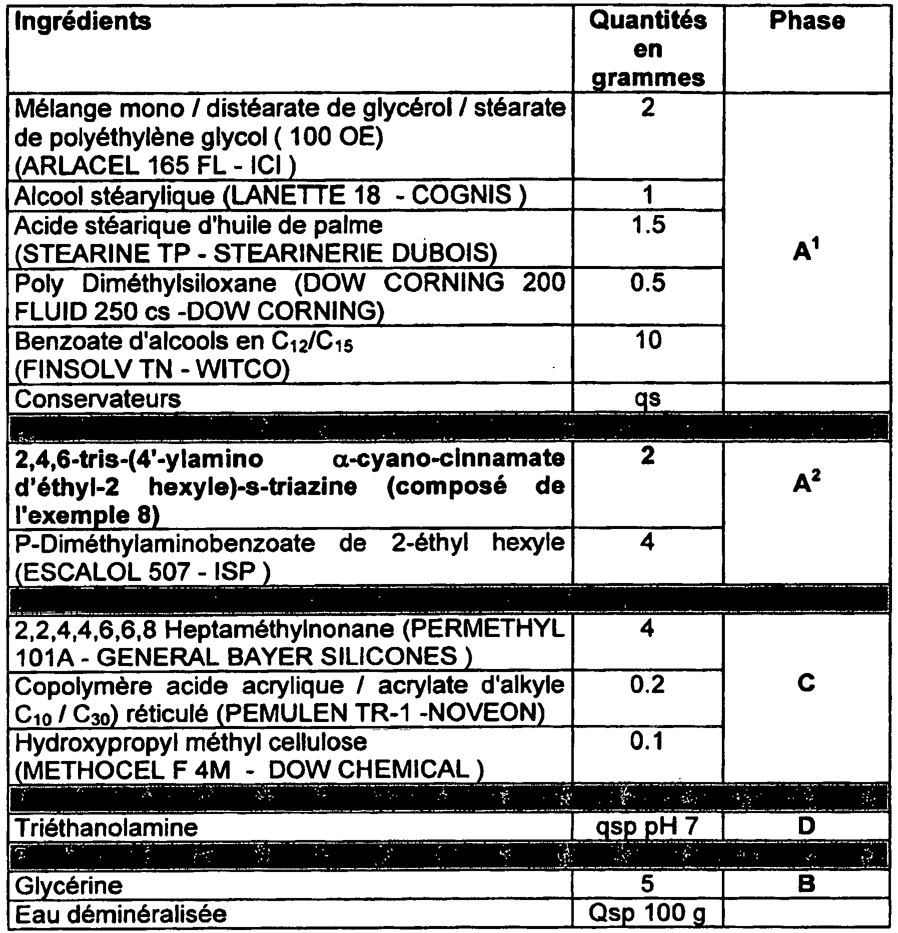

### Mode opératoire

On homogénéise la phase A² à l'aide du broyeur MM200 de Retsch aux conditions suivantes : 2billes de 1,5cm de diamètre, agitation = 20mn , fréquence 30. On introduit la phase A¹ dans le bécher final. On ajoute la phase A². On pèse la phase B dans un bécher annexe. On chauffe les phases (A¹ + A²) et B à 80°C. On met sous agitation la phase B et on ajoute les phases (A¹ + A²). On maintient l'agitation pendant 10mn. Vers 50°C, on ajoute la phase C. On homogénéise et on introduit la phase D. On laisse refroidir jusqu'à 25°C.

### Exemple 12: Emulsion huile dans eau contenant un composé quinonique : la lawsone.

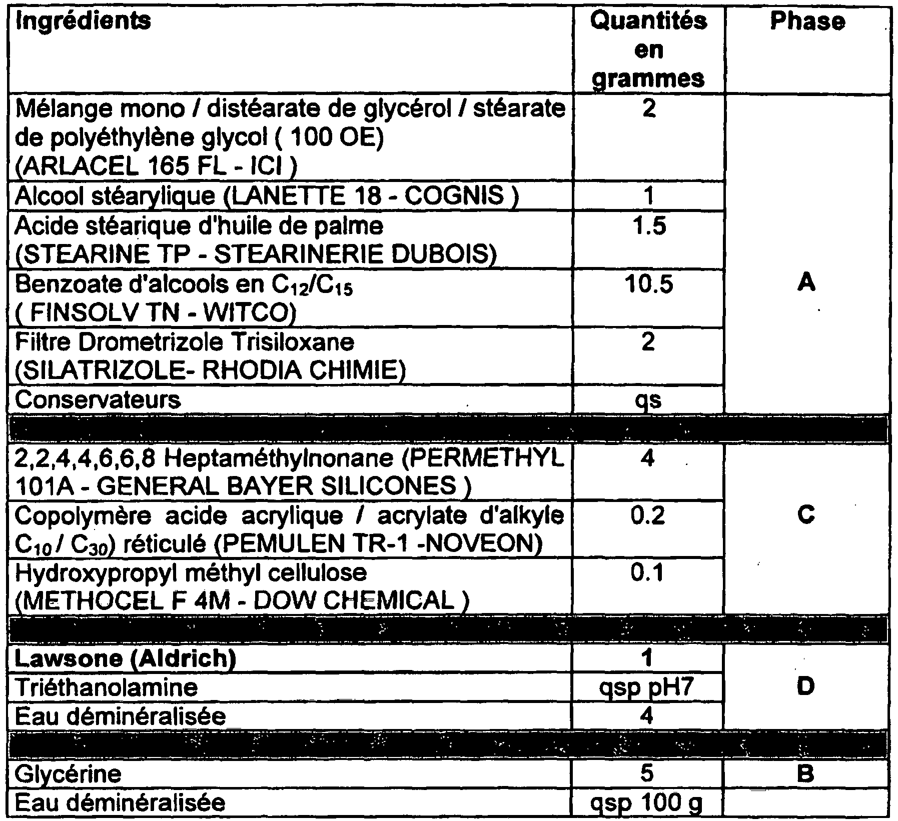

### Mode opératoire

On pèse la phase A dans le bécher final. On pèse la phase B dans un bécher annexe. On chauffe les phases A et B jusqu'à dissolution complète (80°C). On met sous agitation la phase B et on verse la phase A. On maintient l'agitation pendant 10mn. Vers 50°C, on ajoute la phase C. On homogénéise et on introduit la phase D, préalablement solubilisée. On homogénéise et on laisse refroidir jusqu'à 25°C.

### Exemple 13: Emulsion huile dans eau contenant un composé azoïque : la tartrazine.

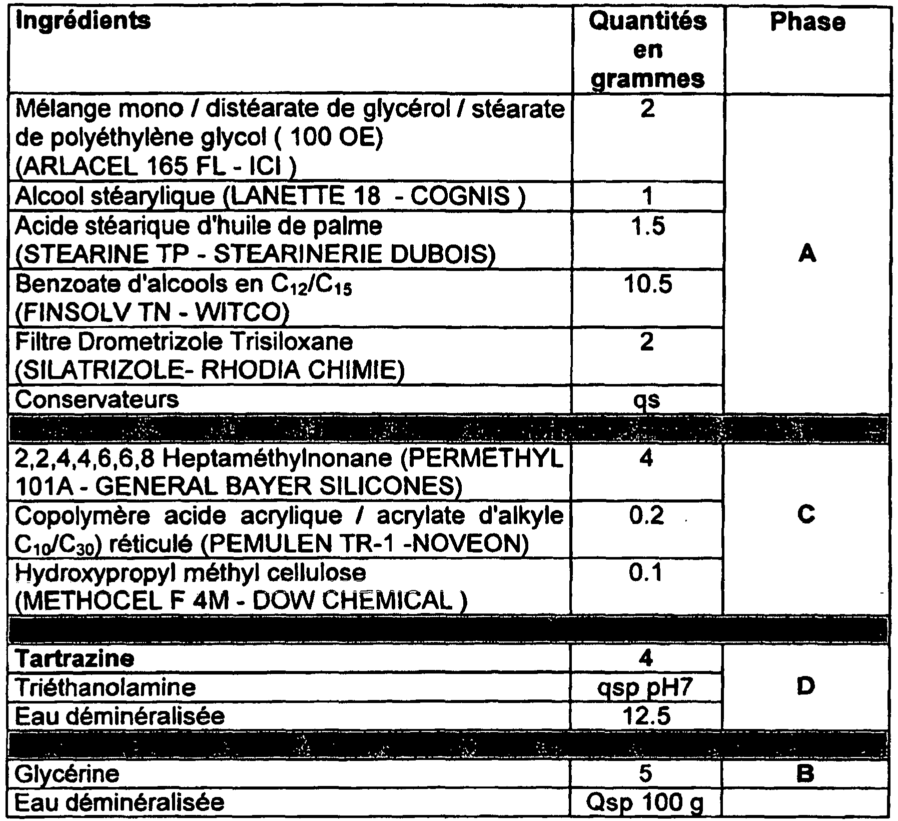

### Mode opératoire

On pèse la phase A dans le bécher final. On pèse la phase B dans un bécher annexe. On chauffe les phases A et B jusqu'à dissolution complète (80°C). On met sous agitation la phase B et on verse la phase A. On maintient l'agitation pendant 10mn.Vers 50°C, on ajoute la phase C. On homogénéise et on introduit la phase D, préalablement solubilisée. On homogénéise et on laisse refroidir jusqu'à 25°C.

### EVALUATION DE L'EFFET PROTECTEUR DES COMPOSITIONS SELON L'INVENTION SUR LES CAROTENOIDES ENDOGENES SOUS EXPOSITION DE RADIATIONS LUMINEUSES DE LONGUEUR D'ONDE ALLANT DE 370 A 500 NM.

Cette évaluation est réalisée au travers d'un test de décoloration du β-carotène basé sur le principe que le β-carotène irradié par la composante bleue de la lumière solaire se dégrade. Cette dégradation se traduit par la décoloration que l'on suit au chromamètre Minolta par sa composante b*.

### Conditions expérimentales

Les expositions en lumière bleue sont réalisées au Sun-Test CPS+ de la société Heraus à 30 J/cm² (exprimée en UVA appliqués)

On réalise une préparation extemporanée de la solution de β-carotène dans le Mygliol 812 à 0.05%. On étale 2 gouttes de la solution sur un papier filtre (à l'intérieur d'un oeillet); on laisse sécher à température ambiante et à l'abri de la lumière.

On étale les formules à tester à raison de 2 mg/cm2 sur des plaques en quartz (5 cm x 5 cm), laisser sécher 30 minutes à TA et à l'abri de la lumière. On dépose la plaque de quartz contenant la formule sur le papier filtre imprégné de β-carotène et on irradie le tout au Sun-Test CPS+

Les valeurs de b* sont relevées au chromamètre Minolta après une exposition de 30J/cm2 mesurée dans l'UVA.

### Compositions testées

On compare chacune des compositions des exemples 12, 13, 14 et 15 telles que définies ci-dessus contenant respectivement comme agent filtrant les radiations de longueur d'onde allant de 370 à 500 nm : un dérivé de triazine de formule (III), la lawsone, la tartrazine et la lutéoline avec une composition placébo de support identique ne contenant pas d'agent filtrant.

### Résultats

On calcule le pourcentage d'amélioration de la stabilité du β-carotène vis à vis de la décoloration induite par la lumière entre 400 et 450 nm obtenu par chaque formulation testée par rapport à la même formulation placébo ne contenant pas d'agent filtrant ladite lumière.

Les résultats obtenus sont indiqués dans le tableau ci-dessus.

**Tableau 1**

| **Composition testée** | **% d'amélioration de la stabilité du β-carotène vis à vis de la décoloration induite par la lumière entre 400 et 450 nm** |
|---|---|
| **Exemple 11 (composé amino aryl vinyl-s-triazine)** | + 40% |
| **Exemple 12 (lawsone)** | +10.3% |
| **Exemple 13 (tartrazine)** | + 41% |

## Revendications

1. Procédé cosmétique pour préserver le teint naturel, et la protection antioxydante naturelle de la peau, apportés par les caroténoïdes endogènes présents dans la peau, **caractérisé en ce qu'**il consiste à appliquer sur la peau une composition comprenant au moins un agent filtrant les radiations lumineuses de longueur d'onde allant de 370 à 500 nm choisi parmi
(v) les carbohydrates ;
(vi) les pigments minéraux jaunes ou jaunes oranges ;
(vii) les composés azoïques ou quinoniques ;
(viii) les colorants dérivés nitrés benzéniques ;
(ix)les composés dérivés d'aryl vinylène cétone ;
(x) les composés amino aryl vinyl-s-triazine ;
et leurs mélanges

2. **Procédé** selon la revendication 1, où les carbohydrates sont choisis parmi les produits colorés jaune issus du chauffage ou oxydation de mono ou polysaccharides.

3. **Procédé** selon la revendication 2, le carbohydrate est le caramel.

4. Procécé selon la revendication 1, où les pigments minéraux jaunes ou oranges sont choisis parmi les pigments d'oxyde de fer et plus particulièrement les nanopigments de taille de particule élémentaire inférieure à 100 nm.

5. **Procédé** selon la revendication 2, où les composés quinoniques sont choisis parmi les naphtoquinones jaune ou jaune-orange et leur produits d'oxydation ; les anthraquinones et leur produits d'oxydation jaune ou jaune-orange.

6. **Procécé** selon la revendication 5, où les composés quinoniques sont choisis parmi la juglone et la lawsone.

7. **Procédé** selon la revendication 1, où le composé azoïque est la tartrazine.

8. **Procédé** selon la revendication 1, où le composé quinonique ou azoïque est choisi parmi ceux de formule (1) ou (2) ou (3) : 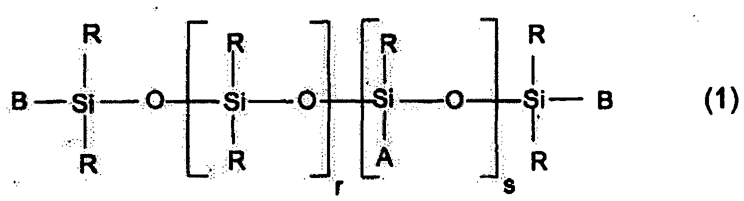 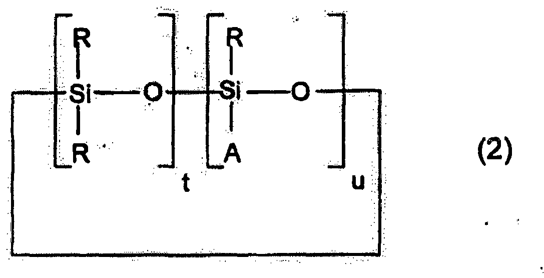 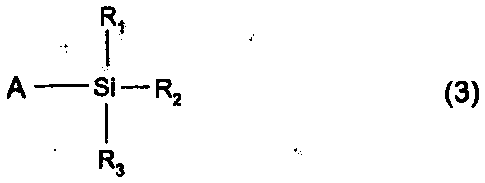 dans lesquelles, pour les formules (1) et (2) :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et
- t est un nombre entier compris entre 0 et 10 inclusivement,
étant entendu que t + u est égal ou supérieur à 3,
- R₁, R₂, R₃, identiques ou différents, sont choisis parmi les radicaux alkyles et alcényles en C₁-C₈, linéaires ou ramifiés, saturés ou insaturés,
- les symboles A désignent identiques ou différents, un radical de formules (4a) (4b) (4c) ou (4d) suivante : 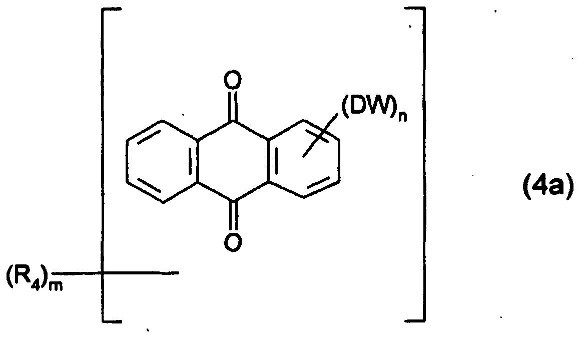 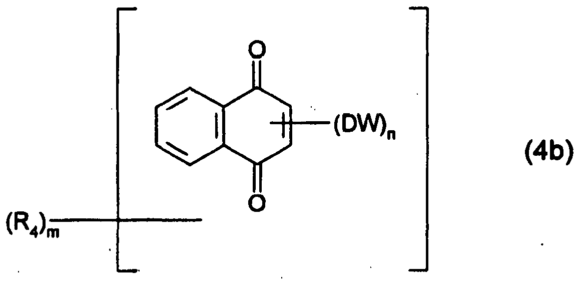 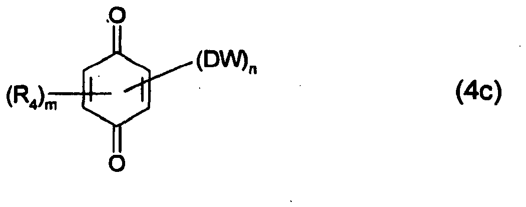 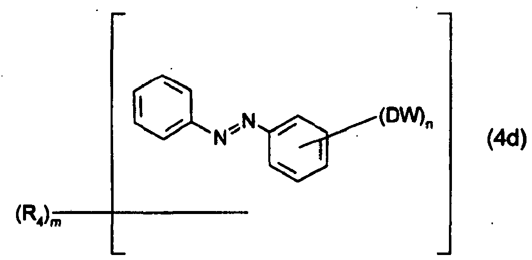
- R₄, identiques ou différents, représentent un radical alkyl, linéaire ou ramifié en C₁-C₆, un radical OH, alcoxy en C₁-C₄, hydroxyalkyl(C₁-C₄), COOH, CONH₂, CN, SO₃H, halogène, N02, NR₅R₆ dans lequel R₅ et R₆, identiques ou différents, désignent un atome d'hydrogène, un radical alkyle en C₁-C₈, ou hydroxyalkyl(C₁-C₄), ou aminoalkyl(C₁-C₄), ou forment ensemble avec l'atome d'azote auquel ils sont attachés un hétérocycle à 5 ou 6 chaînons éventuellement interrompu par un atome d'oxygène ou de soufre,
- m est un nombre entier compris entre 0 et 2 inclusivement,
- n est un nombre entier égal à 1 ou 2,
- D est est un radical -SO₂NH-, -CONH-, -O-, ou un radical -NR₇- dans lequel R₇ est H ou CH₃,
- W est un radical divalent de formule (5) : 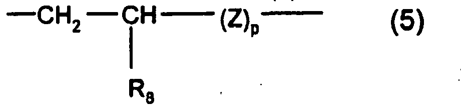 ou de formule (6) :
-HC=CH-(Z)ₚ- (6)
dans lesquelles
- R₈ désigne un atome d'hydrogène, un radical hydroxyle, un radical alkyl en C₁-C₈, linéaire ou ramifié, saturé ou insaturé,
- Z est un radical alkylène en C₁-C₆, linéaire ou ramifié éventuellement substitué par un radical OH ou un radical alcoxy en C₂-C₈ linéaire ou ramifié, saturé ou insaturé,
- p est un nombre entier égal à 0 ou 1.

9. **Procédé** selon la revendication **8**, où le composé de formule (1), (2) ou (3) est choisi parmi les composés suivants :
- 4-(4-diméthylaminophénylazo)-N-[3-[1,3,3,3-tétraméthyl-1-[(triméthylsilyl)-oxy]-disiloxanyl]-propyl]-benzènesulfonamide ;
- 4-(4-diméthylaminophénylazo)-N-[3-[1,3,3,3-tétraméthyl-1-[(triméthylsilyl)-oxy]-disiloxanyl]propyl]-benzamide
- 2-(4-méthoxy-2-nitrophénylazo)-5-(3-triméthylsilanyl-propoxy)-phénol ;
- 2,5-bis-[3-[1,3,3,3-tétraméthyl-1-[(triméthylsilyl)-oxy]-disiloxanyl]-propylamino]-benzoquinone ;
- 2-chloro-3-[3-[1,3,3,3-tétraméthyl-1-[(triméthylsilyl)-oxy]-disiloxanyl]-propyl-amino]-[1,4]-naphtoquinone ;
- 2-[3-[1,3,3,3-tétraméthyl-1-[(triméthylsilyl)-oxy]-disiloxanyl]-propylamino]-[1,4]-naphtoquinone ;
- 1-hydroxy-4-[3-[1,3,3,3-tétraméthyl-1-[(triméthylsilyl)-oxy]-disiloxanyl]-propyl-amino]-anthraquinone ;
- 1,4-di-[3-[1,3,3,3-tétraméthyl-1-[(triméthylsilyl)-oxy]-disiloxanyl]-propyl-amino]-anthraquinone.

10. **Procédé** selon la revendication 1, où le composé nitré benzénique est choisi parmi ceux de formule de formule (15) ou (16) : 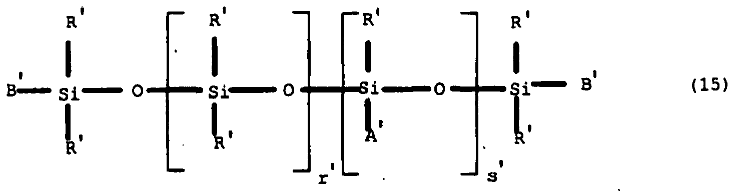 ou 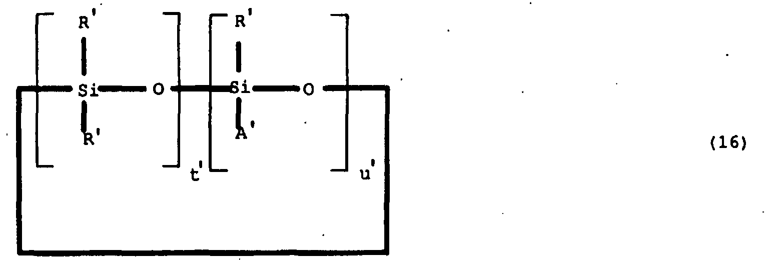 dans lesquelles :
- R', identiques ou différents, sont choisis parmi les radicaux alkyles linéaires ou ramifiés en C₁-C₁₀, phényle et trifluoro-3,3,3 propyle, au moins 80% en nombre des radicaux R' étant méthyle,
- B', identiques ou différents, sont choisis parmi les radicaux R' ci-dessus et le radical A défini ci-après,
- r' est un nombre entier compris entre 0 et 50 inclusivement, et s' est un nombre entier compris entre 0 et 20 inclusivement, avec la condition que si s' est nul alors au moins l'un des deux symboles B désigne A,
- u' est un nombre entier compris entre 1 et 6 inclusivement, et t' est un nombre entier compris entre 0 et 10 inclusivement, étant entendu que t' + u' est égal ou supérieur à 3,
- et le symbole A' désigne un radical monovalent lié directement à un atome de silicium, et qui répond à la formule (17) suivante : 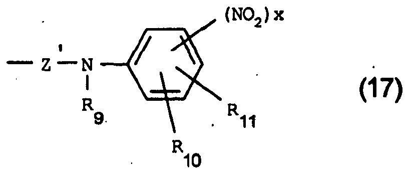
- Z' est le radical divalent : 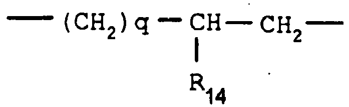
ou hydrogène,
- x est 1 ou 2,
- q représente un nombre entier compris entre 0 et 10, inclusivement,
- R₉ représente l'hydrogène ou un radical alkyle en C₁-C₄,
- R₁₀ représente l'hydrogène ou un radical alkyle en C₁-C₄, ou le radical divalent Z' défini ci-dessus,
- R₁₁ représente l'hydrogène ou un radical NR₁₂R₁₃ dans lequel R₁₂ et R₁₃ représentent l'hydrogène ou un radical alkyle en C₁-C₄, un radical mono- ou dihydroxyalkyle en C₂-C₄ ou le radical divaient Z,
- R₁₄ représente l'hydrogène, un radical OH ou halogène, un radical alkyle en C₁-C₄, ou un radical alkoxy en C₁-C₄.

11. **Procédé** selon la revendication **10**, où le composé de formule (15) ou (16) est choisi parmi les composés suivants :

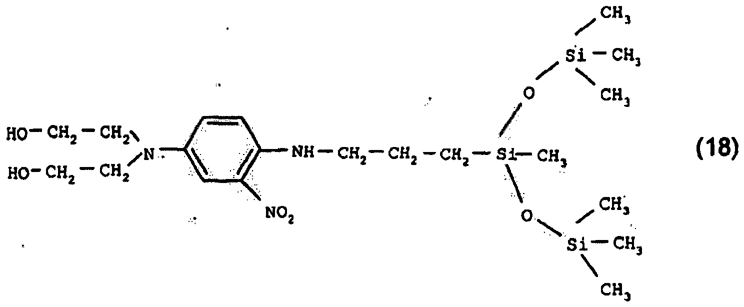

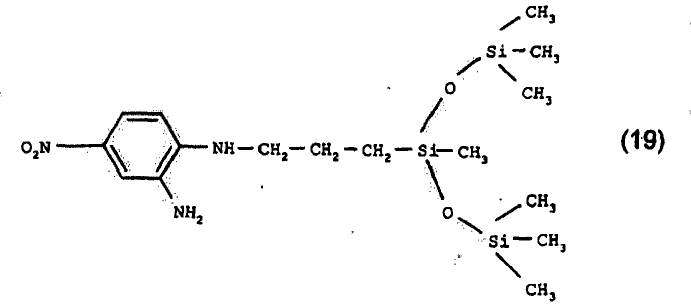

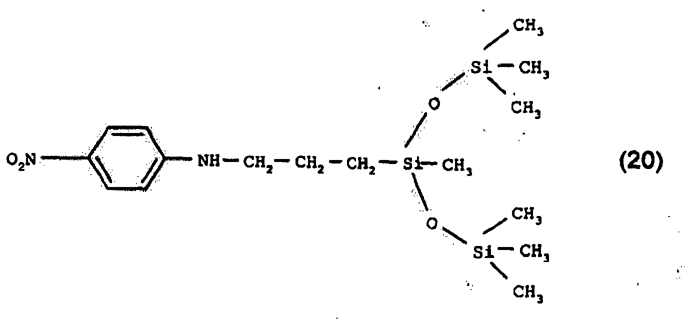

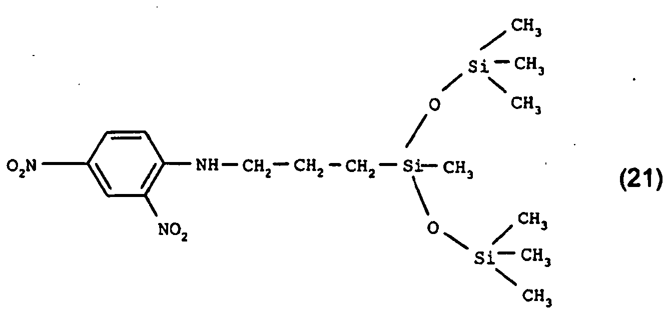

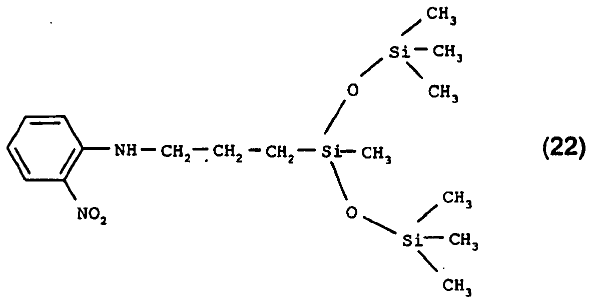

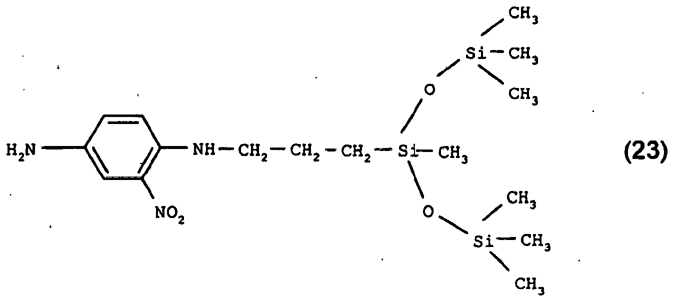

12. Procédé selon la revendication 1, où le composé aryl vinylène cétone est choisi parmi ceux de formule (I) et (II) suivantes : 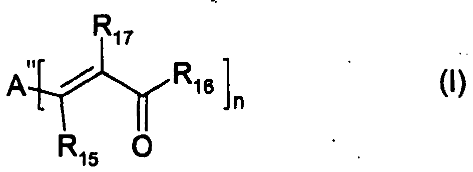 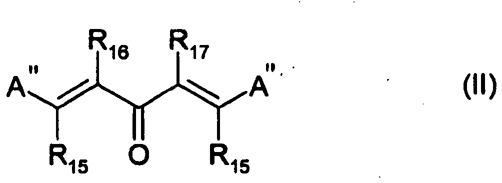 dans lesquelles :
n = 1 ou 2,
A", dans la formule (I) lorsque n=1 ou dans la formule (II), est un radical aryle choisi parmi les formules (a) à (d) suivantes, ou dans la formule (I) lorsque n=2, est un radical choisi parmi les formules (e) à (h) suivantes : 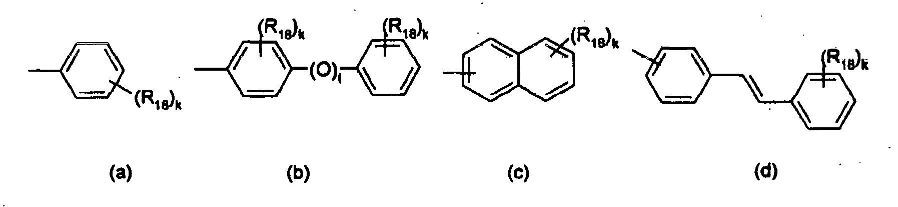 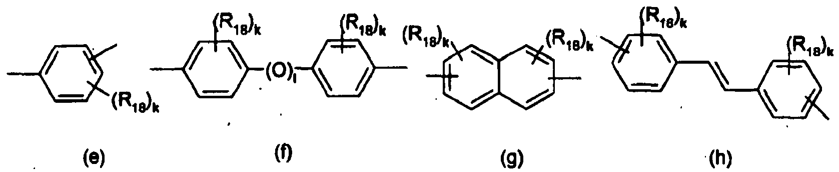
dans lesquelles :
- chacun des symboles R₁₈ représente indépendamment un groupe OH ; un atome d'halogène; un groupe alkyle en C₁-C₆, linéaire ou ramifié et contenant éventuellement un atome de silicium ou un groupement siloxanique ; un groupe alcoxy en C₁-C₆, linéaire ou ramifié et contenant éventuellement un atome de silicium ou un groupement siloxanique ; un groupe alcoxycarbonyle en C₁-C₅, linéaire ou ramifié ou un groupe alkylsulfonamide en C₁-C₆, linéaire ou ramifié et contenant éventuellement un atome de silicium, un groupement siloxanique ou une fonction aminoacide ;
- k représente un nombre entier compris entre 0 et 3 inclus ;
- l représente 0 ou 1;
- R₁₅ représente l'hydrogène ou un groupe OH ;
- R₁₆ représente l'hydrogène, un groupe alkyle en C₁-C₆, linéaire ou ramifié et contenant éventuellement un atome de silicium ou un groupement siloxanique, un groupe cyano, un groupe alkylsulfonyle en C₁-C₆, un groupe phénylsulfonyle,
- R₁₇ représente un groupe alkyle en C₁-C₆, linéaire ou ramifié et contenant éventuellement un atome de silicium, un groupement siloxanique ou un groupe phényle pouvant former un bicycle et éventuellement substitué par un ou deux radicaux R₁₈ tels que définis précédemment,
- R₁₆ et R₁₇ peuvent former ensemble un reste hydrocarboné en C₂-C₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium, un groupement siloxanique ou une fonction aminoacide ; à condition que lorsque n=1, R₁₆ et R₁₇ ne forment pas un noyau camphre.

13. **Procédé** selon la revendication **12**, où le composé de formule (I) est choisi parmi:
- la 1-(2,4,5-triméthoxy-phényl)-4,4-diméthyl-pent-1-èn-3-one : 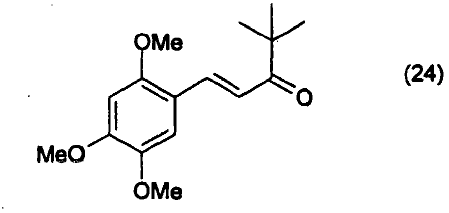
- la 3-(3-éthoxy-4-butoxy-benzylidène)-2,3,4a,8a-tétrahydro-chromèn-4-one : 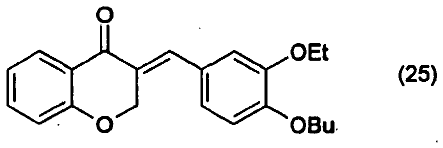
- la 3-(4-méthoxy-benzylidène)-2,3,4a,8a-tétrahydro-chromèn-thione : 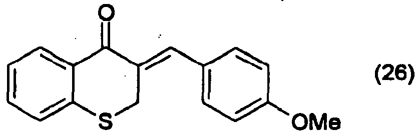
- la 2-(3-méthoxy-4-butoxy-benzylidène)-indan-1-one : 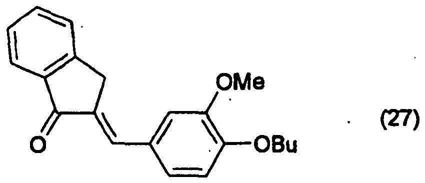
- la 2-(3-méthoxy-4-butoxy-benzylidène)-3,4-dihydro-2H-naphthalen-1-one : 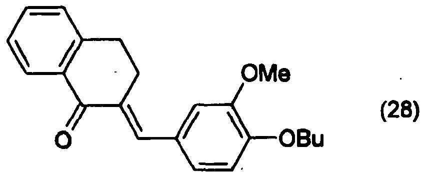
- la 2-benzylidène-benzofuran-3-one : 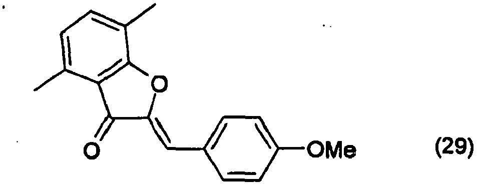
- la 2-(3,5-diméthoxy-4-hydroxy-benzylidène)-indan-1,3-dione : 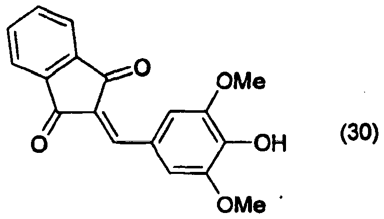
- la 2-benzylidène-benzo[b]thiophen-3-one : 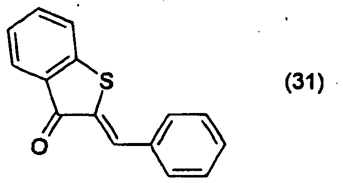
- la 4-(3-méthoxy-4-butoxy-benzylidène)-5-méthyl-2-phényl-2,4-dihydro-pyrazol-3-one : 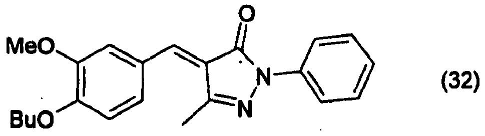
- la 5-(3-méthoxy-4-butoxy-benzylidène)-2-phényl-3,5-dihydro-imidazol-4-one : 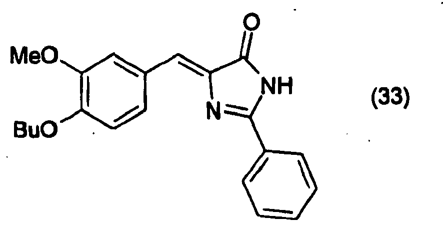
- la 1-(2-hydroxy-4-méthoxy-phényl)-3-(4'-méthoxy-phényl)-propènone : 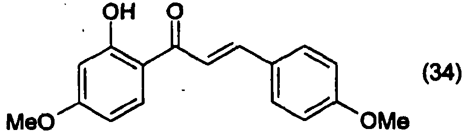
- la 3-hydroxy-1-(2-hydroxy-4-méthoxy-phényl)-3-phényl-propènone : 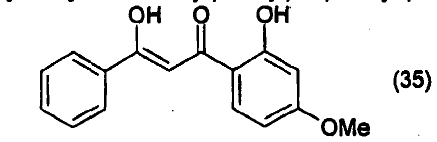
- le composé Para-xylidène suivant : 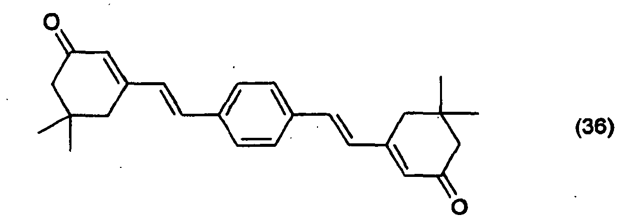
- la 1,4-(2,5-diméthoxy-phénylène)-bis-{3-méthylidène-bicyclo[2.2.1]heptan-2-one} : 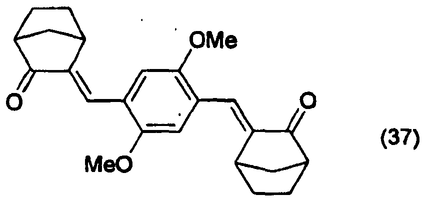
- la 1,4-(2,5-dihexyloxy-phénylène-bis-{3-méthylidène-1,7,7-triméthyl-bicyclo [2.2.1]heptan-2-one} :

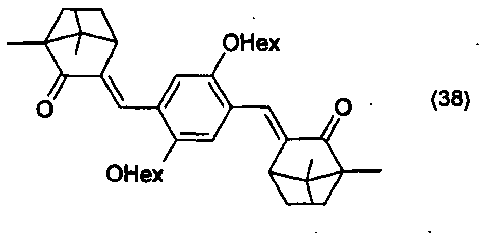

14. **Procédé** selon la revendication **13**, où le composé de formule (II) est choisi parmi
- la 2,5-bis-(4-hexyloxy-benzylidène)-cyclopentanone ;
- le 2,5-Bis-{4-[[1,3,3,3-tetramethyl-1-[(trimethylsilyl)oxy]disiloxanyl]-3-méthyl-propyloxy]-benzylidène}-cyclopentanone

15. **Procédé** selon la revendication **1**, où les composés amino aryl vinyl-s-triazine répondent à la formule (III) suivante : 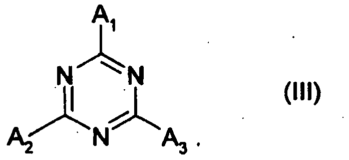 dans laquelle :
les groupes A₁, A₂ et A₃ , identiques ou différents sont choisis parmi les groupes de formules (IV) à (VIII) suivantes étant entendu qu'au moins un groupe de formules (IV) à (VII) est présent : 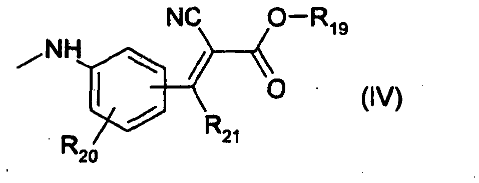 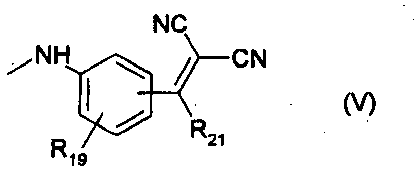 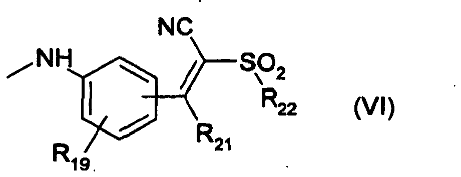 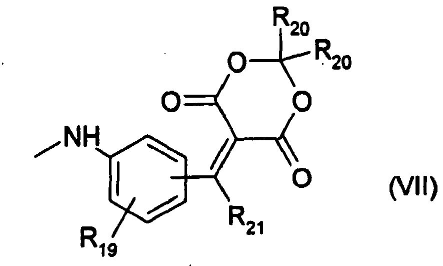
-O-Z₁-W₁ (VIII)
-NH-Z₂-(W₁)ₐ (IX)
dans lesquelles :
R₂₀ représente l'hydrogène, un radical alkyle en C₁-C₄, un radical alcoxy en C₁-C₄,
R₁₉ représente un radical alkyle en C₁-C₂₀, linéaire ou ramifié, un radical hydroxyalkyle en C₂-C₂₀, linéaire ou ramifié ou un radical alcoxy en C₁-C₂₀, linéaire ou ramifié,
R₂₁ représente l'hydrogène, le radical méthyle ou le radical phényle éventuellement substitué avec un radical alkyle en C1-C4 ou un radical alcoxy en C₁-C₄,
R₂₂ représente un radical alkyle en C₁-C₂₀, linéaire ou ramifié ou un radical phényle éventuellement substitué avec un radical alkyle en C₁-C₄ ou un radical alcoxy en C₁-C₄.
Z₁ représente un radical divalent assurant la liaison entre -O- et -W₁,
Z₂ représente un radical divalent lorsque a = 1 et trivalent lorsque a = 2 assurant la liaison entre -NH- et -(W₁)ₐ,
Z₁ et Z₂ pouvant être alkylène en C₁-C₁₂, éventuellement substitué par un ou des groupements hydroxyles et pouvant contenir un ou des atomes d'oxygènes ou un ou des groupements amino et éventuellement contenant une double liaison,
W₁ représente :
(i) soit un radical siliconé comprenant au moins une unité de formule (X) suivante 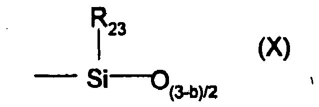 dans laquelle R₂₃ désigne un groupe hydrocarboné saturé ou insaturé, linéaire ou ramifié en C₁-C₃₀ ; un groupe hydrocarboné halogéné en C₁-C₆ ; le radical phényle ;le radical trifluoro-3,3,3 propyle ou un groupe triméthylsilyloxy, et b est égal à 1 ou 2,
(ii) soit un radical de formule (XI) suivante :
-SiR₂₄R₂₅R₂₆ (XI)
dans laquelle R₂₄, R₂₅, R₂₆ , identiques ou différents, sont choisis parmi les radicaux alkyles et alcényles en C₁-C₈, linéaires ou ramifiés ;
les groupes A₁, A₂ et A₃ peuvent également représenter un chromophore absorbant dans l'UVB et/ou UVA et de préférence choisis parmi les groupements aryles substitués par des hydroxyles, des alkyles en C1-C20 linéaires ou ramifiés, des alcoxy en C1-C20, linéaires ou ramifiés ; les amino benzylidène camphre ; les amino benzotriazoles ; les groupements esters d'aminobenzoates, d'aminobenzalmalonates, d'aminosalicylates, d'amino cinnamates en C₁-C₂₀ ou d'anthranilates, linéaires ou ramifiés.

16. **Procédé** selon la revendication **15**, où les composés de formule (III) sont choisis parmi ceux pour lesquels W est un radical siliconé répondant à l'une des trois formules (XII) à (XIV) suivantes : 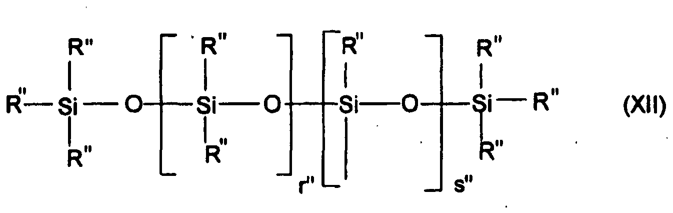 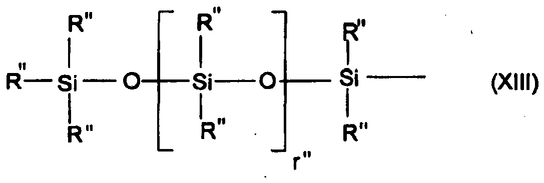 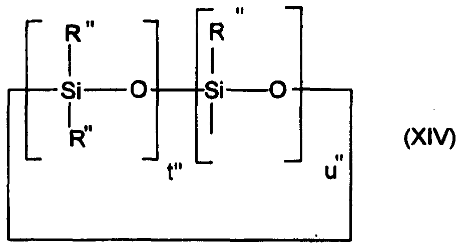 dans lesquelles :
- R", identiques ou différents sont choisis parmi les radicaux alkyles C₁-C₂₀, linéaires ou ramifiés, saturés ou insaturés, le radical phényle et le radical trifluoro-3,3,3 propyle, au moins 80% en nombre des radicaux R étant le radical méthyle,
- r" est un nombre entier choisi entre 0 et 50 inclusivement,
- s" est un nombre entier choisi entre 0 et 20 inclusivement,
- u" est un nombre entier compris entre 1 et 6 inclus,
- t" est un nombre entier entre 0 et 10 inclus,
- t" + u" est égal ou supérieur à 3.

17. **Procédé** selon la revendication **16**, où les composés de formule (III) sont choisi parmi ceux pour lesquels W est un radical siliconé répondant à la formule (XII) ou à la formule (XIII)

18. **Procédé** selon la revendication **17**, où les composés de formule (III) sont choisi parmi les dérivés statistiques ou bien définis à blocs présentant au moins l'une et encore plus préférentiellement l'ensemble des caractéristiques suivantes
- R" est alkyle et encore plus préférentiellement est méthyle,
- r" est compris entre 0 et 3 inclusivement;
- s est compris entre 0 et 3 inclusivement.

19. **Procédé** selon l'une quelconque des revendications **15 à 18**, où les composés de formule (III) sont choisis parmi :
- 2-(4'-ylamino α-cyano-cinnamate d'éthyle)-4-(4-méthoxyphényl)-6-{[1,3,3,3-tetramethyl-1-[(trimethylsilyl)oxy]disiloxanyl]propyl-3-ylamino}-s-triazine,
- 2-(4'-ylamino α-cyano-cinnamate d'éthyle)-4-(4'-ylamino benzoate de butyle)-6-{[1,3,3,3-tetramethyl-1-[(trimethylsilyl)oxy]disiloxanyl]propyl-3-ylamino}-s-triazine ;
- 2-(4'-ylamino-2-méthanesulfonyl-acrylonitrile)-4-(4'-ylamino benzoate de butyle)
- 6-{[1,3,3,3-tetramethyl-1-[(trimethylsilyl)oxy]disiloxanyl]propyl-3-ylamino}-s-triazine
- 2-(4'-ylamino α-cyano-cinnamate d'éthyle)-4-(4'-ylamino benzoate de butyle)-6-(triméthylsilanylméthyl-ylamino}-s-triazine
- 2,4-bis(4'-diylamino benzoate de butyle)-6-(4'-ylamino α-cyano-cinnamate d'éthyle-2 hexyle)-s-triazine,
- 2,4,6-tris-(4'-ylamino α-cyano-cinnamate d'isobutyle)-s-triazine
- 2,4,6-tris-(4'-ylamino α-cyano-cinnamate d'éthyl-2 hexyle)-s-triazine
- 2,4-bis-(4'-ylamino α-cyano-cinnamate d'éthyl-2 hexyle)-6-{[1,3,3,3-tetramethyl-1-[(trimethylsilyl)oxy]disiloxanyl]propyl-3-ylamino}-s-triazine :

20. Composé amino aryl vinyl-s-triazine répondant à la formule (III) suivante : 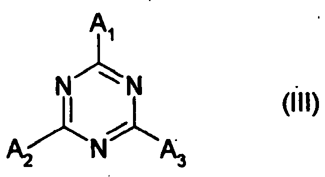 dans laquelle :
les groupes A₁, A₂ et A₃, identiques ou différents sont choisis parmi les groupes de formules (IV) à (VIII) suivantes étant entendu qu'au moins un groupe de formules (IV) à (VII) est présent : 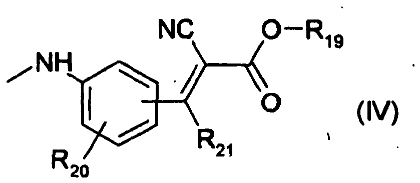 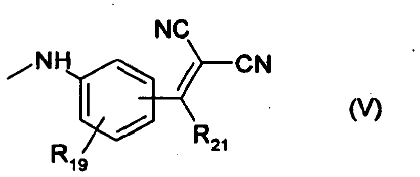 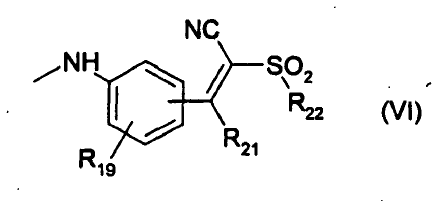 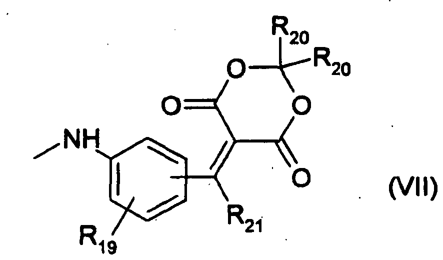
-O-Z₁-W₁ (VIII)
-NH-Z₂-(W₁)ₐ (IX)
dans lesquelles:
R₂₀ représente l'hydrogène, un radical alkyle en C₁-C₄, un radical alcoxy en C₁-C₄,
R₁₉ représente un radical alkyle en C₁-C₂₀, linéaire ou ramifié, un radical hydroxyalkyle en C₂-C₂₀, linéaire ou ramifié ou un radical alcoxy en C₁-C₂₀, linéaire ou ramifié,
R₂₁ représente l'hydrogéne, le radical méthyle ou le radical phényle éventuellement substitué avec un radical alkyle en C₁-C₄ ou un radical alcoxy en C₁-C₄;
R₂₂ représente un radical alkyle en C₁-C₂₀, linéaire ou ramifié ou un radical phényle éventuellement substitué avec un radical alkyle en C₁-C₄ ou un radical alcoxy en C₁-C₄;
Z₁ représente un radical divalent assurant la liaison entre -O- et -W₁,
Z₂ représente un radical divalent lorsque a = 1 et trivalent lorsque a=2 assurant la liaison entre -NH- et -(W₁)ₐ,
Z₁ et Z₂ pouvant être alkylène en C₁-C₁₂, éventuellement substitué par un ou des groupements hydroxyles et pouvant contenir un ou des atomes d'oxygènes ou un ou des groupements amino et éventuellement contenant une double liaison,
W₁ représente
(i) soit un radical siliconé comprenant au moins une unité de formule (X) suivante 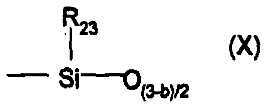 dans laquelle R₂₃ désigne un groupe hydrocarboné saturé ou insaturé, linéaire ou ramifié en C₁-C₃₀ ; un groupe hydrocarboné halogéné en C₁-C₈ ; le radical phényle ;le radical trifluoro-3,3,3 propyle ou un groupe triméthylsilyloxy, et b est égal à 1 ou 2,
(ii) soit un radical de formule (XI) suivante :
-SiR₂₄R₂₅R₂₆ (XI)
dans laquelle R₂₄, R₂₅, R₂₆, identiques ou différents, sont choisis parmi les radicaux alkyles et alcényles en C₁-C₆, linéaires ou ramifiés;
les groupes A₁, A₂ et A₃ peuvent également
être choisis parmi les groupements aryles substitués par des hydroxyles, des alkyles en C1-C20 linéaires ou ramifiés, des alcoxy en C₁-C₂₀, linéaires ou ramifiés; les amino benzylidène camphre ; les amino benzotriazoles ; les groupements esters d'aminobenzoates, d'aminobenzalmalonates, d'aminosalicylates, d'amino cinnamates en C₁-C₂₀ ou d'anthranilates, linéaires ou ramifiés.
sous réserve que :
- les 3 groupements A₁, A₂ et A₃ ne désignent pas la même formule (IV)
- lorsque A₂ et A₃ désignent un groupe de formule (IV) alors A₁ ne désigne pas un groupe de formule (VIII) ou (IX).

21. Composé selon la revendication **20**, où W est un radical siliconé répondant à l'une des tois formules (XII) à (XIII) suivantes : 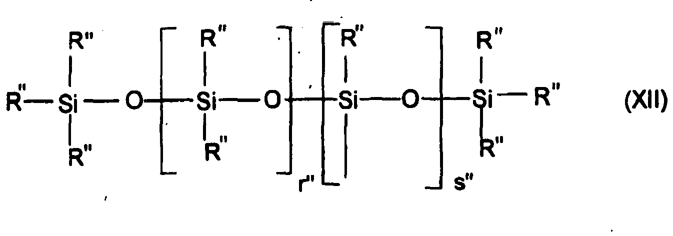 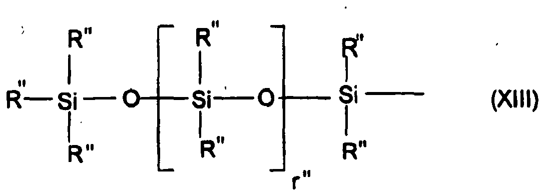 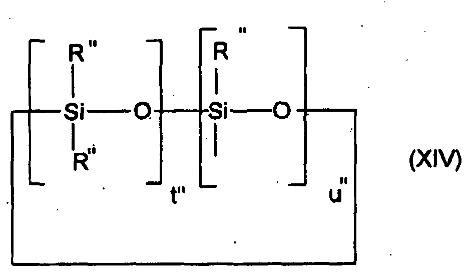 dans lesquelles :
- R", identiques ou différents sont choisis parmi les radicaux alkyles C₁-C₂₀, linéaires ou ramifiés, saturés ou insaturés, le radical phényle et le radical trifluoro-3,3,3 propyle, au moins 80% en nombre des radicaux R étant le radical méthyle,
- r" est un nombre entier choisi entre 0 et 50 inclusivement,
- s" est un nombre entier choisi entre 0 et 20 inclusivement,
- u" est un nombre entier compris entre 1 et 6 inclus,
- t" est un nombre entier entre 0 et 10 inclus,
- t" + u" est égal ou supérieur à 3.

22. Composé selon la revendication **21**, où W est un radical siliconé répondant à la formule (XII) ou à la formule (XIII).

23. Composé selon la revendication **22**, choisi parmi les dérivés statistiques ou bien définis à blocs présentant au moins l'une, et encore plus préférentiellement l'ensemble, des caractéristiques suivantes :
- R" est alkyle et encore plus préférentiellement est méthyle,
- r" est compris entre 0 et 3 inclusivement ; s est compris entre 0 et 3 inclusivement.

24. Composé selon l'une quelconque des revendications **20 à 23**, choisi parmi :
- le 2,4-bis(4'-diylamino benzoate de butyle)-6-(4'-ylamino α-cyano-cinnamate d'éthyle-2 hexyle)-s-triazine ;
- le 2-(4'-ylamino-2-méthanesulfonyl-acrylonitrile)-4-(4'-ylamino benzoate de butyle)-6-{[1,3,3,3-tetramethyl-1-[(trimethylsilyl)oxy]disiloxanyl]propyl-3-ylamino}-s-triazine.

25. Composition cosmétique ou dermatologique contenant dans un milieu physiologiquement acceptable au moins un composé amino aryl vinyl-s-triazine de formule (III) tel que défini dans l'une quelconque des revendications 20 à 24.

26. Composition selon la revendication **25**, où les composés de formule (III) sont présents dans des concentrations allant de 0.1 à 15 % en poids et plus particulièrement de 0.1 à 10 % en poids par rapport au poids total de la composition.

27. Composition selon la revendication **25 ou 26**, comprenant en plus au moins un agent photoprotecteur organique et/ou au moins un agent photoprotecteur inorganique actif dans l'UVA et/ou l'UVB, hydrosoluble ou liposoluble ou bien insoluble dans les solvants cosmétiques couramment utilisés.

28. Composition selon la revendication **27** où les agents photoprotecteurs organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate; les dérivés de benzotriazole; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

29. Composition selon la revendication **28** 44 où les agents photoprotecteurs organiques sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic, Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
la 2,4,6-tris-(4'amino-benzalmalonate de diisobutyle)-s-triazine,
Methylene bis-Benzotriazolyl Tetramethylbutylphenol,
Drometrizole Trisiloxane,
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

30. Composition selon la revendication **27** où les agents photoprotecteurs inorganiques sont choisis parmi des pigments ou bien encore des nanopigments d'oxydes métalliques enrobés ou non

31. Composition selon la revendication **30** où les agents photoprotecteurs inorganiques sont des nanopigments d'oxyde de titane, de fer, de zinc, de zirconium ou de cérium enrobés ou non enrobés.

32. Composition selon l'une quelconque des revendications **25 à 31** où les agents photoprotecteurs sont présents dans des proportions allant de 0,01 à 20% en poids par rapport au poids total de la composition, et de préférence allant de 0,1 à 10% en poids par rapport au poids total de la composition.

33. Composition selon l'une quelconque des revendications **25 à 32,** contenant en plus au moins un agent de bronzage et/ou de brunissage artificiels de la peau.

34. Composition selon l'une quelconque des revendications **25 à 33**, contenant en plus au moins un adjuvant cosmétique choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

## Patentansprüche

1. Kosmetisches Verfahren zur Bewahrung des natürlichen Teints und des natürlichen Antioxidationsschutzes der Haut durch die in der Haut vorliegenden endogenen Carotinoide, **dadurch gekennzeichnet, dass** man auf die Haut eine Zusammensetzung aufbringt, die mindestens ein Lichtstrahlung einer Wellenlänge im Bereich von 370 bis 500 nm herausfilterndes Mittel umfasst, das aus
(v) Kohlenhydraten;
(vi) gelben oder orangegelben anorganischen Pigmenten;
(vii) Azo- oder Chinonverbindungen;
(viii) Farbmitteln, die sich von Nitrobenzol ableiten;
(ix) Verbindungen, die sich von Arylvinylenketon ableiten;
(x) Aminoarylvinyl-s-triazin-Verbindungen und Mischungen davon ausgewählt ist.

2. Verfahren nach Anspruch 1, bei dem die Kohlenhydrate aus gelb gefärbten Produkten des Erhitzens oder der Oxidation von Mono- oder Polysacchariden ausgewählt werden.

3. Verfahren nach Anspruch 2, bei dem es sich bei dem Kohlenhydrat um Karamell handelt.

4. Verfahren nach Anspruch 1, bei dem die gelben oder orangefarbenen anorganischen Pigmente aus Eisenoxidpigmenten und spezielle Nanopigmenten mit einer Elementarteilchengröße von weniger als 100 nm ausgewählt werden.

5. Verfahren nach Anspruch 2, bei dem die Chinonverbindungen aus gelben oder orangegelben Naphthochinonen und Oxidationsprodukten davon; Anthrachinonen und gelben oder orangegelben Oxidationsprodukten davon ausgewählt werden.

6. Verfahren nach Anspruch 5, bei dem die Chinonverbindungen aus Juglon und Lawson ausgewählt werden.

7. Verfahren nach Anspruch 1, bei dem es sich bei der Azoverbindung um Tartrazin handelt.

8. Verfahren nach Anspruch 1, bei dem die Chinon- oder Azoverbindung unter denjenigen der Formel (1) oder (2) oder (3) ausgewählt wird: 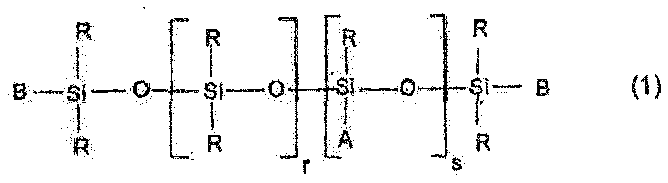 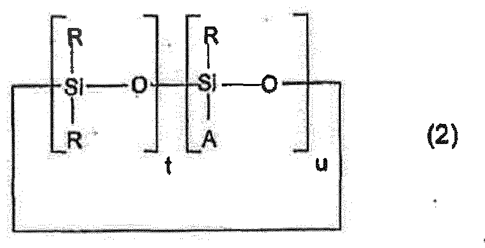 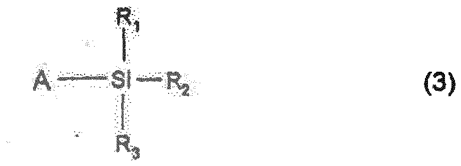 worin für die Formeln (1) und (2):
- die Reste R gleich oder verschieden sind und aus C₁-C₁₀-Alkyl-, Phenyl- und 3,3,3-Trifluorpropylresten ausgewählt sind, wobei mindestens 80 Zahlenprozent der Reste R Methyl sind,
- die Reste B gleich oder verschieden sind und aus den obigen Resten R und dem nachstehend definierten Rest A ausgewählt sind,
- r eine ganze Zahl zwischen 0 und 50 inklusive ist und s eine ganze Zahl zwischen 0 und 20 inklusive ist, mit der Maßgabe, dass dann, wenn s null ist, mindestens eines der beiden Symbole B für A steht,
- u für eine ganze Zahl zwischen 1 und 6 inklusive steht und
- t für eine ganze Zahl zwischen 0 und 10 inklusive steht,
mit der Maßgabe, dass t + u größer gleich 3 ist,
- R₁, R₂ und R₃ gleich oder verschieden sind und aus gesättigten oder ungesättigten, linearen oder verzweigten C₁-C₈-Alkyl- und -Alkenylresten ausgewählt sind,
- die Symbole A gleich oder verschieden sind und für einen Rest der folgenden Formeln (4a), (4b), (4c) oder (4d) stehen: 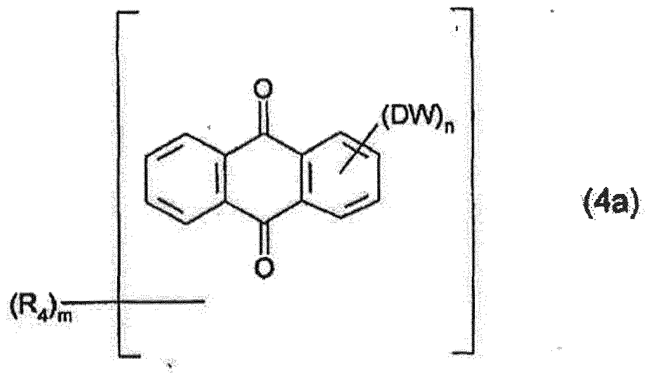 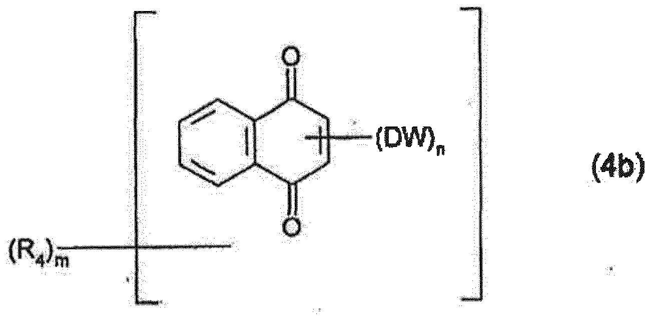 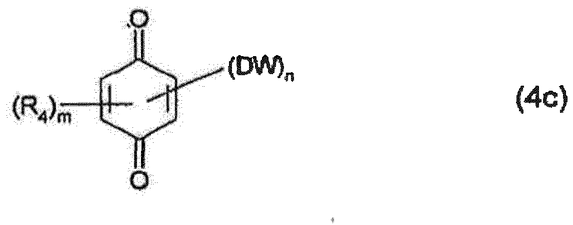 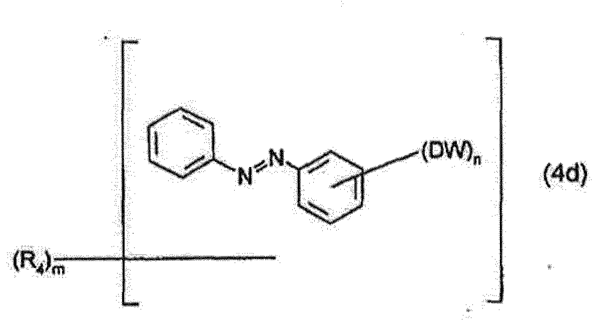
- die Reste R₄ gleich oder verschieden sind und für einen linearen oder verzweigten C₁-C₆-Alkylrest, einen OH-, C₁-C₄-Alkoxy-, Hydroxy(C₁-C₄)alkyl-, COOH-, CONH₂-, CN-, SO₃H-, Halogen- oder NO₂-Rest oder einen NR₅R₆-Rest, worin R₅ und R₆ gleich oder verschieden sind und für ein Wasserstoffatom oder einen C₁-C₈-Alkyl-, Hydroxy(C₁-C₄)alkyl- oder Amino-(C₁-C₄)alkylrest stehen oder zusammen mit dem Stickstoffatom, an das sie gebunden sind, einen 5- oder 6-gliedrigen Heterocyclus, der gegebenenfalls durch ein Sauerstoff- oder Schwefelatom unterbrochen ist, bilden, stehen,
- m eine ganze Zahl zwischen 0 und 2 inklusive ist,
- n eine ganze Zahl mit einem Wert von 1 oder 2 ist,
- D ein -SO₂NH-, -CONH- oder -0-Rest oder ein -NR₇-Rest, worin R₇ H oder CH₃ ist, ist,
- W ein zweiwertiger Rest der Formel (5): 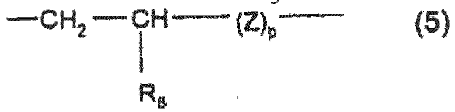 oder der Formel (6):
- HC=CH-(Z)ₚ- (6)
ist, worin
- R₈ für ein Wasserstoffatom, einen Hydroxylrest oder einen gesättigten oder ungesättigten, linearen oder verzweigten C₁-C₈-Alkylrest steht,
- Z ein linearer oder verzweigter C₁-C₆-Alkylenrest ist, der gegebenenfalls durch einen OH-Rest oder einen gesättigten oder ungesättigten, linearen oder verzweigten C₂-C₈-Alkoxyrest substituiert ist,
- p eine ganze Zahl mit einem Wert von 0 oder 1 ist.

9. Verfahren nach Anspruch 8, wobei die Verbindung der Formel (1), (2) oder (3) aus den folgenden Verbindungen ausgewählt wird:
- 4-(4-Dimethylaminophenylazo)-N-[3-[1,3,3,3-tetramethyl-1-[(trimethylsilyl)oxy]disiloxanyl]-propyl]benzolsulfonamid;
- 4-(4-Dimethylaminophenylazo)-N-[3-[1,3,3,3-tetramethyl-1-[(trimethylsilyl)oxy]disiloxanyl]-propyl]benzamid;
- 2-(4-Methoxy-2-nitrophenylazo)-5-(3-trimethyl-silanylpropoxy)phenol;
- 2,5-Bis-[3-[1,3,3,3-tetramethyl-1-[(trimethyl-silyl)oxy]disiloxanyl]propylamino]benzochinon;
- 2-Chlor-3-[3-[1,3,3,3-tetramethyl-1-[(trimethylsilyl)oxy]disiloxanyl]propylamino]-[1,4]-naphthochinon;
- 2-[3-[1,3,3,3-Tetramethyl-1-[(trimethylsilyl)-oxy]disiloxanyl]propylamino]-[1,4]-naphthochinon;
- 1-Hydroxy-4-[3-[1,3,3,3-tetramethyl-1-[(trimethylsilyl)oxy]disiloxanyl]propylamino]anthra-chinon;
- 1,4-Di[3-[1,3,3,3-tetramethyl-1-[(trimethylsilyl)oxy]disiloxanyl]propylamino]anthrachinon.

10. Verfahren nach Anspruch 1, bei dem die Nitrobenzolverbindung aus denjenigen der Formel (15) oder (16) ausgewählt wird: 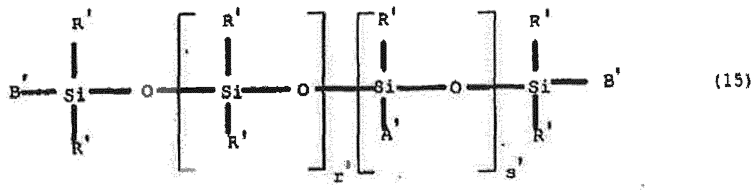 oder 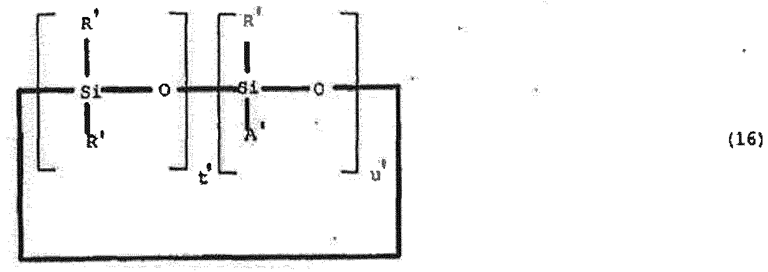 worin:
- die Reste R' gleich oder verschieden sind und aus linearen oder verzweigten C₁-C₁₀-Alkyl-, Phenyl- und 3,3,3-Trifluorpropylresten ausgewählt sind, wobei mindestens 80 Zahlenprozent der Reste R' Methyl sind,
- die Reste B' gleich oder verschieden sind und aus den obigen Resten R' und dem nachstehend definierten Rest A ausgewählt sind,
- r' eine ganze Zahl zwischen 0 und 50 inklusive ist und s' eine ganze Zahl zwischen 0 und 20 inklusive ist, mit der Maßgabe, dass dann, wenn s' null ist, mindestens eines der beiden Symbole B für A steht,
- u' für eine ganze Zahl zwischen 1 und 6 inklusive steht und t' für eine ganze Zahl zwischen 0 und 10 inklusive steht, mit der Maßgabe, dass t' + u' größer gleich 3 ist,
- und das Symbol A' für einen einwertigen Rest, der direkt an ein Siliciumatom gebunden ist und der folgenden Formel (17) entspricht, steht: 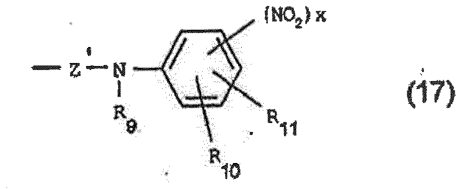
- Z' der zweiwertige Rest: 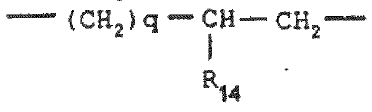
oder Wasserstoff ist,
- x 1 oder 2 ist,
- q eine ganze Zahl zwischen 0 und 10 inklusive ist,
- R₉ für Wasserstoff oder einen C₁-C₄-Alkylrest steht,
- R₁₀ für Wasserstoff oder einen C₁-C₄-Alkylrest oder den oben definierten zweiwertigen Rest Z' steht,
- R₁₁ für Wasserstoff oder einen NR₁₂R₁₃-Rest steht, worin R₁₂ und R₁₃ für Wasserstoff oder einen C₁-C₄-Alkylrest, einen C₂-C₄-Mono- oder -Dihydroxyalkyl-rest oder den zweiwertigen Rest Z stehen, mit der Maßgabe,
- R₁₄ für Wasserstoff, einen OH- oder Halogenrest, einen C₁-C₄-Alkylrest oder einen C₁-C₄-Alkoxyrest steht.

11. Verfahren nach Anspruch 10, bei dem die Verbindung der Formel (15) oder (16) aus den folgenden Verbindungen ausgewählt wird:

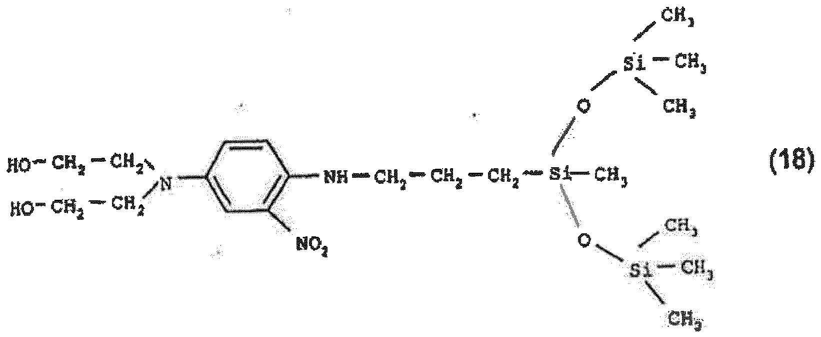

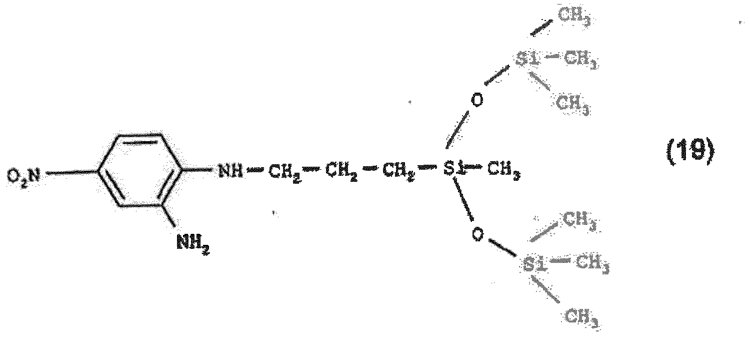

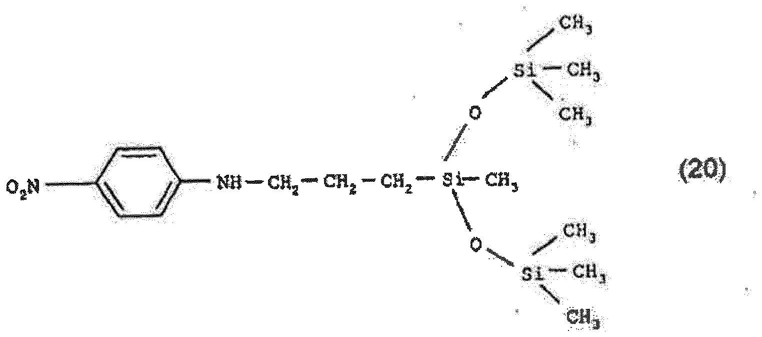

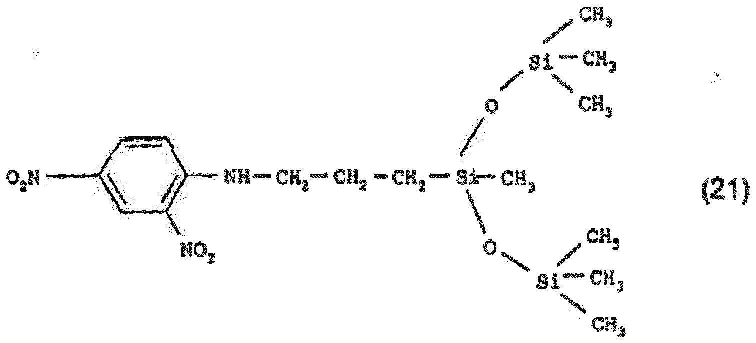

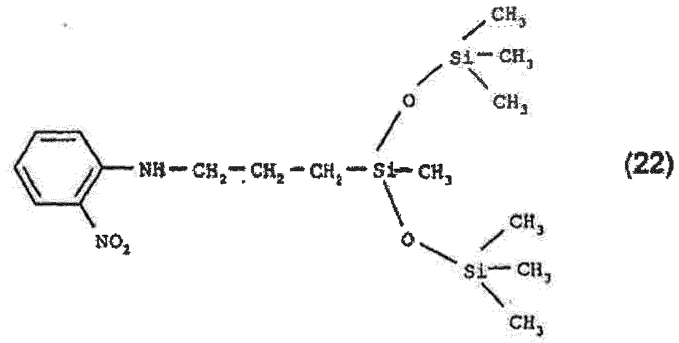

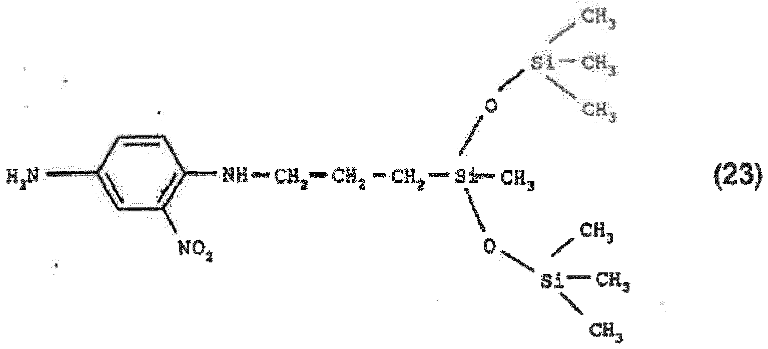

12. Verfahren nach Anspruch 1, bei dem die Arylvinylketonverbindung aus denjenigen der folgenden Formeln (I) und (II) ausgewählt wird: 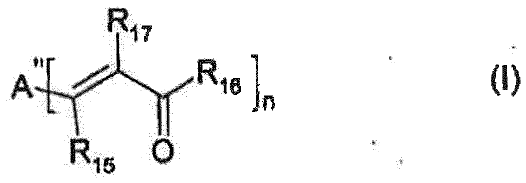 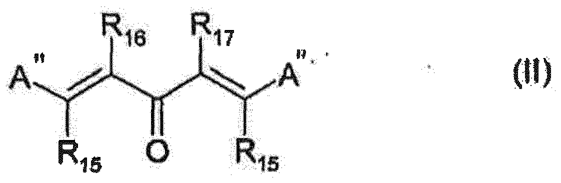 worin:
n = 1 oder 2,
A" in der Formel (I), wenn n = 1, oder in Formel (II) ein Arylrest, der aus den folgenden Formeln (a) bis (d) ausgewählt ist, oder in Formel (I), wenn n = 2, ein Rest, der unter den folgenden Formeln (e) bis (h) ausgewählt ist, ist: 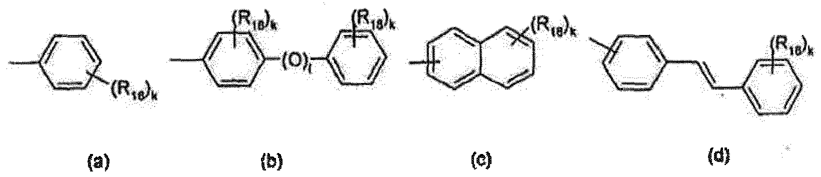 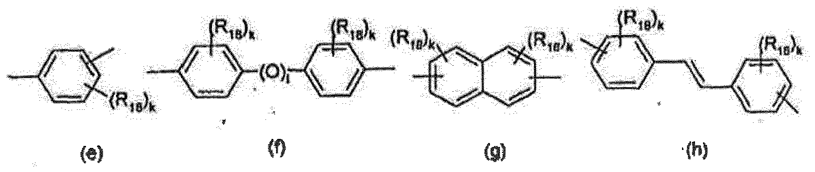
worin:
- jedes der Symbole R₁₈ unabhängig für eine OH-Gruppe; ein Halogenatom; eine lineare oder verzweigte C₁-C₆-Alkylgruppe, die gegebenenfalls ein Siliciumatom oder eine Siloxangruppe enthält; eine lineare oder verzweigte C₁-C₆-Alkoxygruppe, die gegebenenfalls ein Siliciumatom oder eine Siloxangruppe enthält; eine lineare oder verzweigte C₁-C₅-Alkoxycarbonylgruppe oder eine lineare oder verzweigte C₁-C₆-Alkylsulfonamidgruppe, die gegebenenfalls ein Siliciumatom, eine Siloxangruppe oder eine Aminosäurefunktion enthält; steht;
- k für eine ganze Zahl zwischen 0 und 3 inklusive steht;
- 1 für 0 oder 1 steht;
- R₁₅ für Wasserstoff oder eine OH-Gruppe steht;
- R₁₆ für Wasserstoff, eine lineare oder verzweigte C₁-C₆-Alkylgruppe, die gegebenenfalls ein Siliciumatom oder eine Siloxangruppe enthält, eine Cyanogruppe, eine C₁-C₆-Alkylsulfonylgruppe oder eine Phenylsulfonylgruppe steht,
- R₁₇ für eine lineare oder verzweigte C₁-C₆-Alkylgruppe steht, die gegebenenfalls ein Siliciumatom, eine Siloxangruppe oder eine Phenylgruppe, die einen Bicyclus bilden kann und gegebenenfalls durch einen oder zwei Reste R₁₈ gemäß obiger Definition substituiert ist, enthält,
- R₁₆ und R₁₇ zusammen einen monocyclischen, bicyclischen oder tricyclischen C₂-C₁₀-Kohlenwasserstoffrest bilden können, der gegebenenfalls durch ein oder mehrere Stickstoff-, Schwefel- und Sauerstoffatome unterbrochen ist und ein weiteres Carbonyl enthalten kann und gegebenenfalls durch eine lineare oder verzweigte C₁-C₈-Alkylsulfonamidgruppe substituiert ist und gegebenenfalls ein Siliciumatom, eine Siloxangruppe oder eine Aminosäurefunktion enthält; mit der Maßgabe, dass dann, wenn n = 1, R₁₆ und R₁₇ keinen Campherkern bilden.

13. Verfahren nach Anspruch 12, bei dem die Verbindung der Formel (I) ausgewählt ist aus:
- 1-(2,4,5-Trimethoxyphenyl)-4,4-dimethylpent-1-en-3-on: 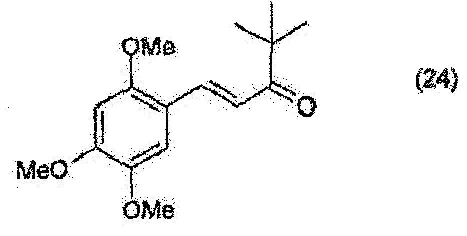
- 3-(3-Ethoxy-4-butoxybenzyliden)-2,3,4a,8a-tetrahydrochromen-4-on: 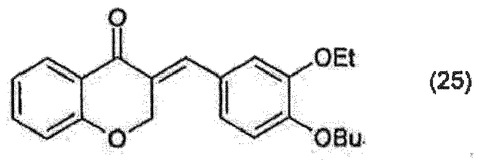
- 3-(4-Methoxybenzyliden)-2,3,4a,8a-tetrahydrochromen-4-thion: 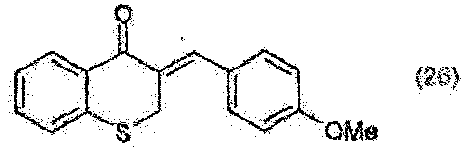
- 2-(3-Methoxy-4-butoxybenzyliden)indan-1-on: 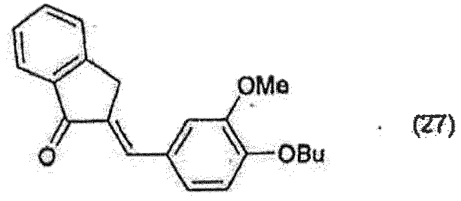
- 2-(3-Methoxy-4-butoxybenzyliden)-3,4-dihydro-2H-naphthalin-1-on: 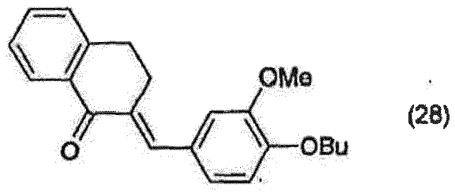
- 2-Benzylidenbenzofuran-3-on: 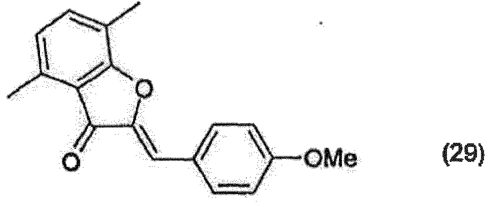
- 2-(3,5-Dimethoxy-4-hydroxybenzyliden)indan-1,3-dion: 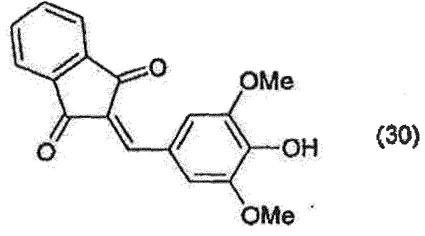
- 2-Benzylidenbenzo[b]thiophen-3-on: 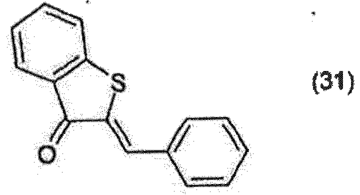
- 4-(3-Methoxy-4-butoxybenzyliden)-5-methyl-2-phenyl-2,4-dihydropyrazol-3-on: 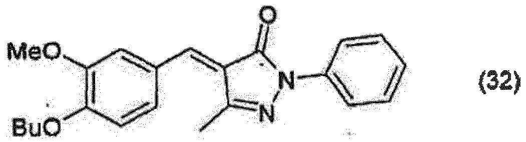
- 5-(3-Methoxy-4-butoxybenzyliden)-2-phenyl-3,5-dihydroimidazol-4-on: 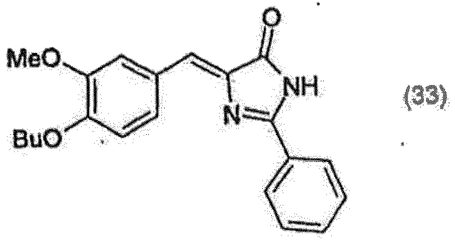
- 1-(2-Hydroxy-4-methoxyphenyl)-3-(4'-methoxy-phenyl)propenon: 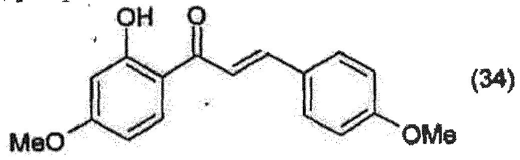
- 3-Hydroxy-1-(2-hydroxy-4-methoxyphenyl)-3-phenylpropenon: 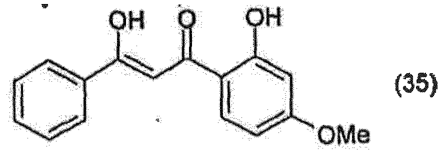
- der nachstehenden para-Xylidenverbindung: 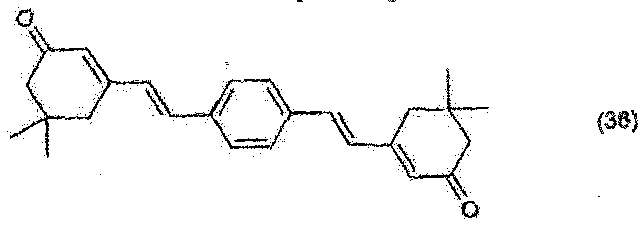
- (2,5-Dimethoxyphenylen)-1,4-bis{3-methyliden-bicyclo[2.2.1]heptan-2-on}: 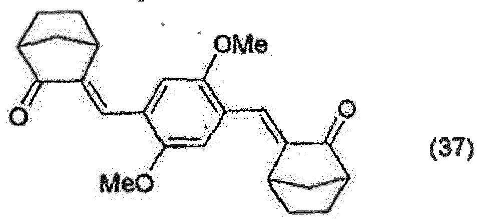
- (2,5-Dihexyloxyphenylen)-1,4-bis{3-methyliden-1,7,7-trimethylbicyclo[2.2.1]heptan-2-on}:

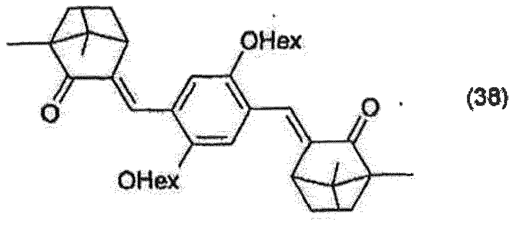

14. Verfahren nach Anspruch 13, bei dem die Verbindung der Formel (II) aus
- 2,5-Bis(4-hexyloxybenzyliden)cyclopentanon und
- 2,5-Bis{4-[[1,3,3,3-tetramethyl-1[(trimethylsilyl) oxy]disiloxanyl]-3-methylpropyloxy]benzyliden}-cyclopentanon
ausgewählt wird.

15. Verfahren nach Anspruch 1, bei dem die Aminoarylvinyl-s-triazin-Verbindungen der folgenden Formel (III) entsprechen: 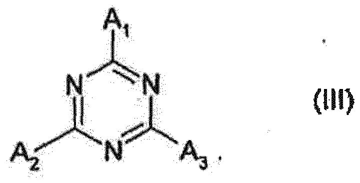 worin:
die Gruppen A₁, A₂ und A₃ gleich oder verschieden sind und aus den Gruppen der nachstehenden Formeln (IV) bis (VIII) ausgewählt sind, mit der Maßgabe, dass mindestens eine Gruppe der Formeln (IV) bis (VII) vorliegt: 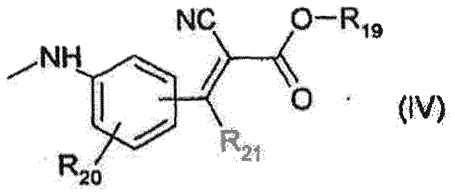 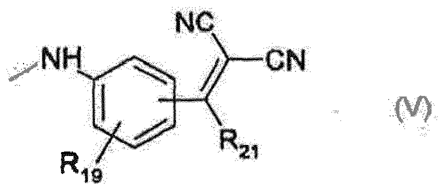 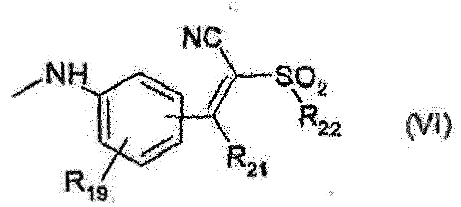 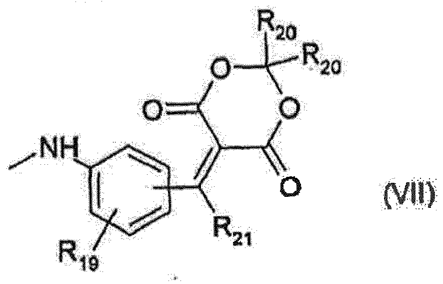
-O-Z₁-W₁ (VIII)
-NH-Z₂-(W₁)ₐ (IX)
worin:
R₂₀ für Wasserstoff, einen C₁-C₄-Alkylrest oder einen C₁-C₄-Alkoxyrest steht,
R₁₉ für einen linearen oder verzweigten C₁-C₂₀-Alkylrest, einen linearen oder verzweigten C₂-C₂₀-Hydroxyalkylrest oder einen linearen oder verzweigten C₁-C₂₀-Alkoxyrest steht,
R₂₁ für Wasserstoff, einen Methylrest oder einen Phenylrest, der gegebenenfalls durch einen C₁-C₄-Alkylrest oder einen C₁-C₄-Alkoxyrest substituiert ist, steht,
R₂₂ für einen linearen oder verzweigten C₁-C₂₀-Alkylrest oder einen Phenylrest, der gegebenenfalls durch einen C₁-C₄-Alkylrest oder einen C₁-C₄-Alkoxyrest substituiert ist, steht,
Z₁ für einen zweiwertigen Rest steht, der die Bindung zwischen -O- und -W₁ gewährleistet,
Z₂ im Fall von a = 1 für einen zweiwertigen Rest und im Fall von a = 2 für einen dreiwertigen Rest, der die Bindung zwischen -NH- und -(W₁)ₐ gewährleistet, steht,
wobei Z₁ und Z₂ C₁-C₁₂-Alkylen, das gegebenenfalls durch eine oder mehrere Hydroxylgruppen substituiert ist und ein oder mehrere Sauerstoffatome oder eine oder mehrere Aminogruppen enthalten kann und gegebenenfalls eine Doppelbindung enthält, sein können,
W₁ für:
(i) entweder einen Silikonrest mit mindestens einer Einheit der folgenden Formel (X) 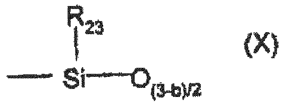 worin R₂₃ für eine lineare oder verzweigte, gesättigte oder ungesättigte C₁-C₃₀-Kohlenwasserstoffgruppe; eine C₁-C₈-Halogenkohlenwasserstoffgruppe; einen Phenylrest; einen 3,3,3-Trifluorpropylrest oder eine Trimethylsilyloxygruppe steht und b gleich 1 oder 2 ist,
(ii) oder einen Rest der folgenden Formel (XI):
-SiR₂₄R₂₅R₂₆ (XI)
worin R₂₄, R₂₅ und R₂₆ gleich oder verschieden sind und aus linearen oder verzweigten C₁-C₈-Alkyl- und -Alkenylresten ausgewählt sind,
steht;
wobei die Gruppen A₁, A₂ und A₃ auch für ein im UVB und/oder UVA absorbierendes Chromophor, das vorzugsweise aus Arylgruppen, die durch Hydroxylgruppen, lineare oder verzweigte C₁-C₂₀-Alkylgruppen und lineare oder verzweigte C₁-C₂₀-Alkoxygruppen substituiert sind; Aminobenzylidencamphergruppen; Aminobenzotriazolgruppen und linearen oder verzweigten Aminobenzoat-, Amino-benzalmalonat-, Aminosalicylat-, C₁-C₂₀-Amino-cinnamat- oder Anthranilatestergruppen ausgewählt ist, stehen können.

16. Verfahren nach Anspruch 15, bei dem die Verbindungen der Formel (III) aus denjenigen ausgewählt werden, für die W ein Silikonrest ist, der einer der drei folgenden Formeln (XII) bis (XIV) entspricht: 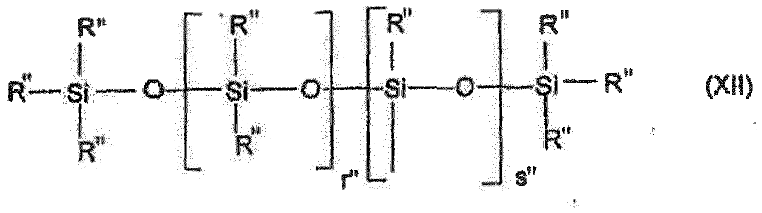 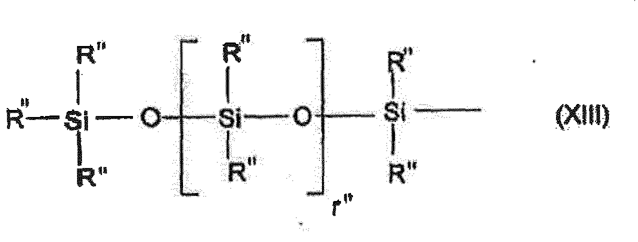 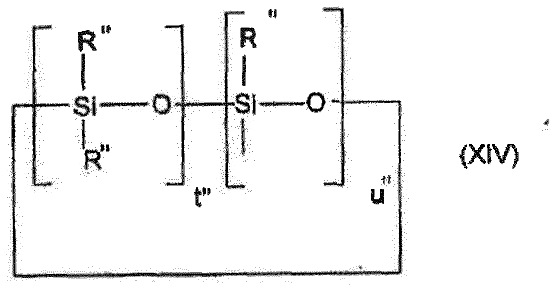 worin:
- die Reste R" gleich oder verschieden sind und aus gesättigten oder ungesättigten, linearen oder verzweigten C₁-C₂₀-Alkylresten, einem Phenylrest und einem 3,3,3-Trifluorpropylrest ausgewählt sind, wobei mindestens 80 Zahlenprozent der Reste R Methyl sind,
- r" eine ganze Zahl zwischen 0 und 50 inklusive ist,
- s" eine ganze Zahl zwischen 0 und 20 inklusive ist,
- u" eine ganze Zahl zwischen 1 und 6 inklusive ist,
- t" eine ganze Zahl zwischen 0 und 10 inklusive ist,
- t" + u" größer gleich 3 ist.

17. Verfahren nach Anspruch 16, bei dem die Verbindungen der Formel (III) unter denjenigen ausgewählt werden, für die W ein Silikonrest ist, der der Formel (XII) oder der Formel (XIII) entspricht.

18. Verfahren nach Anspruch 17, bei dem die Verbindungen der Formel (III) aus statistisch aufgebauten Derivaten oder Derivaten mit gut definierten Blöcken mit mindestens einer und noch weiter bevorzugt allen der folgenden Eigenschaften ausgewählt werden:
- R" ist Alkyl und noch weiter bevorzugt Methyl,
- r" liegt zwischen 0 und 3 inklusive,
- s liegt zwischen 0 und 3 inklusive.

19. Verfahren nach einem der Ansprüche 15 bis 18, bei dem die Verbindungen der Formel (III) aus:
- 2-(Ethyl-4'-ylamino-a-cyanocinnamat)-4-(4-methoxyphenyl)-6-{[1,3,3,3-tetramethyl-1-[(tri-methylsilyl)oxy]disiloxanyl]propyl-3-ylamino}-s-triazin,
- 2-(Ethyl-4'-ylamino-a-cyanocinnamat)-4-(butyl-4'-ylaminobenzoat)-6-{[1,3,3,3-tetramethyl-1-[(trimethylsilyl)oxy]disiloxanyl]propyl-3-yl-amino}-s-triazin,
- 2-(4'-Ylamino-2-methansulfonylacrylnitril)-4-(butyl-4'-ylaminobenzoat)-6-{[1,3,3,3-tetramethyl-1-[(trimethylsilyl)oxy]disiloxanyl]propyl-3-yl-amino}-s-triazin,
- 2-(Ethyl-4'-ylamino-α-cyanocinnamat)-4-(butyl-4'-ylaminobenzoat)-6-(trimethylsilanylmethylyl-amino)-s-triazin,
- 2,4-Bis(butyl-4'-diylaminobenzoat)-6-(2-ethyl-hexyl-4'-ylamino-α-cyanocinnamat)-s-triazin,
- 2,4,6-Tris(isobutyl-4'-ylamino-α-cyano-cinnamat)-s-triazin,
- 2,4,6-Tris(2-ethylhexyl-4'-ylamino-α-cyano-cinnamat)-s-triazin und
- 2,4-Bis(2-ethylhexyl-4'-ylamino-α-cyano-cinnamat)-6-{[1,3,3,3-tetramethyl-1-[(trimethylsilyl)oxy]disiloxanyl]propyl-3-ylamino}-s-triazin ausgewählt werden.

20. Aminoarylvinyl-s-triazin-Verbindung, die der folgenden Formel (III) entspricht: 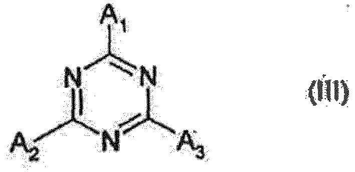 worin:
die Gruppen A₁, A₂ und A₃ gleich oder verschieden sind und aus den Gruppen der nachstehenden Formeln (IV) bis (VIII) ausgewählt sind, mit der Maßgabe, dass mindestens eine Gruppe der Formeln (IV) bis (VII) vorliegt: 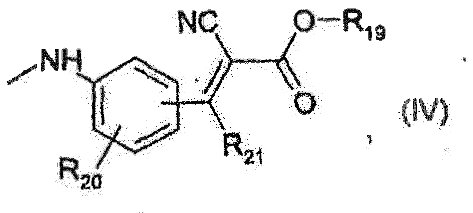 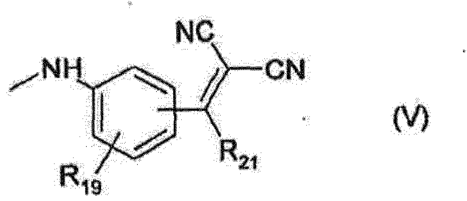 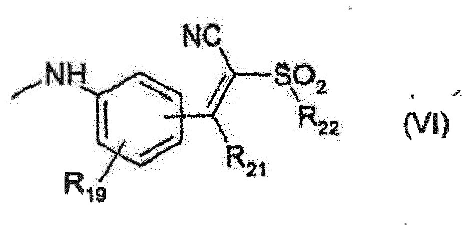 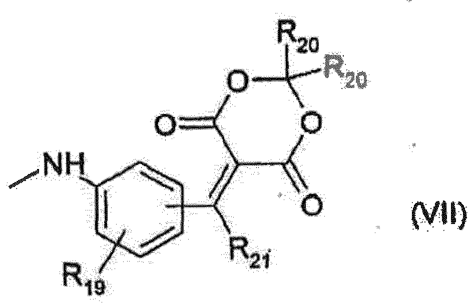
**-O-Z₁-W₁** **(VIII)**
**-NH-Z₂-(W₁)ₐ** **(IX)**
worin:
R₂₀ für Wasserstoff, einen C₁-C₄-Alkylrest oder einen C₁-C₄-Alkoxyrest steht,
R₁₉ für einen linearen oder verzweigten C₁-C₂₀-Alkylrest, einen linearen oder verzweigten C₂-C₂₀-Hydroxyalkylrest oder einen linearen oder verzweigten C₁-C₂₀-Alkoxyrest steht,
R₂₁ für Wasserstoff, einen Methylrest oder einen Phenylrest, der gegebenenfalls durch einen C₁-C₄-Alkylrest oder einen C₁-C₄-Alkoxyrest substituiert ist, steht,
R₂₂ für einen linearen oder verzweigten C₁-C₂₀-Alkylrest oder einen Phenylrest, der gegebenenfalls durch einen C₁-C₄-Alkylrest oder einen C₁-C₄-Alkoxyrest substituiert ist, steht,
Z₁ für einen zweiwertigen Rest steht, der die Bindung zwischen -O- und -W₁ gewährleistet,
Z₂ im Fall von a = 1 für einen zweiwertigen Rest und im Fall von a = 2 für einen dreiwertigen Rest, der die Bindung zwischen -NH- und -(W₁)ₐ gewährleistet, steht,
wobei Z₁ und Z₂ C₁-C₁₂-Alkylen, das gegebenenfalls durch eine oder mehrere Hydroxylgruppen substituiert ist und ein oder mehrere Sauerstoffatome oder eine oder mehrere Aminogruppen enthalten kann und gegebenenfalls eine Doppelbindung enthält, sein können,
W₁ für:
(i) entweder einen Silikonrest mit mindestens einer Einheit der folgenden Formel (X) 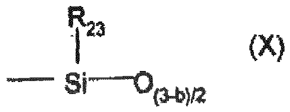 worin R₂₃ für eine lineare oder verzweigte, gesättigte oder ungesättigte C₁-C₃₀-Kohlenwasserstoffgruppe; eine C₁-C₈-Halogenkohlenwasserstoffgruppe; einen Phenylrest; einen 3,3,3-Trifluorpropylrest oder eine Trimethylsilyloxygruppe steht und b gleich 1 oder 2 ist,
(ii) oder einen Rest der folgenden Formel (XI):
-SiR₂₄R₂₅R₂₆ (XI)
worin R₂₄, R₂₅ und R₂₆ gleich oder verschieden sind und aus linearen oder verzweigten C₁-C₈-Alkyl- und -Alkenylresten ausgewählt sind,
steht;
wobei die Gruppen A₁, A₂ und A₃ auch aus Arylgruppen, die durch Hydroxylgruppen, lineare oder verzweigte C₁-C₂₀-Alkylgruppen und lineare oder verzweigte C₁-C₂₀-Alkoxygruppen substituiert sind; Aminobenzylidencamphergruppen; Aminobenzotriazolgruppen und linearen oder verzweigten Aminobenzoat-, Aminobenzalmalonat-, Aminosalicylat-, C₁-C₂₀-Aminocinnamat- oder Anthranilatestergruppen ausgewählt sein können,
mit der Maßgabe, dass:
- die 3 Gruppen A₁, A₂ und A₃ nicht für die gleiche Formel (IV) stehen,
- A₁ dann, wenn A₂ und A₃ für eine Gruppe der Formel (IV) stehen, nicht für eine Gruppe der Formel (VIII) oder (IX) steht.

21. Verbindung nach Anspruch 20, wobei W ein Silikonrest ist, der einer der drei folgenden Formeln (XII) bis (XIV) entspricht: 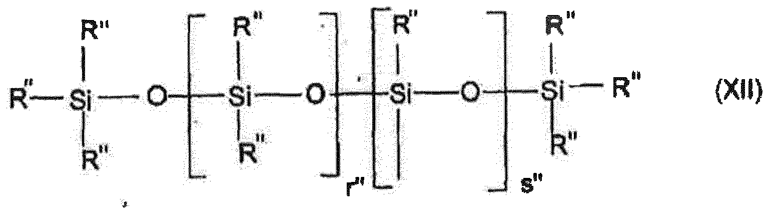 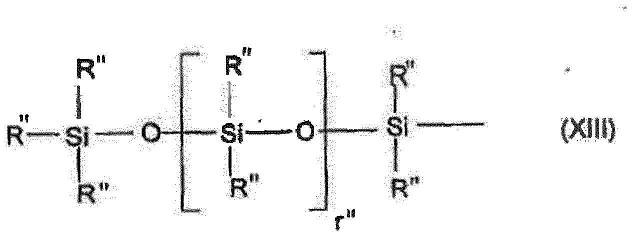 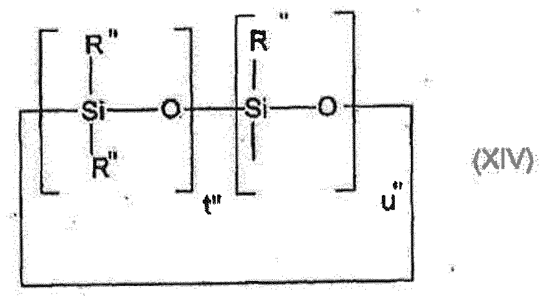 worin:
- die Reste R" gleich oder verschieden sind und aus gesättigten oder ungesättigten, linearen oder verzweigten C₁-C₂₀-Alkylresten, einem Phenylrest und einem 3,3,3-Trifluorpropylrest ausgewählt sind, wobei mindestens 80 Zahlenprozent der Reste R Methyl sind,
- r" eine ganze Zahl zwischen 0 und 50 inklusive ist,
- s" eine ganze Zahl zwischen 0 und 20 inklusive ist,
- u" eine ganze Zahl zwischen 1 und 6 inklusive ist,
- t" eine ganze Zahl zwischen 0 und 10 inklusive ist,
- t" + u" größer gleich 3 ist.

22. Verbindung nach Anspruch 21, wobei W ein Silikonrest ist, der der Formel (XII) oder der Formel (XIII) entspricht.

23. Verbindung nach Anspruch 22, ausgewählt aus statistisch aufgebauten Derivaten oder Derivaten mit gut definierten Blöcken mit mindestens einer und noch weiter bevorzugt allen der folgenden Eigenschaften:
- R" ist Alkyl und noch weiter bevorzugt Methyl,
- r" liegt zwischen 0 und 3 inklusive,
- s liegt zwischen 0 und 3 inklusive.

24. Verbindung nach einem der Ansprüche 20 bis 23, ausgewählt aus:
- 2,4-Bis(butyl-4'-diylaminobenzoat)-6-(2-ethylhexyl-4'-ylamino-α-cyanocinnamat)-s-triazin und
- 2-(4'-Ylamino-2-methansulfonylacrylnitril)-4-(butyl-4'-ylaminobenzoat)-6-{[1,3,3,3-tetramethyl-1-[(trimethylsilyl)oxy]disiloxanyl]propyl-3-yl-amino}-s-triazin.

25. Kosmetische oder dermatologische Zusammensetzung, die in einem physiologisch unbedenklichen Medium mindestens eine Aminoarylvinyl-s-triazin-Verbindung der Formel (III) gemäß einem der Ansprüche 20 bis 24 enthält.

26. Zusammensetzung nach Anspruch 25, wobei die Verbindungen der Formel (III) in Konzentrationen im Bereich von 0,1 bis 15 Gew.-% und weiter bevorzugt von 0,1 bis 10 Gew.-%, bezogen auf das Gesamtgewicht der Zusammensetzung, vorliegen.

27. Zusammensetzung nach Anspruch 25 oder 26, die ferner mindestens ein organisches Lichtschutzmittel und/oder mindestens ein anorganisches Lichtschutzmittel, das bzw. die im UVA und/oder UVB aktiv, wasserlöslich oder fettlöslich oder auch in gemeinhin verwendeten kosmetischen Lösungsmitteln unlöslich ist bzw. sind.

28. Zusammensetzung nach Anspruch 27, wobei die organischen Lichtschutzmittel aus Anthranilaten; Zimtsäurederivaten; Dibenzoylmethanderivaten; Salicylsäurederivaten; Campherderivaten; Triazinderivaten; Benzophenonderivaten; β,β'-Diphenylacrylatderivaten; Benzotriazolderivaten; Benzalmalonatderivaten; Benzimidazolderivaten; Imidazolinen; Bisbenzoazolylderivaten; p-Aminobenzoesäurederivaten (PABA-Derivaten); Methylenbis(hydroxyphenylbenzotriazol)derivaten; Benzoxazolderivaten; Filterpolymeren und Filtersilikonen; von α-Alkylstyrol abgeleiteten Dimeren; 4,4-Diarylbutadienen und Mischungen davon ausgewählt sind.

29. Zusammensetzung nach Anspruch 28, wobei die organischen Lichtschutzmittel aus den folgenden Verbindungen ausgewählt sind:
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,
Benzophenone-3,
Benzophenone-4,
Benzophenone-5,
2-(4-Diethylamino-2-hydroxybenzoyl)benzoesäure-n-hexylester,
4-Methylbenzylidene camphor,
Disodium Phenyl Dibenzimidazole Tetrasulfonate,
Anisotriazine,
Ethylhexyl triazone,
Diethylhexyl Butamido Triazone,
2,4,6-Tris(diisobutyl-4'-aminobenzalmalonat)-s-triazin
Methylene bis-Benzotriazolyl Tetramethylbutylphenol,
Drometrizole Trisiloxane,
Polysilicone-15,
1,1-Dicarboxy(2,2'-dimethylpropyl)-4,4-diphenyl-butadien,
2,4-Bis[5-1(dimethylpropyl)benzoxazol-2-yl(4-phenyl)imino]-6-(2-ethylhexyl)imino-1,3,5-triazin und Mischungen davon ausgewählt sind.

30. Zusammensetzung nach Anspruch 27, wobei die anorganischen Lichtschutzmittel aus beschichteten oder unbeschichteten Metalloxidpigmenten oder -nano-pigmenten ausgewählt sind.

31. Zusammensetzung nach Anspruch 30, wobei es sich bei den anorganischen Lichtschutzmitteln um beschichtete oder unbeschichtete Titanoxid-, Eisenoxid-, Zinkoxid-, Zirconiumoxid- oder Ceroxid-Nanopigmente handelt.

32. Zusammensetzung nach einem der Ansprüche 25 bis 31, wobei die Lichtschutzmittel in Anteilen im Bereich von 0,01 bis 20 Gew.-%, bezogen auf das Gesamtgewicht der Zusammensetzung, und vorzugsweise von 0,1 bis 10 Gew.-%, bezogen auf das Gesamtgewicht der Zusammensetzung, vorliegen.

33. Zusammensetzung nach einem der Ansprüche 25 bis 32, die außerdem mindestens ein Mittel zur künstlichen Bräunung und/oder Braunfärbung der Haut umfasst.

34. Zusammensetzung nach einem der Ansprüche 25 bis 33, die außerdem mindestens einen kosmetischen Hilfsstoff, der unter Fettsubstanzen, organischen Lösungsmitteln, ionischen oder nichtionischen, hydrophilen oder lipophilen Verdickungsmitteln, zartmachenden Mitteln, Feuchtigkeitsspendern, Trübungsmitteln, Stabilisatoren, Emollientien, Silikonen, Schaumhemmern, Duftstoffen, Konservierungsmitteln, anionischen, kationischen, nichtionischen, zwitterionischen oder amphoteren Tensiden, Wirkstoffen, Füllstoffen, Polymeren, Treibmitteln, Alkalinisierungsmitteln oder Ansäuerungsmitteln oder einem beliebigen anderen Inhaltsstoff, der in der Kosmetik und/oder Dermatologie gemeinhin verwendet wird, ausgewählt ist, umfasst.

## Claims

1. Cosmetic process for preserving the natural complexion, and the natural antioxidant protection of the skin, afforded by the endogenous carotenoids present in the skin, **characterized in that** it consists in applying to the skin a composition comprising at least one agent for screening out light radiation with a wavelength ranging from 370 to 500 nm chosen from
(v) carbohydrates;
(vi) yellow or orange-yellow mineral pigments;
(vii) azo or quinone compounds;
(viii) nitrobenzene-based dyes;
(ix) aryl vinylene ketone-based compounds;
(x) aminoarylvinyl-s-triazine compounds;
and mixtures thereof.

2. Process according to Claim 1, in which the carbohydrates are chosen from yellow-coloured products derived from the heating or oxidation of monosaccharides or polysaccharides.

3. Process according to Claim 2, in which the carbohydrate is caramel.

4. Process according to Claim 1, in which the yellow or orange mineral pigments are chosen from iron oxide pigments and more particularly nanopigments with an elemental particle size of less than 100 nm.

5. Process according to Claim 2, in which the quinone compounds are chosen from yellow or orange-yellow naphthoquinones and the oxidation products thereof; anthraquinones and the yellow or orange-yellow oxidation products thereof.

6. Process according to Claim 5, in which the quinone compounds are chosen from juglone and lawsone.

7. Process according to Claim 1, in which the azo compound is tartrazine.

8. Process according to Claim 1, in which the quinone or azo compound is chosen from those of formula (1), (2) or (3): 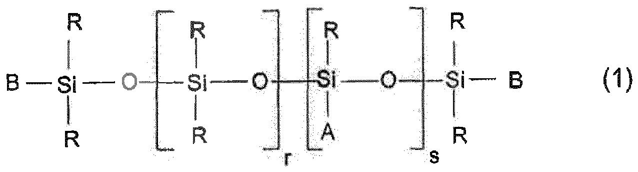 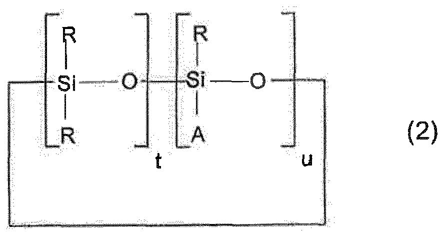 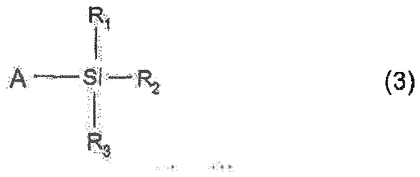 in which, for formulae (1) and (2):
- R, which may be identical or different, are chosen from C₁-C₁₀ alkyl, phenyl and 3,3,3-trifluoropropyl radicals, at least 80%, in numerical terms, of the radicals R being methyl,
- B, which may be identical or different, are chosen from the radicals R above and the radical A defined below,
- r is an integer between 0 and 50 inclusive, and s is an integer between 0 and 20 inclusive, with the condition that if s is 0, then at least one of the two symbols B denotes A,
- u is an integer between 1 and 6 inclusive, and
- t is an integer between 0 and 10 inclusive,
it being understood that t + u is greater than or equal to 3,
- R₁, R₂ and R₃, which may be identical or different, are chosen from saturated or unsaturated, linear or branched C₁-C₈ alkyl and alkenyl radicals,
- the symbols A, which may be identical or different, denote a radical of formula (4a), (4b), (4c) or (4d) below: 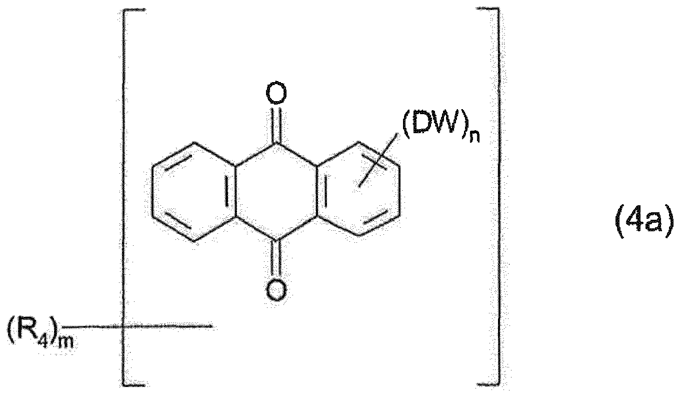 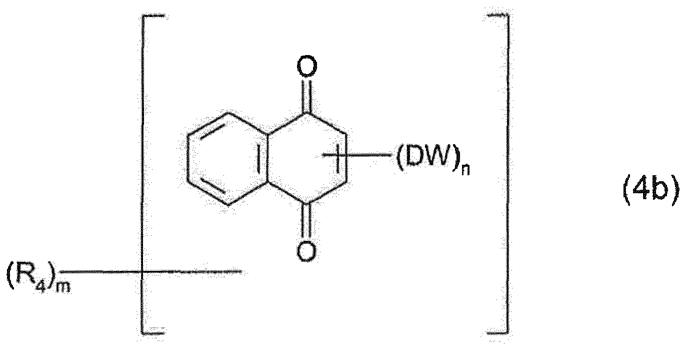 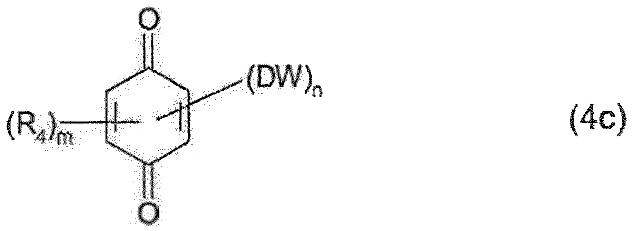 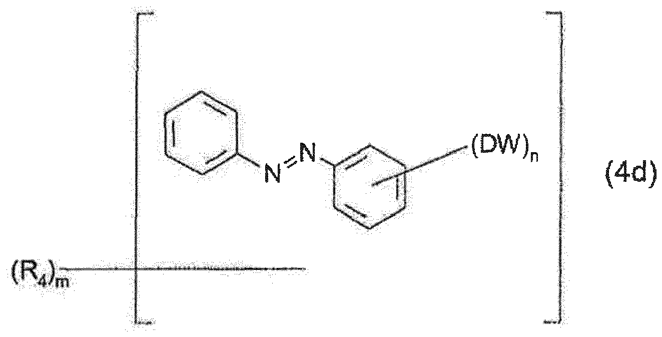
- R₄, which may be identical or different, represent a linear or branched C₁-C₆ alkyl radical, an OH, C₁-C₄ alkoxy, hydroxy (C₁-C₄) alkyl, COOH, CONH₂, CN, SO₃H, halogen or NO₂ radical, a radical NR₅R₆ in which R₅ and R₆, which may be identical or different, denote a hydrogen atom or a C₁-C₈ alkyl, hydroxy (C₁-C₄) alkyl or amino(C₁-C₄)alkyl radical, or form, together with the nitrogen atom to which they are attached, a 5- or 6-membered heterocycle optionally interrupted with an oxygen or sulfur atom,
- m is an integer between 0 and 2 inclusive,
- n is an integer equal to 1 or 2,
- D is an -SO₂NH-, -CONH- or -O- radical or a radical
- NR₇- in which R₇ is H or CH₃,
- W is a divalent radical of formula (5): 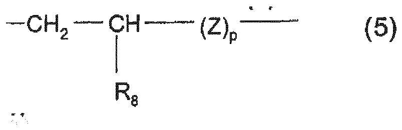
or of formula (6) :
- HC =C-(Z)ₚ- (6)
in which
- R₈ denotes a hydrogen atom, a hydroxyl radical or a linear or branched, saturated or unsaturated C₁-C₈ alkyl radical,
- Z is a linear or branched C₁-C₆ alkylene radical optionally substituted with an OH radical or a linear or branched, saturated or unsaturated C₂-C₈ alkoxy radical,
- p is an integer equal to 0 or 1.

9. Process according to Claim 8, in which the compound of formula (1), (2) or (3) is chosen from the following compounds:
- 4-(4-dimethylaminophenylazo)-N-[3-[1,3,3,3-tetramethyl-1-[(trimethylsilyl)oxy]disiloxanyl]propyl]-benzenesulfonamide;
- 4-(4-dimethylaminophenylazo)-N-[3-[1,3,3,3-tetramethyl-1-[(trimethylsilyl)oxy]disiloxanyl]propyl]benzamide;
- 2-(4-methoxy-2-nitrophenylazo)-5-(3-trimethylsilanylpropoxy)phenol;
- 2,5-bis-[3-[1,3,3,3-tetramethyl-1-[(trimethylsilyl)-oxy]disiloxanyl]propylamino]benzoquinone;
- 2-chloro-3-[3-[1,3,3,3-tetramethyl-1-[(trimethylsilyl)oxy]disiloxanyl]propylamino]-[1,4]-naphthoquinone;
- 2-[3-[1,3,3,3-tetramethyl-1-[(trimethylsilyl)oxy]-disiloxanyl]propylamino]-[1,4]-naphthoquinone;
- 1-hydroxy-4-[3-[1,3,3,3-tetramethyl-1-[(trimethylsilyl)oxy]disiloxanyl]propylamino]anthraquinone;
- 1,4-di[3-[1,3,3,3-tetramethyl-1-[(trimethylsilyl)-oxy]disiloxanyl]propylamino]anthraquinone.

10. Process according to Claim 1, in which the nitrobenzene compound is chosen from those of formula (15) or (16): 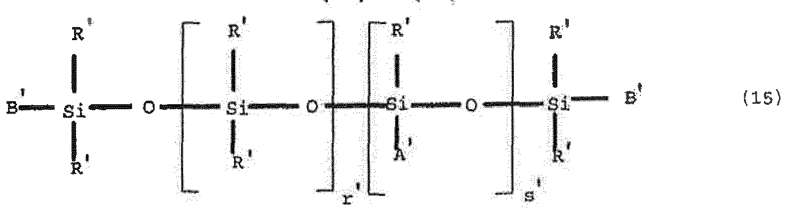 or 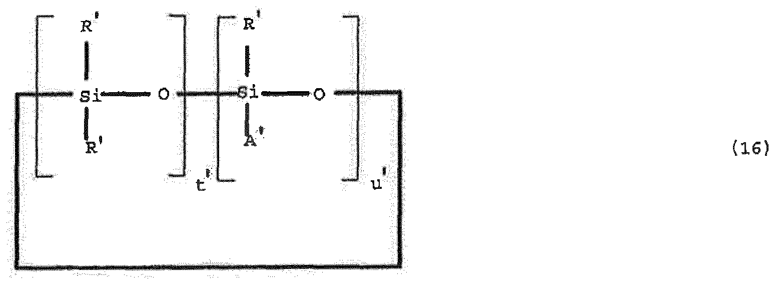 in which:
- R', which may be identical or different, are chosen from linear or branched C₁-C₁₀ alkyl, phenyl and 3,3,3-trifluoropropyl radicals, at least 80% in numerical terms of the radicals R' being methyl,
- B', which may be identical or different, are chosen from the radicals R' above and the radical A defined below,
- r' is an integer between 0 and 50 inclusive and s' is an integer between 0 and 20 inclusive, with the condition that if s' is zero then at least one of the two symbols B denotes A,
- u' is an integer between 1 and 6 inclusive, and t' is an integer between 0 and 10 inclusive, it being understood that t' + u' is greater than or equal to 3,
- and the symbol A' denotes a monovalent radical directly linked to a silicon atom, and which corresponds to formula (17) below: 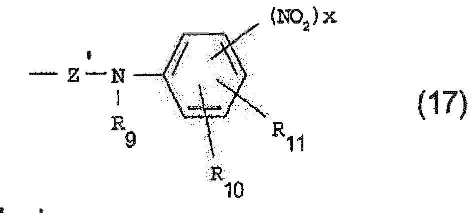
- Z' is the divalent radical: 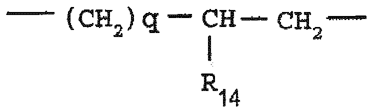
or hydrogen,
- x is 1 or 2,
- q represents an integer between 0 and 10 inclusive,
- R₉ represents hydrogen or a C₁-C₄ alkyl radical,
- R₁₀ represents hydrogen or a C₁-C₄ alkyl radical, or the divalent radical Z' defined above,
- R₁₁ represents hydrogen or a radical NR₁₂R₁₃ in which R₁₂ and R₁₃ represent hydrogen or a C₁-C₄ alkyl radical, a C₂-C₄ monohydroxyalkyl or dihydroxyalkyl radical or the divalent radical Z,
- R₁₄ represents hydrogen, an OH or halogen radical, a C₁-C₄ alkyl radical or a C₁-C₄ alkoxy radical.

11. Process according to Claim 10, in which the compound of formula (15) or (16) is chosen from the following compounds:

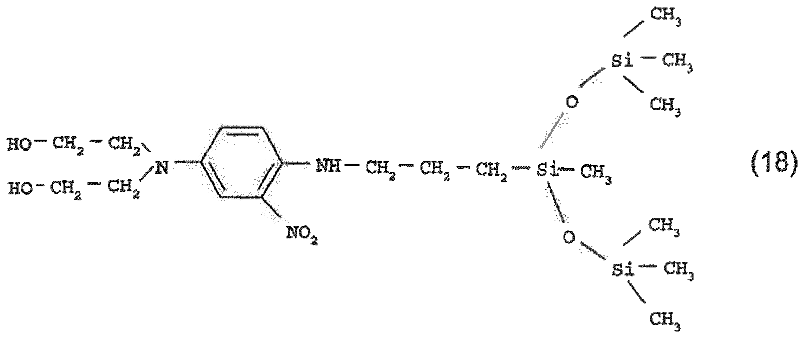

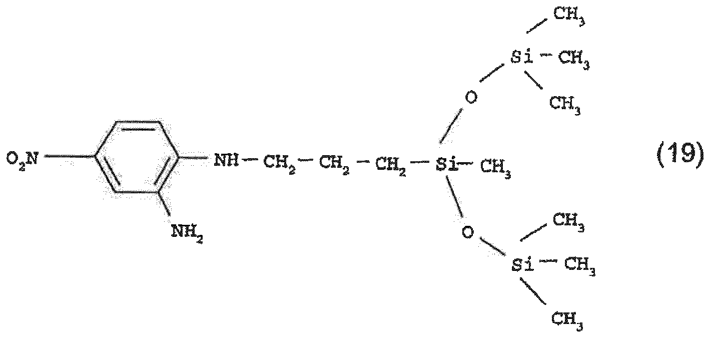

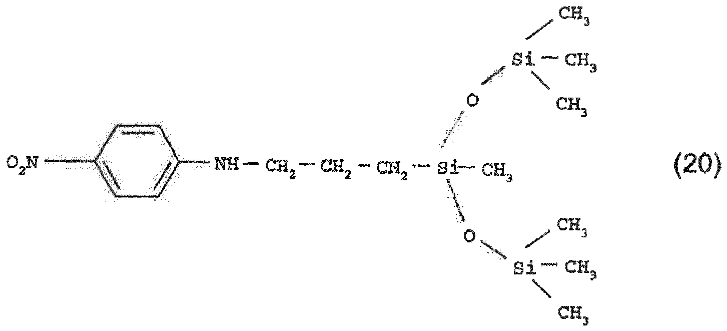

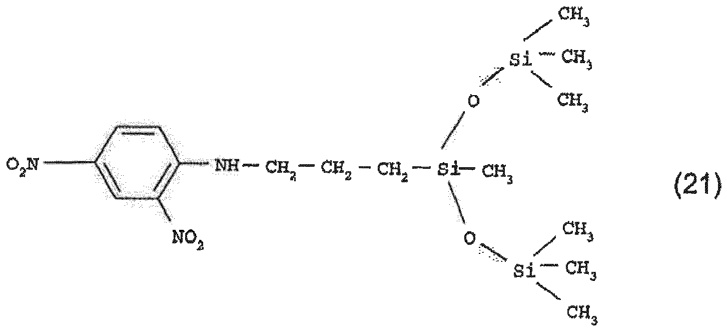

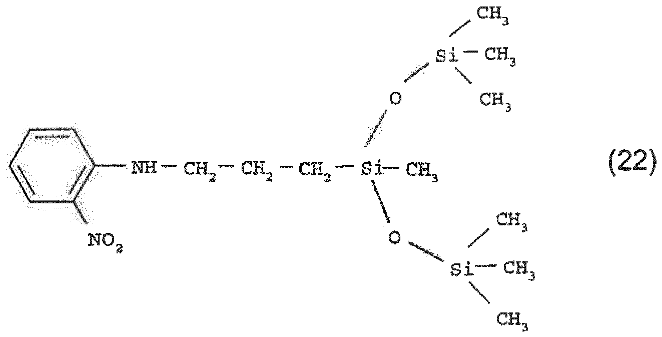

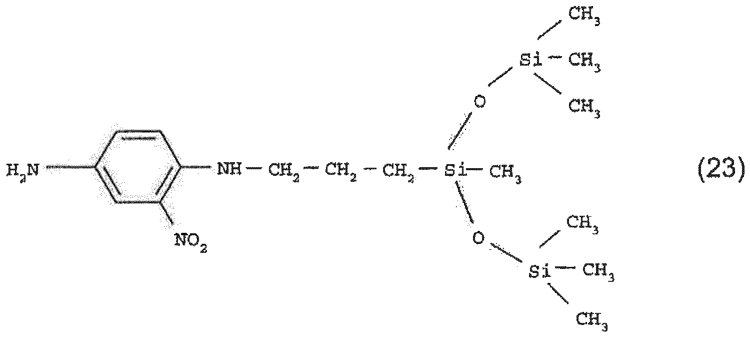

12. Process according to Claim 1, in which the aryl vinyl ketone compound is chosen from those of formulae (I) and (II) below: 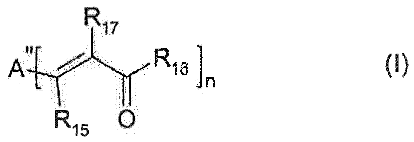 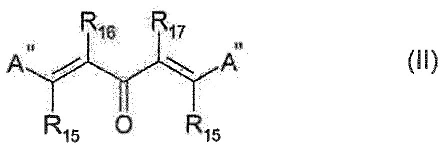 in which:
n = 1 or 2,
A", in formula (I) when n = 1 or in formula (II), is an aryl radical chosen from formulae (a) to (d) below, or in formula (I) when n = 2, is a radical chosen from formulae (e) to (h) below: 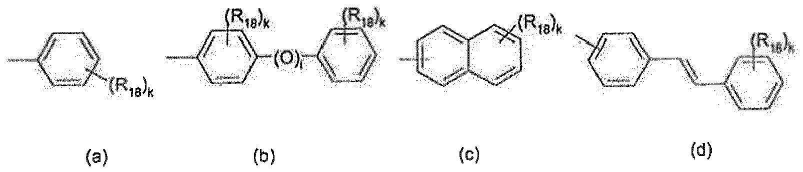 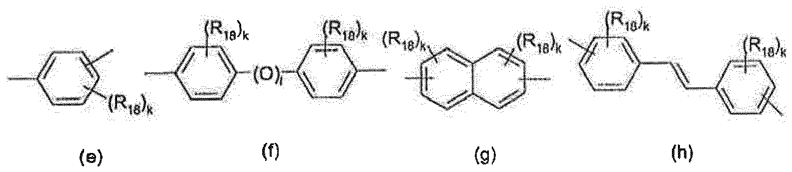
in which:
- each of the symbols R₁₈ independently represents an OH group, a halogen atom, a linear or branched C₁₋₆ alkyl group optionally containing a silicon atom or a siloxane group, a linear or branched C₁₋₆ alkoxy group optionally containing a silicon atom or a siloxane group, a linear or branched C₁₋₅ alkoxycarbonyl group, or a linear or branched C₁₋₆ alkylsulfonamide group optionally containing a silicon atom, a siloxane group or an amino acid function,
- k represents an integer between 0 and 3 inclusive,
- 1 represents 0 or 1,
- R₁₅ represents hydrogen or an OH group,
- R₁₆ represents hydrogen, a linear or branched C₁₋₆ alkyl group optionally containing a silicon atom or a siloxane group, a cyano group, a C₁₋₆ alkylsulfonyl group or a phenylsulfonyl group,
- R₁₇ represents a linear or branched C₁₋₆ alkyl group optionally containing a silicon atom, a siloxane group or a phenyl group that can form a bicycle and optionally substituted with one or two radicals R₁₈ as defined above,
- R₁₆ and R₁₇ may together form a monocyclic, bicyclic or tricyclic C₂₋₁₀ hydrocarbon-based residue, optionally interrupted with one or more nitrogen, sulfur and oxygen atoms and possibly containing another carbonyl, and optionally substituted with a linear or branched C₁-C₈ alkylsulfonamide group, optionally containing a silicon atom, a siloxane group or an amino acid function; on condition that when n = 1, R₁₆ and R₁₇ do not form a camphor nucleus.

13. Process according to Claim 12, in which the compound of formula (I) is chosen from:
- 1-(2,4,5-trimethoxyphenyl)-4,4-dimethylpent-1-en-3-one: 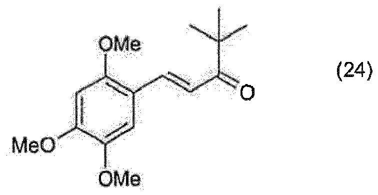
- 3-(3-ethoxy-4-butoxybenzylidene)-2,3,4a,8a-tetrahydrochromen-4-one: 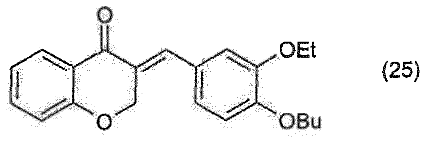
- 3-(4-methoxybenzylidene)-2,3,4a,8a-tetrahydrochromene-4-thione: 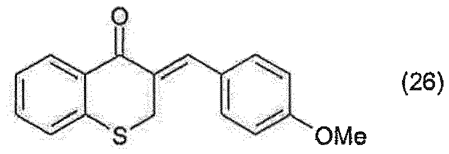
- 2-(3-methoxy-4-butoxybenzylidene)-indan-1-one: 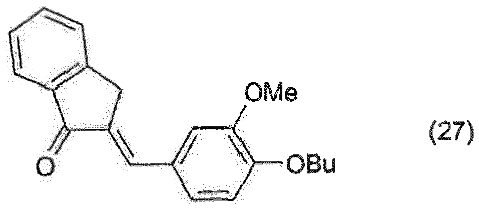
- 2-(3-methoxy-4-butoxybenzylidene)-3,4-dihydro-2H-naphthalen-1-one: 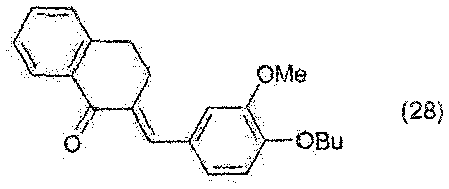
- 2-benzylidenebenzofuran-3-one: 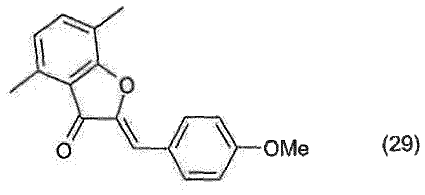
- 2-(3,5-dimethoxy-4-hydroxybenzylidene)indan-1,3-dione: 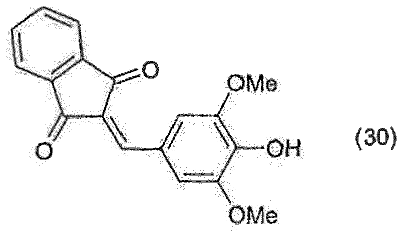
- 2-benzylidenebenzo[b]thiophen-3-one: 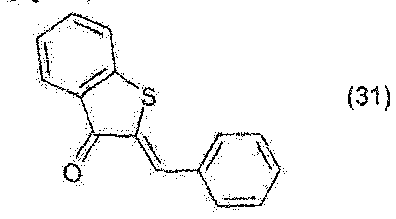
- 4-(3-methoxy-4-butoxybenzylidene)-5-methyl-2-phenyl-2,4-dihydropyrazol-3-one: 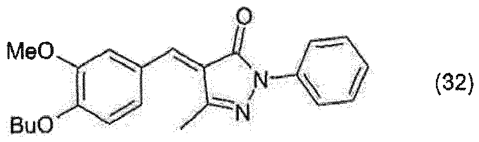
- 5-(3-methoxy-4-butoxybenzylidene)-2-phenyl-3,5-dihydroimidazol-4-one: 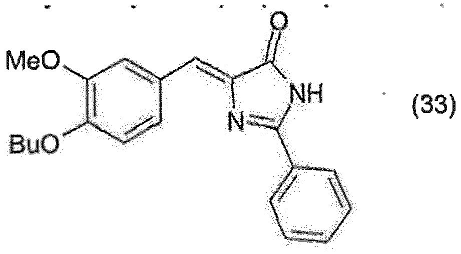
- 1-(2-hydroxy-4-methoxyphenyl)-3-(4'-methoxyphenyl)-propenone: 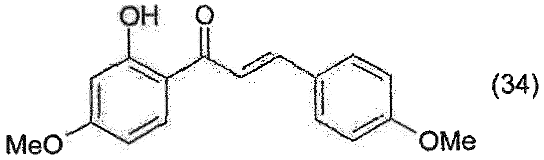
- 3-hydroxy-1-(2-hydroxy-4-methoxyphenyl)-3-phenylpropenone: 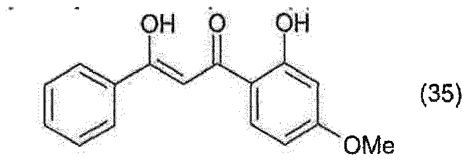
- the para-xylidene compound below: 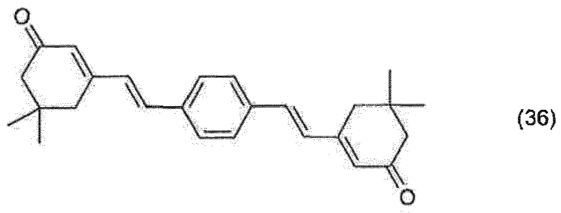
- 2,5-dimethoxyphenylene-1,4-bis(3-methylidene-bicyclo[2.2.1]heptan-2-one): 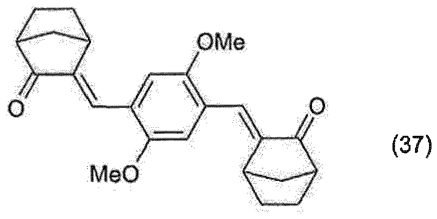
- 2,5-dihexyloxyphenylene-1,4-bis(3-methylidene-1,7,7-trimethylbicyclo[2.2.1]heptan-2-one):

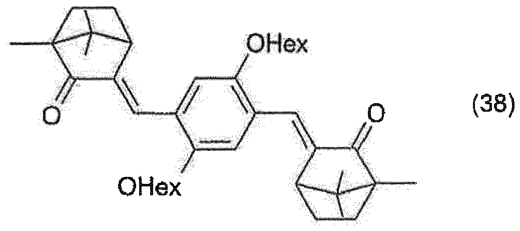

14. Process according to Claim 13, in which the compound of formula (II) is chosen from:
- 2,5-bis(4-hexyloxybenzylidene)cyclopentanone;
- 2,5-bis{4-[[1,3,3,3-tetramethyl-1-[(trimethylsilyl)oxy]disiloxanyl]-3-methylpropyloxy]benzylidene}cyclopentanone.

15. Process according to Claim 1, in which the aminoarylvinyl-s-triazine compounds correspond to formula (III) below: 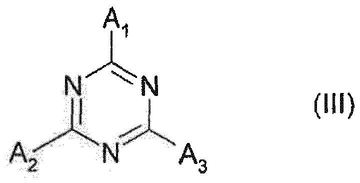 in which:
The groups A₁, A₂ and A₃, which may be identical or different, are chosen from the groups of formulae (IV) to (VIII) below, it being understood that at least one group of formulae (IV) to (VII) is present: 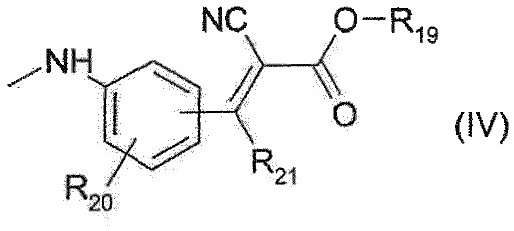 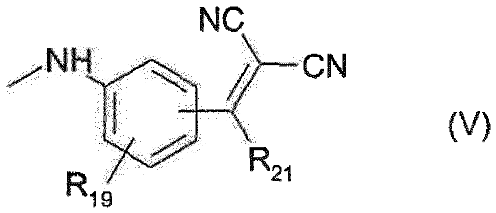 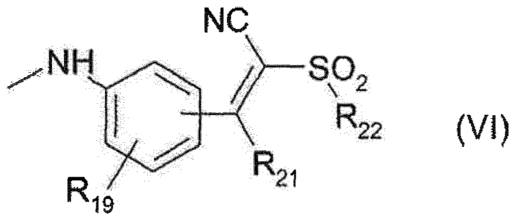 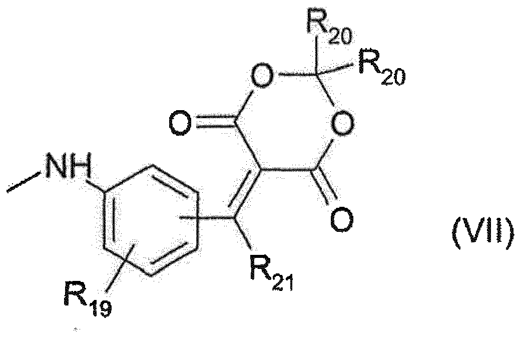
**-O-Z₁-W₁** **(VIII)**
**-NH-Z₂-(W₁)ₐ** **(IX)**
in which:
R₂₀ represents hydrogen, a C₁-C₄ alkyl radical or a C₁-C₄ alkoxy radical,
R₁₉ represents a linear or branched C₁-C₂₀ alkyl radical, a linear or branched C₂-C₂₀ hydroxyalkyl radical or a linear or branched C₁-C₂₀ alkoxy radical,
R₂₁ represents hydrogen, the methyl radical or the phenyl radical optionally substituted with a C₁-C₄ alkyl radical or a C₁-C₄ alkoxy radical,
R₂₂ represents a linear or branched C₁-C₂₀ alkyl radical or a phenyl radical optionally substituted with a C₁-C₄ alkyl radical or a C₁-C₄ alkoxy radical,
Z₁ represents a divalent radical providing the bond between -0- and -W₁,
Z₂ represents a divalent radical when a = 1 and a trivalent radical when a = 2, providing the bond between -NH- and - (W₁)a,
Z₁ and Z₂ possibly being C₁-C₁₂ alkylene, optionally substituted with one or more hydroxyl groups and possibly containing one or more oxygen atoms or one or more amino groups and optionally containing a double bond,
W₁ represents:
(i) either a silicone radical comprising at least one unit of formula (X) below 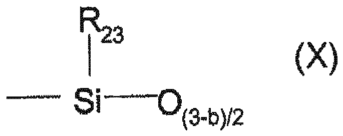 in which R₂₃ denotes a saturated or unsaturated, linear or branched C₁-C₃₀ hydrocarbon-based group; a C₁-C₈ halohydrocarbon group; the phenyl radical; the 3,3,3-trifluoropropyl radical or a trimethylsilyloxy group, and b is equal to 1 or 2,
(ii) or a radical of formula (XI) below:
-SiR₂₄R₂₅R₂₆ (XI)
in which R₂₄, R₂₅ and R₂₆, which may be identical or different, are chosen from linear or branched C₁-C₈ alkyl and alkenyl radicals;
the groups A₁, A₂ and A₃ may also represent a UVB- and/or UVA-absorbing chromophore preferably chosen from aryl groups substituted with hydroxyl groups, linear or branched C₁-C₂₀ alkyl groups and linear or branched C₁-C₂₀ alkoxy groups; aminobenzylidenecamphor groups; aminobenzotriazole groups; linear or branched aminobenzoate, aminobenzalmalonate, aminosalicylate, C₁-C₂₀ aminocinnamate or anthranilate ester groups.

16. Process according to Claim 15, in which the compounds of formula (III) are chosen from those for which W is a silicone radical corresponding to one of the three formulae (XII) to (XIV) below: 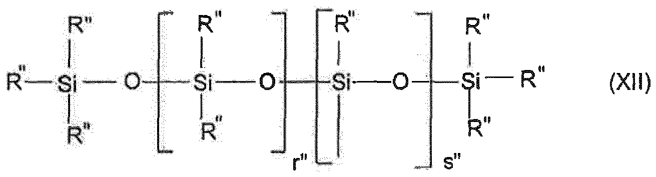 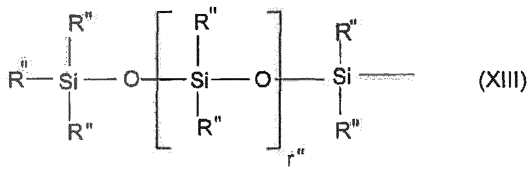 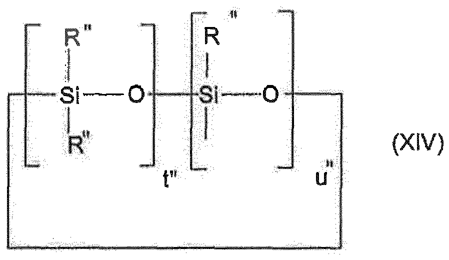 in which:
- R", which may be identical or different, are chosen from linear or branched, saturated or unsaturated C₁-C₂₀ alkyl radicals, the phenyl radical and the 3,3,3-trifluoropropyl radical, at least 80% in numerical terms of the radicals R being the methyl radical,
- r" is an integer chosen between 0 and 50 inclusive,
- s" is an integer chosen between 0 and 20 inclusive,
- u" is an integer between 1 and 6 inclusive,
- t" is an integer between 0 and 10 inclusive,
- t" + u" is greater than or equal to 3.

17. Process according to Claim 16, in which the compounds of formula (III) are chosen from those for which W is a silicone radical corresponding to formula (XII) or formula (XIII).

18. Process according to Claim 17, in which the compounds of formula (III) are chosen from random derivatives or else defined derivatives with blocks having at least one and even more preferably all of the following characteristics:
- R" is alkyl and even more preferably is methyl,
- r" is between 0 and 3 inclusive;
- s is between 0 and 3 inclusive.

19. Process according to any one of Claims 15 to 18, in which the compounds of formula (III) are chosen from:
- 2-(ethyl 4'-ylamino-α-cyanocinnamate)-4-(4-methoxyphenyl)-6-{[1,3,3,3-tetramethyl-1-[(trimethylsilyl)-oxy]disiloxanyl]propyl-3-ylamino}-s-triazine,
- 2-(ethyl 4'-ylamino-α-cyanocinnamate)-4-(butyl 4'-ylaminobenzoate)-6-{[1,3,3,3-tetramethyl-1-[(trimethylsilyl)oxy]disiloxanyl]propyl-3-ylamino}-s-triazine,
- 2-(4'-ylamino-2-methanesulfonylacrylonitrile)-4-(butyl 4'-ylaminobenzoate)-5-{[1,3,3,3-tetramethyl-1-[(trimethylsilyl)oxy]disiloxanyl]propyl-3-ylamino}-s-triazine,
- 2-(ethyl 4'-ylamino-α-cyanocinnamate)-4-(butyl 4'-ylaminobenzoate)-6-(trimethylsilanylmethylylamino)-s-triazine,
- 2,4-bis(butyl 4'-diylaminobenzoate)-6-(2-ethylhexyl 4'-ylamino-a-cyanocinnamate)-s-triazine,
- 2,4,6-tris(isobutyl 4'-ylamino-α-cyanocinnamate)-s-triazine,
- 2,4,6-tris(2-ethylhexyl 4'-ylamino-α-cyanocinnamate)-s-triazine,
- 2,4-bis(2-ethylhexyl 4'-ylamino-α-cyanocinnamate)-6-{[1,3,3,3-tetramethyl-1-[(trimethylsilyl)oxy]disiloxanyl]propyl-3-ylamino}-s-triazine.

20. Aminoarylvinyl-s-triazine compound corresponding to formula (III) below: 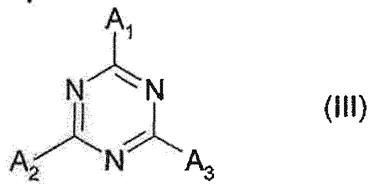 in which:
the groups A₁, A₂ and A₃, which may be identical or different, are chosen from the groups of formulae (IV) to (VIII) below, it being understood that at least one group of formulae (IV) to (VII) is present: 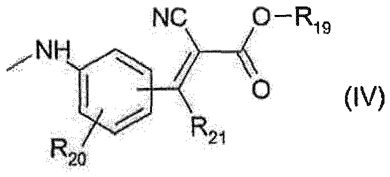 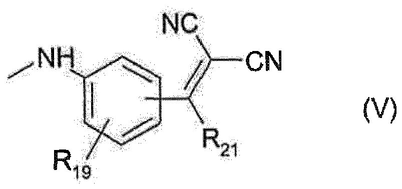 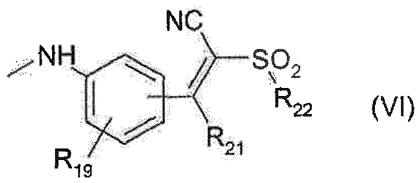 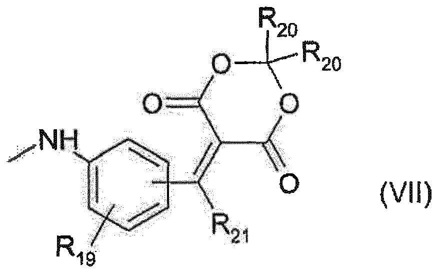
-O-Z₁-W₁ (VIII)
-NH-Z₂-(W₁)ₐ (IX)
in which:
R₂₀ represents hydrogen, a C₁-C₄ alkyl radical or a C₁-C₄ alkoxy radical,
R₁₉ represents a linear or branched C₁-C₂₀ alkyl radical, a linear or branched C₂-C₂₀ hydroxyalkyl radical or a linear or branched C₁-C₂₀ alkoxy radical,
R₂₁ represents hydrogen, the methyl radical or the phenyl radical optionally substituted with a C₁-C₄ alkyl radical or a C₁-C₄ alkoxy radical,
R₂₂ represents a linear or branched C₁-C₂₀ alkyl radical or a phenyl radical optionally substituted with a C₁-C₄ alkyl radical or a C₁-C₄ alkoxy radical,
Z₁ represents a divalent radical providing the bond between -O- and -W₁,
Z₂ represents a divalent radical when a = 1 and a trivalent radical when a = 2, providing the bond between -NH- and - (W₁) a,
Z₁ and Z₂ possibly being C₁-C₁₂ alkylene, optionally substituted with one or more hydroxyl groups and possibly containing one or more oxygen atoms or one or more amino groups and optionally containing a double bond,
W₁ represents:
(i) either a silicone radical comprising at least one unit of formula (X) below: 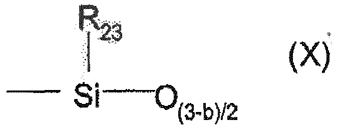 in which R₂₃ denotes a saturated or unsaturated, linear or branched C₁-C₃₀ hydrocarbon-based group; a C₁-C₈ halohydrocarbon group; a phenyl radical; the 3,3,3-trifluoropropyl radical or a trimethylsilyloxy group, and b is equal to 1 or 2,
(ii) or a radical of formula (XI) below:
-SiR₂₄R₂₅R₂₆ (XI)
in which R₂₄, R₂₅ and R₂₆, which may be identical or different, are chosen from linear or branched C₁-C₈ alkyl and alkenyl radicals;
the groups A₁, A₂ and A₃ may also be chosen from aryl groups substituted with hydroxyl groups, linear or branched C₁-C₂₀ alkyl groups and linear or branched C₁-C₂₀ alkoxy groups; aminobenzylidenecamphor groups; aminobenzotriazole groups; linear or branched aminobenzoate, aminobenzalmalonate, aminosalicylate, C₁-C₂₀ aminocinnamate or anthranilate ester groups; with the proviso that:
- the three groups A₁, A₂ and A₃ do not denote the same formula (IV),
- when A₂ and A₃ denote a group of formula (IV), then A₁ does not denote a group of formula (VIII) or (IX).

21. Compound according to Claim 20, in which W is a silicone radical corresponding to one of the three formulae (XII) to (XIV) below: 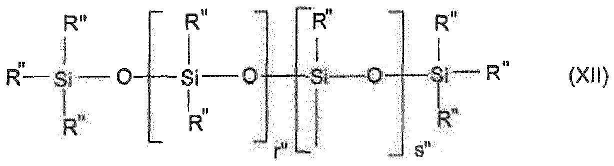 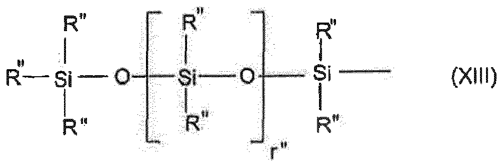 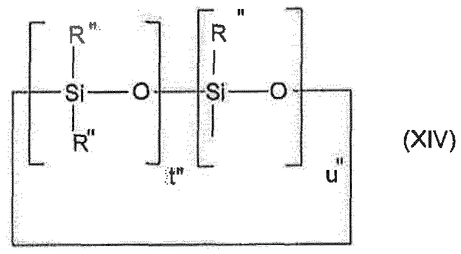 in which:
- R", which may be identical or different, are chosen from linear or branched, saturated or unsaturated C₁-C₂₀ alkyl radicals, the phenyl radical and the 3,3,3-trifluoropropyl radical, at least 80% in numerical terms of the radicals R being the methyl radical,
- r" is an integer chosen between 0 and 50 inclusive,
- s" is an integer chosen between 0 and 20 inclusive,
- u" is an integer between 1 and 6 inclusive,
- t" is an integer between 0 and 10 inclusive,
- t" + u" is greater than or equal to 3.

22. Compound according to Claim 21, in which W is a silicone radical corresponding to formula (XII) or to formula (XIII).

23. Compound according to Claim 22, chosen from random derivatives or else defined derivatives with blocks having at least one and even more preferably all of the following characteristics:
- R" is alkyl and even more preferably is methyl,
- r" is between 0 and 3 inclusive; s is between 0 and 3 inclusive.

24. Compound according to any one of Claims 20 to 23, chosen from:
- 2,4-bis(butyl 4'-diylaminobenzoate)-6-(2-ethylhexyl 4'-ylamino-α-cyanocinnamate)-s-triazine;
- 2-(4'-ylamino-2-methanesulfonylacrylonitrile)-4-(butyl 4'-ylaminobenzoate)-6-{[1,3,3,3-tetramethyl-1-[(trimethylsilyl)oxy]disiloxanyl]propyl-3-ylamino}-s-triazine.

25. Cosmetic or dermatological composition containing, in a physiologically acceptable medium, at least one aminoarylvinyl-s-triazine compound of formula (III) as defined in any one of Claims 20 to 24.

26. Composition according to Claim 25, in which the compounds of formula (III) are present in concentrations ranging from 0.1% to 15% by weight and more particularly from 0.1% to 10% by weight relative to the total weight of the composition.

27. Composition according to Claim 25 or 26, also comprising at least one UVA-active and/or UVB-active organic photoprotective agent and/or at least one UVA-active and/or UVB-active mineral photoprotective agent, which is (are) water-soluble or liposoluble or even insoluble in the cosmetic solvents commonly used.

28. Composition according to Claim 27, in which the organic photoprotective agents are chosen from anthranilates; cinnamic derivatives; dibenzoylmethane derivatives; salicylic derivatives; camphor derivatives; triazine derivatives; benzophenone derivatives; β,β'-diphenylacrylate derivatives;
benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkylstyrene; 4,4-diarylbutadienes, and mixtures thereof.

29. Composition according to Claim 28, in which the organic photoprotective agents are chosen from the following compounds:
- Ethylhexyl salicylate,
- Butyl methoxydibenzoylmethane,
- Ethylhexyl methoxycinnamate,
- Octocrylene,
- Phenylbenzimidazolesulfonic acid,
- Terephthalylidenedicamphorsulfonic acid,
- Benzophenone-3,
- Benzophenone-4,
- Benzophenone-5,
- n-Hexyl 2-(4-diethylamino-2-hydroxybenzoyl)-benzoate,
- 4-Methylbenzylidenecamphor,
- Disodium phenyldibenzimidazoletetrasulfonate,
- Anisotriazine,
- Ethylhexyltriazone,
- Diethylhexylbutamidotriazone,
- 2,4,6-Tris(diisobutyl 4'-aminobenzalmalonate)-s-triazine,
- Methylenebis(benzotriazolyl)tetramethylbutylphenol,
- Drometrizole trisiloxane,
- Polysilicone-15,
- 1,1-Dicarboxy(2,2'-dimethylpropyl)-4,4-diphenylbutadiene,
- 2,4-Bis[5-1(dimethylpropyl)benzoxazol-2-yl(4-phenyl)imino]-6-(2-ethylhexyl)imino-1,3,5-triazine,
and mixtures thereof.

30. Composition according to Claim 27, in which the mineral photoprotective agents are chosen from coated or uncoated metal oxide pigments or nanopigments.

31. Composition according to Claim 30, in which the mineral photoprotective agents are nanopigments of titanium oxide, iron oxide, zinc oxide, zirconium oxide or cerium oxide, which are coated or uncoated.

32. Composition according to any one of Claims 25 to 31, in which the photoprotective agents are present in proportions ranging from 0.01% to 20% by weight relative to the total weight of the composition and preferably ranging from 0.1% to 10% by weight relative to the total weight of the composition.

33. Composition according to any one of Claims 25 to 32, also containing an agent for artificially tanning and/or browning the skin.

34. Composition according to any one of Claims 25 to 33, also containing at least one cosmetic adjuvant chosen from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, acidifying or basifying agents or any other ingredient usually used in cosmetics and/or dermatology.
